# EUROPEAN PATENT APPLICATION

(11) **EP 3 928 789 A1**
(43) Date of publication of application: **29.12.2021**
(21) Application number: 20382558.3
(22) Date of filing: 24.06.2020
(51) Int. Cl.: A61K 39/12, A61P 31/14

(54) **MVA-BASED VACCINE AGAINST COVID-19 EXPRESSING SARS-COV-2 ANTIGENS**

(71) Applicant: Consejo Superior de Investigaciones Científicas (CSIC), 28006 Madrid (ES)
(72) Inventor: GARCÍA ARRIAZA, Juan Francisco, 28006 Madrid (ES); ESTEBAN RODRÍGUEZ, Mariano, 28006 Madrid (ES)
(74) Representative: Hoffmann Eitle

(57) **Abstract**

The present invention is directed to a recombinant modified vaccinia virus Ankara (MVA), which carries a nucleic acid sequence coding for an antigenic protein of SARS-CoV-2 useful for vaccinating against COVID19. The present invention is further directed to a vaccine composition containing said recombinant MVA as well as to a method for enhancing T cell and humoral immune responses in a mammal against COVID19.

SEQUENCE LISTING

<110> Consejo Superior de Investigaciones Cientificas (CSIC)

<120> MVA-BASED VACCINE AGAINST COVID-19 EXPRESSING SARS-CoV-2 ANTIGENS

<130> 905 344

<160> 20

<170> BiSSAP 1.3.6

<210> 1
<211> 3822
<212> DNA
<213> Coronaviridae


<220>
<223> SARS-CoV-2 S nucleotide sequence

<400> 1
atgttcgtgt ttctggtgct gctgcctctg gtgtccagcc agtgtgtgaa cctgaccacc 60

agaacacagc tgcctccagc ctacaccaac agctttacca gaggcgtgta ctaccccgac 120

aaggtgttca gatccagcgt gctgcactct acccaggacc tgttcctgcc tttcttcagc 180

aacgtgacct ggttccacgc catccacgtg tccggcacca atggcaccaa gagattcgac 240

aaccccgtgc tgcccttcaa cgacggggtg tactttgcca gcaccgagaa gtccaacatc 300

atcagaggct ggatcttcgg caccacactg gacagcaaga cccagagcct gctgatcgtg 360

aacaacgcca ccaacgtggt catcaaagtg tgcgagttcc agttctgcaa cgaccccttc 420

ctgggcgtct actaccacaa gaacaacaag agctggatgg aaagcgagtt ccgggtgtac 480

agcagcgcca acaactgcac cttcgagtac gtgtcccagc ctttcctgat ggacctggaa 540

ggcaagcagg gcaacttcaa gaacctgcgc gagttcgtgt tcaagaacat cgacggctac 600

ttcaagatct acagcaagca cacccctatc aacctcgtgc gggatctgcc tcagggcttc 660

tctgctctgg aacccctggt ggatctgccc atcggcatca acatcacccg gtttcagaca 720

ctgctggccc tgcacagaag ctacctgaca cctggcgata gcagcagcgg atggacagct 780

ggtgccgccg cttactatgt gggctacctg cagcctagaa ccttcctgct gaagtacaac 840

gagaacggca ccatcaccga cgccgtggat tgtgctctgg atcctctgag cgagacaaag 900

tgcaccctga agtccttcac cgtggaaaag ggcatctacc agaccagcaa cttccgggtg 960

cagcccaccg aatccatcgt gcggttcccc aatatcacca atctgtgccc cttcggcgag 1020

gtgttcaatg ccaccagatt cgcctctgtg tacgcctgga accggaagcg gatcagcaat 1080

tgcgtggccg actactccgt gctgtacaac tccgccagct tcagcacctt caagtgctac 1140

ggcgtgtccc ctaccaagct gaacgacctg tgcttcacaa acgtgtacgc cgacagcttc 1200

gtgatccggg gagatgaagt gcggcagatt gcccctggac agacaggcaa gatcgccgac 1260

tacaactaca agctgcccga cgacttcacc ggctgtgtga ttgcctggaa cagcaacaac 1320

ctggactcca aagtcggcgg caactacaat tacctgtacc ggctgttccg gaagtccaat 1380

ctgaagccct tcgagcggga catctccacc gagatctatc aggccggcag caccccttgt 1440

aacggcgtgg aaggcttcaa ctgctacttc ccactgcagt cctacggctt tcagcccaca 1500

aatggcgtgg gctatcagcc ctacagagtg gtggtgctga gcttcgaact gctgcatgcc 1560

cctgccacag tgtgcggccc taagaaaagc accaatctcg tgaagaacaa atgcgtgaac 1620

ttcaacttca acggcctgac cggcaccggc gtgctgacag agagcaacaa gaagttcctg 1680

ccattccagc agtttggccg ggatatcgcc gataccacag acgccgttag agatccccag 1740

acactggaaa tcctggacat caccccttgc agcttcggcg gagtgtctgt gatcacccct 1800

ggcaccaaca ccagcaatca ggtggcagtg ctgtaccagg acgtgaactg taccgaagtg 1860

cccgtggcca ttcacgccga tcagctgaca cctacatggc gggtgtactc caccggcagc 1920

aatgtgtttc agaccagagc cggctgtctg atcggagccg agcacgtgaa caatagctac 1980

gagtgcgaca tccccatcgg cgctggcatc tgtgccagct accagacaca gacaaacagc 2040

cccagacggg ccagatctgt ggccagccag agcatcattg cctacacaat gtctctgggc 2100

gccgagaaca gcgtggccta ctccaacaac tctatcgcta tccccaccaa cttcaccatc 2160

agcgtgacca cagagatcct gcctgtgtcc atgaccaaga ccagcgtgga ctgcaccatg 2220

tacatctgcg gcgattccac cgagtgctcc aacctgctgc tgcagtacgg cagcttctgc 2280

acccagctga atagagccct gacagggatc gccgtggaac aggacaagaa cacccaagag 2340

gtgttcgccc aagtgaagca gatctacaag acccctccta tcaaggactt cggcggcttc 2400

aatttcagcc agattctgcc cgatcctagc aagcccagca agcggagctt catcgaggac 2460

ctgctgttca acaaagtgac actggccgac gccggcttca tcaagcagta tggcgattgt 2520

ctgggcgaca ttgccgccag ggatctgatt tgcgcccaga agtttaacgg actgacagtg 2580

ctgcctcctc tgctgaccga tgagatgatc gcccagtaca catctgccct gctggccggc 2640

acaatcacaa gcggctggac atttggagct ggcgccgctc tgcagatccc ctttgctatg 2700

cagatggcct accggttcaa cggcatcgga gtgacccaga atgtgctgta cgagaaccag 2760

aagctgatcg ccaaccagtt caacagcgcc atcggcaaga tccaggacag cctgagcagc 2820

acagcaagcg ccctgggaaa gctgcaggac gtggtcaacc agaatgccca ggcactgaac 2880

accctggtca agcagctgtc ctccaacttc ggcgccatca gctctgtgct gaacgatatc 2940

ctgagcagac tggacaaggt ggaagccgag gtgcagatcg acagactgat caccggaagg 3000

ctgcagtccc tgcagaccta cgttacccag cagctgatca gagccgccga gattagagcc 3060

tctgccaatc tggccgccac caagatgtct gagtgtgtgc tgggccagag caagagagtg 3120

gacttttgcg gcaagggcta ccacctgatg agcttccctc agtctgcccc tcacggcgtg 3180

gtgtttctgc acgtgacata cgtgcccgct caagagaaga atttcaccac cgctccagcc 3240

atctgccacg acggcaaagc ccactttcct agagaaggcg tgttcgtgtc caacggcacc 3300

cattggttcg tgacccagcg gaacttctac gagccccaga tcatcaccac cgacaacacc 3360

ttcgtgtctg gcaactgcga cgtcgtgatc ggcattgtga acaataccgt gtacgaccct 3420

ctgcagcccg agctggacag cttcaaagag gaactggata agtactttaa gaaccacaca 3480

agccccgacg tggacctggg cgatatcagc ggaatcaatg ccagcgtcgt gaacatccag 3540

aaagagatcg accggctgaa cgaggtggcc aagaatctga acgagagcct gatcgacctg 3600

caagaactgg ggaagtacga gcagtacatc aagtggccct ggtacatctg gctgggcttt 3660

atcgccggac tgattgccat cgtgatggtc acaatcatgc tgtgttgcat gaccagctgc 3720

tgtagctgcc tgaagggctg ttgtagctgt ggcagctgct gcaagttcga cgaggacgat 3780

tctgagcccg tgctgaaggg cgtgaaactg cactacacct ga 3822

<210> 2
<211> 1273
<212> PRT
<213> Coronaviridae

<220>
<223> SARS-CoV-2 S protein sequence

<400> 2

<210> 3
<211> 228
<212> DNA
<213> Coronaviridae

<220>
<223> SARS-CoV-2 E nucleotide sequence

<400> 3
atgtacagct tcgtgtccga ggaaaccggc acactgatcg tgaacagcgt gctgctgttc      60

ctggccttcg tggtgttcct gctggttacc ctggctatcc tgacagccct gagactgtgc     120

gcctactgct gcaacatcgt gaacgtgtcc ctcgtgaagc ccagcttcta cgtgtacagc     180

agagtgaaga acctgaactc cagcagggtg cccgacctgc tggtctaa                  228

<210> 4
<211> 75
<212> PRT
<213> Coronaviridae

<220>
<223> SARS-CoV-2 E protein sequence

<400> 4
Met Tyr Ser Phe Val Ser Glu Glu Thr Gly Thr Leu Ile Val Asn Ser
1               5                   10                  15
Val Leu Leu Phe Leu Ala Phe Val Val Phe Leu Leu Val Thr Leu Ala
            20                  25                  30
Ile Leu Thr Ala Leu Arg Leu Cys Ala Tyr Cys Cys Asn Ile Val Asn
        35                  40                  45
Val Ser Leu Val Lys Pro Ser Phe Tyr Val Tyr Ser Arg Val Lys Asn
    50                  55                  60
Leu Asn Ser Ser Arg Val Pro Asp Leu Leu Val
65                  70                  75

<210> 5
<211> 669
<212> DNA
<213> Coronaviridae

<220>
<223> SARS-CoV-2 M nucleotide sequence

<400> 5
atggccgata gcaatggcac catcaccgtg gaagaactga agaaactgct ggaacagtgg      60

aacctcgtga tcggcttcct gttcctgacc tggatctgcc tgctgcagtt cgcctacgcc     120

aaccggaaca gattcctgta tatcatcaag ctgatctttc tgtggctgct gtggcccgtg     180

acactggcct gttttgtgct ggccgccgtg tacagaatca actggatcac cggcggaatc 240

gccattgcca tggcttgtct tgtgggcctg atgtggctga gctactttat cgccagcttc 300

cggctgttcg cccggaccag atccatgtgg tccttcaatc ccgagacaaa catcctgctg 360

aacgtgcccc tgcacggcac cattctgaca agacctctgc tggaaagcga gctggttatc 420

ggcgccgtga tcctgagagg acacctgaga attgccggcc accacctggg cagatgcgat 480

atcaaggacc tgcctaaaga aatcacagtg gccaccagca gaaccctgtc ctactataag 540

ctgggcgcct ctcagagagt ggccggcgat agcggatttg ccgcctacag cagataccgg 600

atcggcaact acaagctgaa caccgaccac agctccagca gcgacaatat cgcactgctg 660

gtgcagtga 669

<210> 6
<211> 222
<212> PRT
<213> Coronaviridae

<220>
<223> SARS-CoV-2 M protein sequence

<400> 6
Met Ala Asp Ser Asn Gly Thr Ile Thr Val Glu Glu Leu Lys Lys Leu
1               5                   10                  15
Leu Glu Gln Trp Asn Leu Val Ile Gly Phe Leu Phe Leu Thr Trp Ile
            20                  25                  30
Cys Leu Leu Gln Phe Ala Tyr Ala Asn Arg Asn Arg Phe Leu Tyr Ile
        35                  40                  45
Ile Lys Leu Ile Phe Leu Trp Leu Leu Trp Pro Val Thr Leu Ala Cys
    50                  55                  60
Phe Val Leu Ala Ala Val Tyr Arg Ile Asn Trp Ile Thr Gly Gly Ile
65                  70                  75                  80
Ala Ile Ala Met Ala Cys Leu Val Gly Leu Met Trp Leu Ser Tyr Phe
                85                  90                  95
Ile Ala Ser Phe Arg Leu Phe Ala Arg Thr Arg Ser Met Trp Ser Phe
            100                 105                 110
Asn Pro Glu Thr Asn Ile Leu Leu Asn Val Pro Leu His Gly Thr Ile
        115                 120                 125
Leu Thr Arg Pro Leu Leu Glu Ser Glu Leu Val Ile Gly Ala Val Ile
    130                 135                 140
Leu Arg Gly His Leu Arg Ile Ala Gly His His Leu Gly Arg Cys Asp
145                 150                 155                 160
Ile Lys Asp Leu Pro Lys Glu Ile Thr Val Ala Thr Ser Arg Thr Leu
                165                 170                 175
Ser Tyr Tyr Lys Leu Gly Ala Ser Gln Arg Val Ala Gly Asp Ser Gly
            180                 185                 190
Phe Ala Ala Tyr Ser Arg Tyr Arg Ile Gly Asn Tyr Lys Leu Asn Thr
        195                 200                 205
Asp His Ser Ser Ser Ser Asp Asn Ile Ala Leu Leu Val Gln
    210                 215                 220

<210> 7
<211> 693
<212> DNA
<213> Coronaviridae

<220>
<223> SARS-CoV-2 RBD nucleotide sequence

<400> 7
atggaaaccg acacactgct gctgtgggtg ctgcttttgt gggtgccagg cagcacaggc      60

gattaccccg ggtctagatt ccccaacatc accaatctgt gccccttcgg cgaggtgttc     120

aatgccacaa gatttgccag cgtgtacgcc tggaaccgga agagaatcag caactgcgtg     180

gccgactaca gcgtgctgta caatagcgcc agcttcagca ccttcaagtg ctacggcgtg     240

tcccctacca agctgaacga cctgtgcttc accaatgtgt acgccgacag cttcgtgatc     300

agaggcgacg aagttcggca gatcgctcct ggacagacag gcaagatcgc cgattacaac     360

tacaagctgc ccgacgactt caccggctgc gtgatcgcct ggaatagcaa caacctggac     420

agcaaagtcg gcggcaacta caactacctg taccggctgt tccggaagtc caacctgaag     480

cctttcgagc gggacatcag caccgagatc tatcaggccg gcagcacccc ttgtaatggc     540

gtggaaggct tcaactgcta cttcccactg cagtcctacg gcttccagcc tacaaacggc     600

gtgggctacc agccttatag agtggtggtg ctgagcttcg aactgctgca tgcccctgct     660

accgtgtgtg gccctaagaa gtccaccaac tga                                  693


<210> 8
<211> 230
<212> PRT
<213> Coronaviridae


<220>
<223> SARS-CoV-2 RBD protein sequence

<400> 8
Met Glu Thr Asp Thr Leu Leu Leu Trp Val Leu Leu Leu Trp Val Pro
1               5                   10                  15
Gly Ser Thr Gly Asp Tyr Pro Gly Ser Arg Phe Pro Asn Ile Thr Asn
            20                  25                  30
Leu Cys Pro Phe Gly Glu Val Phe Asn Ala Thr Arg Phe Ala Ser Val
        35                  40                  45
Tyr Ala Trp Asn Arg Lys Arg Ile Ser Asn Cys Val Ala Asp Tyr Ser
    50                  55                  60
Val Leu Tyr Asn Ser Ala Ser Phe Ser Thr Phe Lys Cys Tyr Gly Val
65                  70                  75                  80
Ser Pro Thr Lys Leu Asn Asp Leu Cys Phe Thr Asn Val Tyr Ala Asp
                85                  90                  95
Ser Phe Val Ile Arg Gly Asp Glu Val Arg Gln Ile Ala Pro Gly Gln
            100                 105                 110
Thr Gly Lys Ile Ala Asp Tyr Asn Tyr Lys Leu Pro Asp Asp Phe Thr
        115                 120                 125
Gly Cys Val Ile Ala Trp Asn Ser Asn Asn Leu Asp Ser Lys Val Gly
    130                 135                 140
Gly Asn Tyr Asn Tyr Leu Tyr Arg Leu Phe Arg Lys Ser Asn Leu Lys
145                 150                 155                 160
Pro Phe Glu Arg Asp Ile Ser Thr Glu Ile Tyr Gln Ala Gly Ser Thr
                165                 170                 175
Pro Cys Asn Gly Val Glu Gly Phe Asn Cys Tyr Phe Pro Leu Gln Ser

180                 185                 190
Tyr Gly Phe Gln Pro Thr Asn Gly Val Gly Tyr Gln Pro Tyr Arg Val
        195                 200                 205
Val Val Leu Ser Phe Glu Leu Leu His Ala Pro Ala Thr Val Cys Gly
    210                 215                 220
Pro Lys Lys Ser Thr Asn
225                 230

<210> 9
<211> 1260
<212> DNA
<213> Coronaviridae

<220>
<223> SARS-CoV-2 N nucleotide sequence

<400> 9
atgagcgaca acggccctca gaatcagcgg aacgccccta gaatcacatt tggcggccct      60

agcgatagca ccggcagcaa tcagaatggc gagagaagcg gcgccagaag caaacagaga     120

aggcctcagg gcctgcctaa caacaccgcc tcttggttta cagccctgac acagcacggc     180

aaagaggacc tgaagtttcc tagaggccag ggcgtgccca tcaacaccaa tagcagccct     240

gatgaccaga tcggctacta cagacgggcc accagaagaa ttagaggcgg cgacggcaag     300

atgaaggatc tgagccccag atggtacttc tactacctcg gaacaggacc cgaggccgga     360

cttccttatg gcgccaacaa ggatggcatc atctgggttg caacagaagg cgccctgaac     420

acccctaagg accacatcgg aaccagaaat cccgccaaca atgccgccat tgtgctgcag     480

ttgccacagg gcacaaccct gcctaagggc ttttatgccg agggctctag aggcggaagc     540

caggcctcta gcagaagcag cagcagaagc cggaactcca gcagaaatag cacccctgga     600

agctccagag gcacatctcc tgccagaatg gccggaaatg gcggagatgc tgctcttgct     660

ctgctgctcc tggatcggct gaaccagctg gaaagcaaga tgtctggaaa gggccagcag     720

caacagggcc agaccgtgac aaaaaaatct gccgccgagg ccagcaagaa gcccagacag     780

aagagaaccg ccaccaaggc ctacaacgtg acccaggcct ttggaagaag aggcccagag     840

cagacccagg gcaatttcgg cgatcaagag ctgatcagac agggcaccga ctacaagcac     900

tggcctcaga ttgcccagtt tgcccctagc gcctccgcct tctttggcat gtctagaatc     960

ggcatggaag tgacccctag cggcacctgg ttgacataca caggcgccat caagctggac    1020

gacaaggacc ccaacttcaa ggaccaagtg atcctgctga acaagcacat cgacgcctac    1080

aagacattcc ctccaaccga gcctaagaag gacaagaaga agaaggccga cgagacacag    1140

gccctgccac agagacagaa aaaacagcag acagtgaccc tgctgcctgc cgctgatctg    1200

gacgattttt ccaagcagct gcagcagtcc atgagcagcg cagatagcac acaggcctga    1260

<210> 10
<211> 419

<212> PRT
<213> Coronaviridae

<220>
<223> SARS-CoV-2 N protein sequence

<400> 10
Met Ser Asp Asn Gly Pro Gln Asn Gln Arg Asn Ala Pro Arg Ile Thr
1               5                   10                  15
Phe Gly Gly Pro Ser Asp Ser Thr Gly Ser Asn Gln Asn Gly Glu Arg
            20                  25                  30
Ser Gly Ala Arg Ser Lys Gln Arg Arg Pro Gln Gly Leu Pro Asn Asn
        35                  40                  45
Thr Ala Ser Trp Phe Thr Ala Leu Thr Gln His Gly Lys Glu Asp Leu
    50                  55                  60
Lys Phe Pro Arg Gly Gln Gly Val Pro Ile Asn Thr Asn Ser Ser Pro
65                  70                  75                  80
Asp Asp Gln Ile Gly Tyr Tyr Arg Arg Ala Thr Arg Arg Ile Arg Gly
                85                  90                  95
Gly Asp Gly Lys Met Lys Asp Leu Ser Pro Arg Trp Tyr Phe Tyr Tyr
            100                 105                 110
Leu Gly Thr Gly Pro Glu Ala Gly Leu Pro Tyr Gly Ala Asn Lys Asp
        115                 120                 125
Gly Ile Ile Trp Val Ala Thr Glu Gly Ala Leu Asn Thr Pro Lys Asp
    130                 135                 140
His Ile Gly Thr Arg Asn Pro Ala Asn Asn Ala Ala Ile Val Leu Gln
145                 150                 155                 160
Leu Pro Gln Gly Thr Thr Leu Pro Lys Gly Phe Tyr Ala Glu Gly Ser
                165                 170                 175
Arg Gly Gly Ser Gln Ala Ser Ser Arg Ser Ser Ser Arg Ser Arg Asn
            180                 185                 190
Ser Ser Arg Asn Ser Thr Pro Gly Ser Ser Arg Gly Thr Ser Pro Ala
        195                 200                 205
Arg Met Ala Gly Asn Gly Gly Asp Ala Ala Leu Ala Leu Leu Leu Leu
    210                 215                 220
Asp Arg Leu Asn Gln Leu Glu Ser Lys Met Ser Gly Lys Gly Gln Gln
225                 230                 235                 240
Gln Gln Gly Gln Thr Val Thr Lys Lys Ser Ala Ala Glu Ala Ser Lys
                245                 250                 255
Lys Pro Arg Gln Lys Arg Thr Ala Thr Lys Ala Tyr Asn Val Thr Gln
            260                 265                 270
Ala Phe Gly Arg Arg Gly Pro Glu Gln Thr Gln Gly Asn Phe Gly Asp
        275                 280                 285
Gln Glu Leu Ile Arg Gln Gly Thr Asp Tyr Lys His Trp Pro Gln Ile
    290                 295                 300
Ala Gln Phe Ala Pro Ser Ala Ser Ala Phe Phe Gly Met Ser Arg Ile
305                 310                 315                 320
Gly Met Glu Val Thr Pro Ser Gly Thr Trp Leu Thr Tyr Thr Gly Ala
                325                 330                 335
Ile Lys Leu Asp Asp Lys Asp Pro Asn Phe Lys Asp Gln Val Ile Leu
            340                 345                 350
Leu Asn Lys His Ile Asp Ala Tyr Lys Thr Phe Pro Pro Thr Glu Pro
        355                 360                 365
Lys Lys Asp Lys Lys Lys Lys Ala Asp Glu Thr Gln Ala Leu Pro Gln
    370                 375                 380
Arg Gln Lys Lys Gln Gln Thr Val Thr Leu Leu Pro Ala Ala Asp Leu
385                 390                 395                 400
Asp Asp Phe Ser Lys Gln Leu Gln Gln Ser Met Ser Ser Ala Asp Ser
                405                 410                 415
Thr Gln Ala

<210> 11
<211> 21
<212> DNA
<213> Artificial Sequence

<220>
<223> Myc A (F)

<400> 11
ggcgaatggg tgagtaacac g                                                21

<210> 12
<211> 21
<212> DNA
<213> Artificial Sequence

<220>
<223> Myc B (R)

<400> 12
cggataacgc tgcgacctat g                                                21

<210> 13
<211> 20
<212> DNA
<213> Artificial Sequence

<220>
<223> TK-L (F)

<400> 13
tgattagttt gatgcgattc                                                  20

<210> 14
<211> 17
<212> DNA
<213> Artificial Sequence

<220>
<223> TK-R (R)

<400> 14
tgtccttgat acggcag                                                     17

<210> 15
<211> 36
<212> DNA
<213> Artificial Sequence

<220>
<223> SARS-CoV-2-S-F

<400> 15
agctcgaggc caccatgttc gtgtttctgg tgctgc                                36

<210> 16
<211> 32
<212> DNA
<213> Artificial Sequence

<220>
<223> SARS-CoV-2-S-F1

<400> 16
ggtttcagac actgctggcc ctgcacagaa gc                                    32

<210> 17
<211> 32
<212> DNA
<213> Artificial Sequence

<220>
<223> SARS-CoV-2-S-F2

<400> 17
gcagtcctac ggctttcagc ccacaaatgg cg                                    32

<210> 18
<211> 33
<212> DNA
<213> Artificial Sequence

<220>
<223> SARS-CoV-2-S-R2

<400> 18
atccctgtca gggctctatt cagctgggtg cag                                   33

<210> 19
<211> 36
<212> DNA
<213> Artificial Sequence

<220>
<223> SARS-CoV-2-S-R1

<400> 19
tctggcccag cacacactca gacatcttgg tggcgg                                36

<210> 20
<211> 35
<212> DNA
<213> Artificial Sequence

<220>
<223> SARS-CoV-2-S-R

<400> 20
aggcggccgc tcaggtgtag tgcagtttca cgccc 35

## Description

### TECHNICAL FIELD OF THE INVENTION

The present invention is directed to a recombinant modified vaccinia virus Ankara (MVA), which carries a nucleic acid sequence coding for an antigenic protein useful for vaccinating against SARS-CoV-2. The present invention is further directed to a vaccine composition containing said recombinant MVA as well as to a method for generating a protective immune response in a mammal against SARS-CoV-2.

### BACKGROUND OF THE INVENTION

The emergence of the strain of coronavirus SARS-CoV-2 (severe acute respiratory syndrome coronavirus 2) and its high impact on global health have made imperative the development of safe and effective vaccines for this lethal strain, the causal agent of COVID-19 (coronavirus disease 2019). SARS-CoV-2 now adds to the list of coronavirus diseases that have threatened global health, along with the SARS (severe acute respiratory syndrome) and MERS (Middle East respiratory syndrome) coronaviruses that emerged in 2002/2003 and 2012, respectively. As of June 2020, no vaccine is commercially available for these coronavirus strains. Nevertheless, the knowledge obtained from the vaccine development efforts for SARS and MERS can be of high value for COVID-19.

Here, we provide an effective vaccine, as well as vaccine regimens, against SARS-CoV-2.

### BRIEF DESCRIPTION OF THE FIGURES

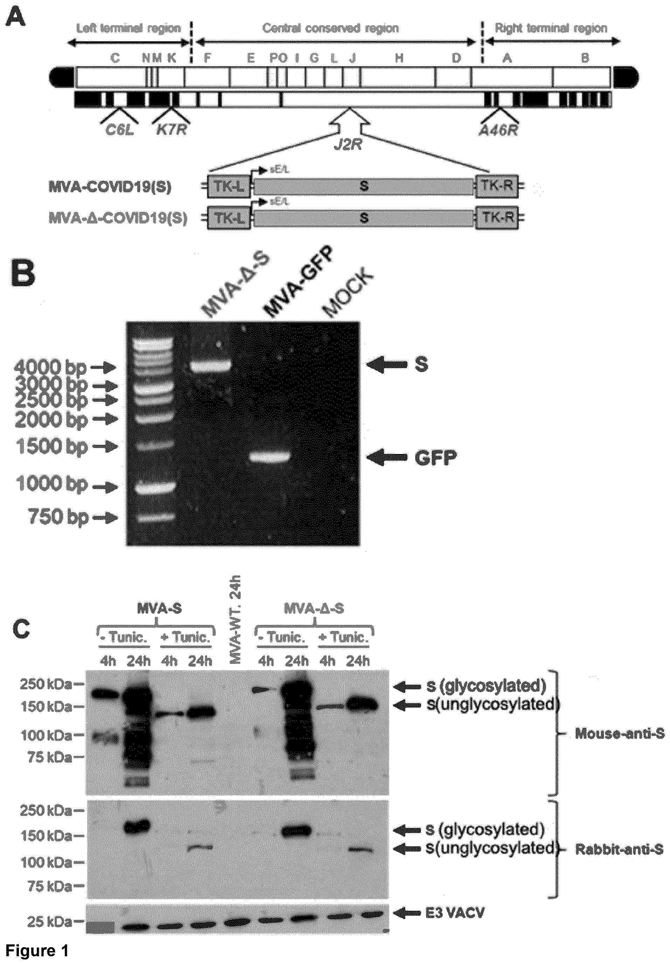

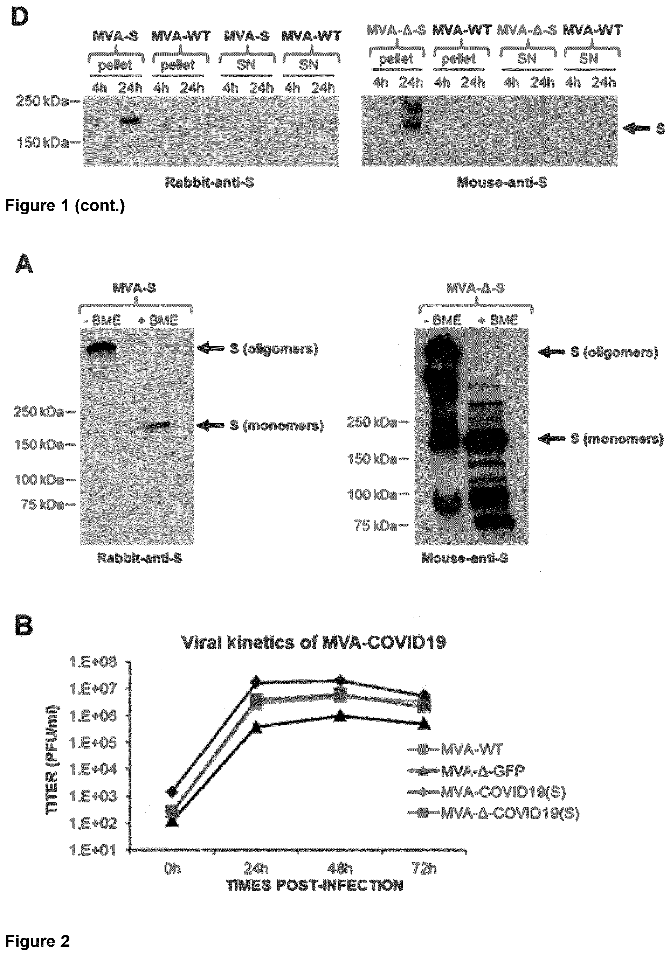

**Figure 1****. Design and generation and in vitro characterization of vaccine candidates MVA-COVID19(S) and MVA-Δ-COVlD19(S). (A)** Genome map of vaccine candidates MVA-COVID19(S) and MVA-Δ-COVID19(S), expressing the coronavirus SARS-CoV-2 S-protein. The different regions of the MVA genome are indicated with capital letters and the central conserved region and the left and right terminal regions are shown. Below the map, the deleted or fragmented MVA genes are depicted as black boxes. The MVA genes *C6L, K7R,* and *A46R* which have been deleted in one of the parental viruses (MVA-GFP), used for generating the vaccine candidate MVA-Δ-COVID19(S), are indicated. The coronavirus SARS-CoV-2 S genes inserted within the MVA TK viral locus (J2R) of MVA-WT or MVA-GFP parental viruses (to generated MVA-COVID19(S) and MVA-Δ-COVID19(S), respectively) and driven by the sE/L virus promoter are indicated. TK-L: TK left; TK-R: TK right. **(B)** PCR analysis of MVA TK locus. Viral DNA was extracted from DF-1 cells that were not infected (mock) or infected with 5 PFU/cell of vaccine candidates MVA-COVID19(S) (referred to as MVA-S) and MVA-Δ-COVID19(S) (referred to as MVA-Δ-S), MVA-GFP, or MVA-WT. Oligonucleotides which hybridize in the flanking regions of the *TK* locus were used for the PCR analysis of the coronavirus SARS-CoV-2 S gene inserted in the *TK* locus. A molecular weight marker (1 kb) with the corresponding bp sizes is indicated on the left side of the images, and the amplified DNA products are indicated with arrows on the right side. **(C)** Expression of the coronavirus SARS-CoV-2 S-protein. DF-1 cells were left uninfected (mock) or infected for 4 or 24 hours (in the absence or presence of tunicamycin) at 5 PFU/cell with vaccine candidates MVA-COVID19(S) and MVA-Δ-COVID19(S) and MVA-WT. At 4 and 24 hours post-infection cells were lysed in Laemmli 1X + β-mercaptoethanol buffer, fractionated by 7% SDS-PAGE, and the presence of the S protein was analyzed by Western blotting using a mouse monoclonal antibody against SARS-CoV (against SARS-CoV S2 region) or a rabbit polyclonal antibody against SARS-CoV-2 (against SARS-CoV-2 S1 region). A rabbit anti-VACV E3 protein antibody was used as a loading control for the amount of viral proteins. The arrows on the right side of the images indicate the S protein (glycosylated or unglycosylated), together with the VACV E3 protein, whereas the sizes of standards (in kilodaltons [kDa]) (Precision Plus protein standards; Bio-Rad Laboratories) are indicated on the left side. **(D)** Kinetics of expression of the coronavirus SARS-CoV-2 S protein in cell extracts (pellet) and supernatants (SN) from cells infected with vaccine candidates MVA-COVID19(S) and MVA-Δ-COVlD19(S). DF-1 cells were left uninfected (mock) or infected with vaccine candidates MVA-COVID19(S) and MVA-Δ-COVID19(S), and MVA-WT, at 5 PFU/cell. The cell extracts and the supernatants were collected 4 and 24 hours post-infection (the supernatants were first precipitated with 10% TCA), lysed in Laemmli 1X + β-mercaptoethanol buffer, fractioned with 7% SDS-PAGE, and analyzed by Western blotting using a mouse monoclonal antibody against SARS-CoV (against SARS-CoV S2 region) or a rabbit polyclonal antibody against SARS-CoV-2 (against SARS-CoV-2 S1 region). The arrows on the right side indicate the S protein. The sizes of standards (in kilodaltons [kDa]) (Precision Plus protein standards; Bio-Rad Laboratories) are indicated on the left.

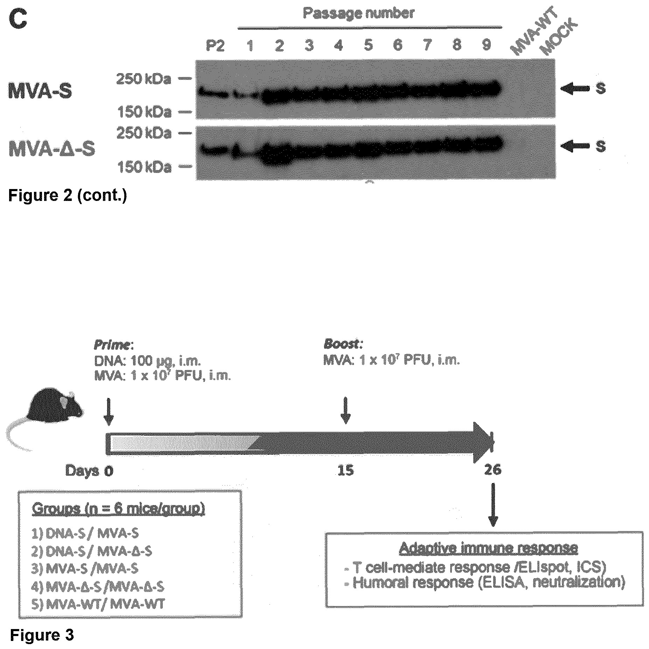

**Figure 2****. *In vitro* characterization of vaccine candidates MVA-COVID19(S) and MVA-Δ-COVlD19(S). (A)** (A) Expression of SARS-CoV-2 S protein under reducing and nonreducing conditions. DF-1 cells were infected with MVA-S and MVA-Δ-S vaccine candidates and at 24 hpi, cells were lysed under reducing (Laemmli 1X + β-mercaptoethanol (BME) buffer) or nonreducing conditions (Laemmli 1X - β-mercaptoethanol buffer), fractionated by 7% SDS-PAGE, and analyzed by Western blotting using a mouse monoclonal antibody against SARS-CoV (against SARS-CoV S2 region) or a rabbit polyclonal antibody against SARS-CoV-2 (against SARS-CoV-2 S1 region). Arrows on the right indicates the SARS-CoV-2 S protein in the form of monomers or oligomers. The sizes of standards (in kilodaltons [kDa]) (Precision Plus protein standards; Bio-Rad Laboratories) are indicated on the left. **(B)** Viral growth kinetics of vaccine candidates MVA-COVID19(S) and MVA-Δ-COVlD19(S). DF-1 cell monolayers were infected with MVA-WT, MVA-GFP, and vaccine candidates MVA-COVID19(S) and MVA-Δ-COVID19(S) at 0.01 PFU/cell. Cells were collected at different times post-infection (0, 24, 48, and 72 hours) and the viral titers in the cell lysates were quantified by a plaque immunostaining assay with anti-VACV antibodies. The means and standard deviations of the results obtained from two independent experiments are shown. **(C)** Genetic stability of vaccine candidates MVA-COVID19(S) and MVA-Δ-COVlD19(S). DF-1 cell monolayers were infected with the P2 stocks of vaccine candidates MVA-COVID19(S) and MVA-Δ-COVID19(S) at a low MOI for 9 serial passages. Then, DF-1 cells were the left uninfected (*mock*) or infected with vaccine candidates MVA-COVID19(S) and MVA-Δ-COVID19(S) from the different passages, or with MVA-WT, and the cells were lysed at 24 hours post-infection in Laemmli 1X + β-mercaptoethanol buffer, fractionated by 7% SDS-PAGE, and analyzed by Western Blotting using a rabbit polyclonal antibody against SARS-CoV-2 (against SARS-CoV-2 S1 region). The arrows on the right side indicate the position of the S protein. The sizes of standards (in kilodaltons [kDa]) (Precision Plus protein standards; Bio-Rad Laboratories) are indicated on the left.
**Figure 3****. Immunization schedule of the heterologous (DNA/MVA) and homologous (MVA/MVA) prime/boost immunization protocols performed in C57BL/6 mice immunized with vaccine candidates MVA-COVID19(S) or MVA-Δ-COVlD19(S).** Six C57BL/6 mice per group were inoculated at days 0 and 15 (prime/boost) with the different indicated vaccine candidates (either DNA-S or MVAs in the prime or the different MVAs in the boost). Immunization groups are indicated (n=6 mice per group), together with the time points at which animals were immunized (dose and route of administration are shown) and sacrificed to analyze the adaptive SARS-CoV-2-specific T cell and humoral immune responses. The mice were sacrificed 11 days after the boost (day 26) and the following was obtained: i) spleens of the mice for studying adaptive T cell-mediated immune responses against SARS-CoV-2 by means of ELISpot and ICS, and ii) serum samples originating from blood for studying adaptive humoral immune responses against SARS-CoV-2, by means of ELISA and a neutralization assay. PFU: Plaque forming units; i.m.: intramuscular.

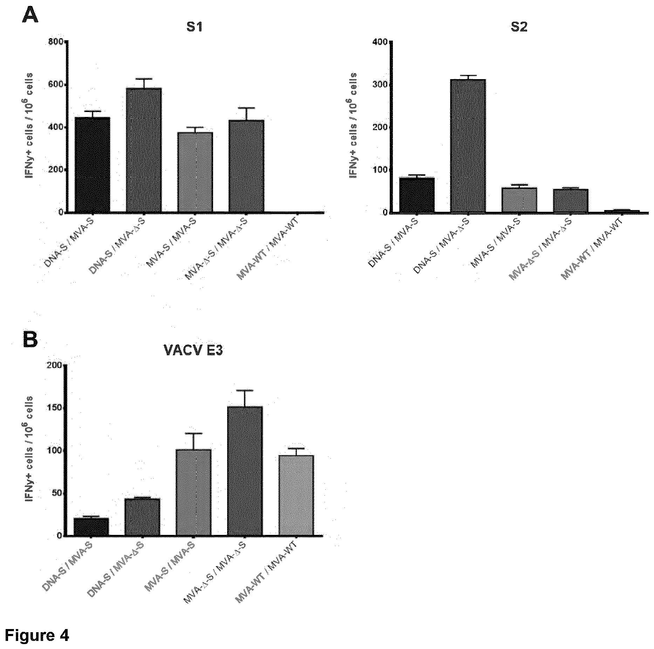

**Figure 4****. ELISpot analysis of the adaptive SARS-CoV-2 S-specific cell-mediated immune responses induced by vaccine candidates MVA-COVID19(S) or MVA-Δ-COVID19(S) in C57BL/6 mice immunized with a heterologous (DNA/MVA) or homologous (MVA/MVA) prime-boost immunization protocol.** Six mice per group were sacrificed at 11 days post-boost, and the splenic SARS-CoV-2-specific cells secreting IFN-γ were evaluated by an ELISpot assay, as described in Materials and Methods. Samples from each group of mice were analysed in triplicate, and the bars indicate the mean values and the standard deviation. The responses obtained have been obtained by subtracting the RPMI values for each condition and are represented as IFN-γ positive cells per million of splenocytes. **(A)** Magnitude of SARS-CoV-2-specific cells secreting IFN-γ and directed against SARS-CoV-2 S1 and S2 peptide pools. **(B)** Magnitude of VACV E3-specific cells secreting IFN-γ.

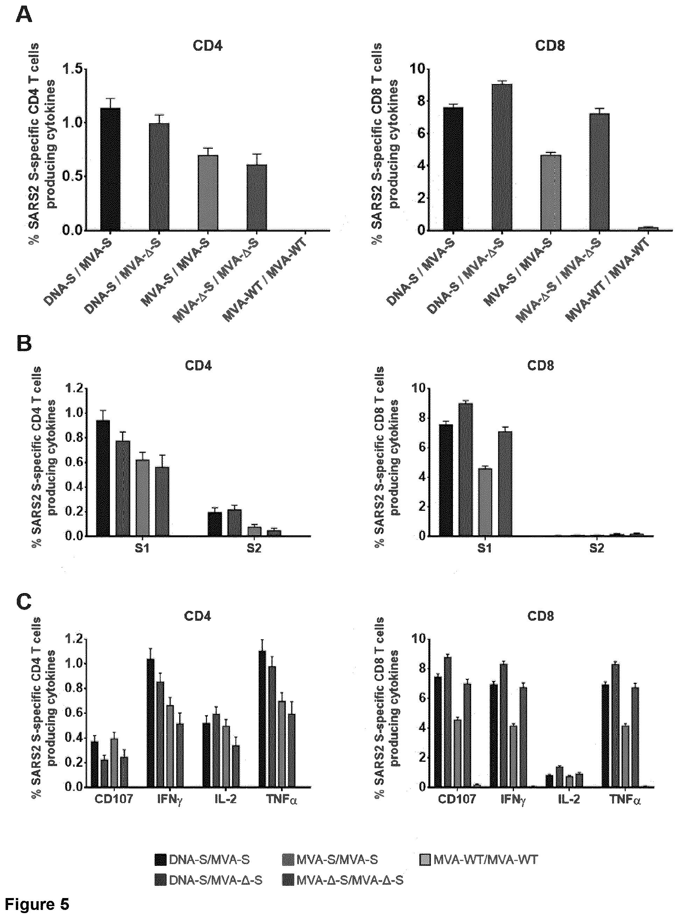

**Figure 5****. SARS-CoV-2 S-specific adaptive CD4⁺ (left) and CD8⁺ (right) T cell-mediated immune responses induced by vaccine candidates MVA-COVID19(S) or MVA-Δ-COVID19(S) in C57BL/6 mice immunized with a heterologous (DNA/MVA) or homologous (MVA/MVA) prime-boost immunization protocol.** CD4⁺ and CD8⁺ T cell-mediated responses in the splenocytes of mice (n=6) immunized with a heterologous (DNA/MVA) or homologous (MVA/MVA) prime-boost protocol were analysed by ICS, as described in Materials and Methods 11 days after the boost. The data is depicted with its corresponding confidence intervals. **(A)** The total magnitude of SARS-CoV-2 S (S1 + S2)-specific adaptive CD4⁺ T cell-mediated (left) and CD8⁺ T cell-mediated (right) immune responses. The y-axis shows the percentage measured as the sum of SARS-CoV-2 S (mixture of S1 + S2 peptide pools)-specific T cells expressing CD107a and/or producing IFN-γ and/or TNF-α and/or IL-2. **(B)** Breadth of SARS-CoV-2-specific CD4⁺ and CD8⁺ T cell adaptive immune responses. S1 or S2 peptide pool-specific adaptive CD4⁺ T cell-mediated (left) and CD8⁺ T cell-mediated (right) immune responses. The y-axis shows the percentage measured as the sum of SARS-CoV-2 S1 or S2 peptide pool-specific T cells expressing CD107a and/or producing IFN-γ and/or TNF-α and/or IL-2. **(C)** Adaptive CD4⁺ T cell-mediated (left) and CD8⁺ T cell-mediated (right) immune responses shown as the percentage of SARS-CoV-2 S (S1 + S2)-specific cells expressing CD107a or producing IFN-γ or TNF-α or IL-2.

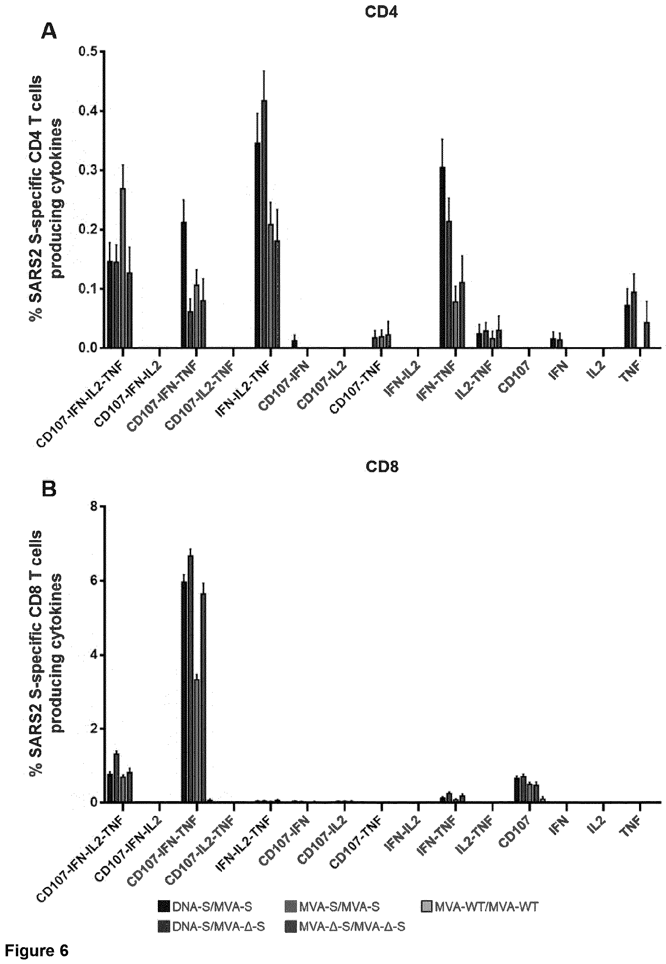

**Figure 6****. Polyfunctionality of SARS-CoV-2 S-specific adaptive CD4⁺ and CD8⁺ T cell-mediated immune responses induced by vaccine candidates MVA-COVID19(S) or MVA-Δ-COVID19(S) in C57BL/6 mice immunized with a heterologous (DNA/MVA) or homologous (MVA/MVA) prime-boost immunization protocol.** Six mice per group were sacrificed at 11 days post-boost, and the splenic SARS-CoV-2-specific CD4⁺ and CD8⁺ T cell immune responses were analysed by ICS, as described in Materials and Methods. Polyfunctional profiles of total SARS-CoV-2-specific CD4⁺ **(A)** and CD8⁺ **(B)** T cell adaptive immune responses directed against a mixture of S1, and S2 SARS-CoV-2 S peptide pools. All of the possible combinations of responses are shown on the x axis, while the percentages of T cells expressing CD107a and/or IFN-γ and/or TNF-α and/or IL-2 against S1 plus S2 peptide pools are shown on the y axis.

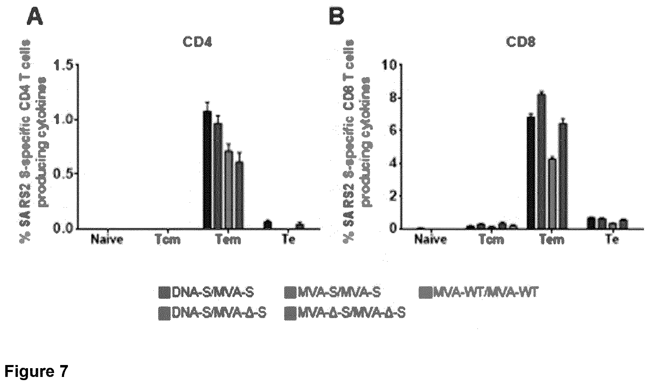

**Figure 7****. Phenotype profile of SARS-CoV-2 S-specific adaptive CD4⁺ and CD8⁺ T cells induced by vaccine candidates MVA-COVID19(S) or MVA-Δ-COVID19(S), in C57BL/6 mice immunized with a heterologous (DNA/MVA) or homologous (MVA/MVA) prime-boost immunization protocol.** Splenocytes of mice (n=6) immunized with a heterologous (DNA/MVA) or homologous (MVA/MVA) prime-boost protocol were obtained 11 days after the boost, and SARS-CoV-2 S (S1 +S2 peptide pools)-specific CD4⁺ T cell-mediated **(A)** and CD8⁺ T cell-mediated **(B)** immune responses were analysed by ICS. The y-axis indicates the percentage of SARS-CoV-2 S (S1 +S2)-specific CD4⁺ and CD8⁺ central memory T cells (TCM; CD127⁺, CD62L⁺), effector memory T cells (TEM; CD127⁺, CD62L⁻), and effector T cells (TE; CD127⁻, CD62L⁻) expressing CD107a and/or IFN-γ and/or TNF-α and/or IL-2 (total response). The data is depicted with its corresponding confidence intervals.

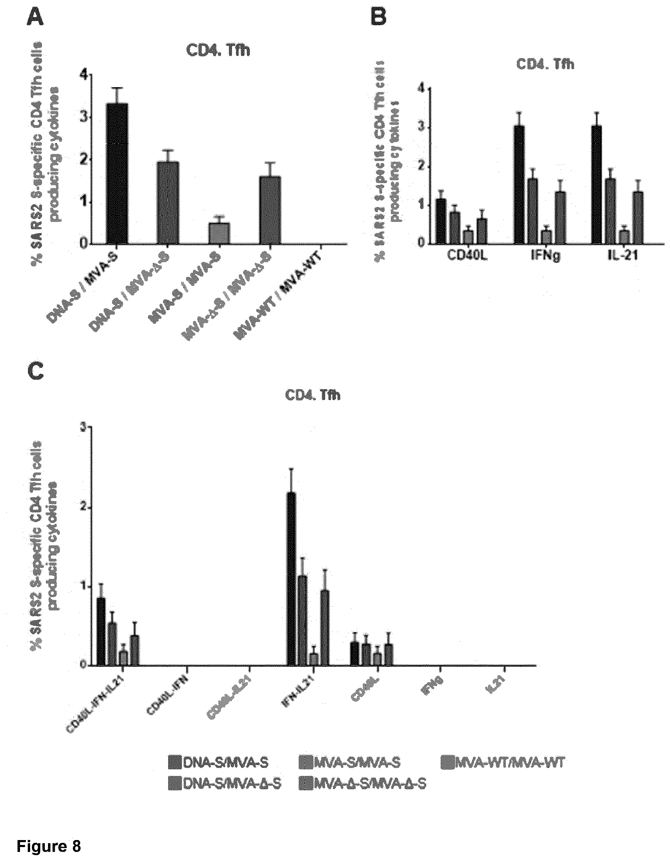

**Figure 8****. SARS-CoV-2 S-specific adaptive CD4⁺ T follicular helper (Tfh) cell-mediated immune responses induced by vaccine candidates MVA-COVID19(S) or MVA-Δ-COVID19(S), in C57BL/6 mice immunized with a heterologous (DNA/MVA) or homologous (MVA/MVA) prime-boost immunization protocol.** Splenocytes of mice (n=6) immunized with a heterologous (DNA/MVA) or homologous (MVA/MVA) prime-boost protocol were obtained 11 days after the boost, and SARS-CoV-2 S (S protein plus S1 +S2 peptide pools)-specific CD4⁺ Tfh cell-mediated immune responses were analysed by ICS. The data is depicted with its corresponding confidence intervals. **(A)** Magnitude of total SARS-CoV-2-specific adaptive CD4+ Tfh cell immune responses directed against SARS-CoV-2 S protein plus S1 and S2 peptide pools. Percentages of CD4+ Tfh cells (CXCR5+, PD1+) expressing CD40L and/or producing IFN-γ and/or IL-21 against a mixture of SARS-CoV-2 S protein plus S1, and S2 SARS-CoV-2 S peptide pools are represented. **(B)** Percentages of total SARS-CoV-2-specific CD4+ Tfh cells expressing CD40L or producing IFN-γ or IL-21 against a mixture of SARS-CoV-2 S protein plus S1, and S2 SARS-CoV-2 S peptide pools. **(C)** Polyfunctionality of total SARS-CoV-2-specific adaptive CD4+ Tfh cells directed against a mixture of SARS-CoV-2 S protein plus S1 and S2 peptide pools. All of the possible combinations of responses are shown on the x axis, while the percentages of T cells expressing CD40L and/or IFN-γ and/or IL-21 against SARS-CoV-2 S protein plus S1 and S2 peptide pools are shown on the y axis.

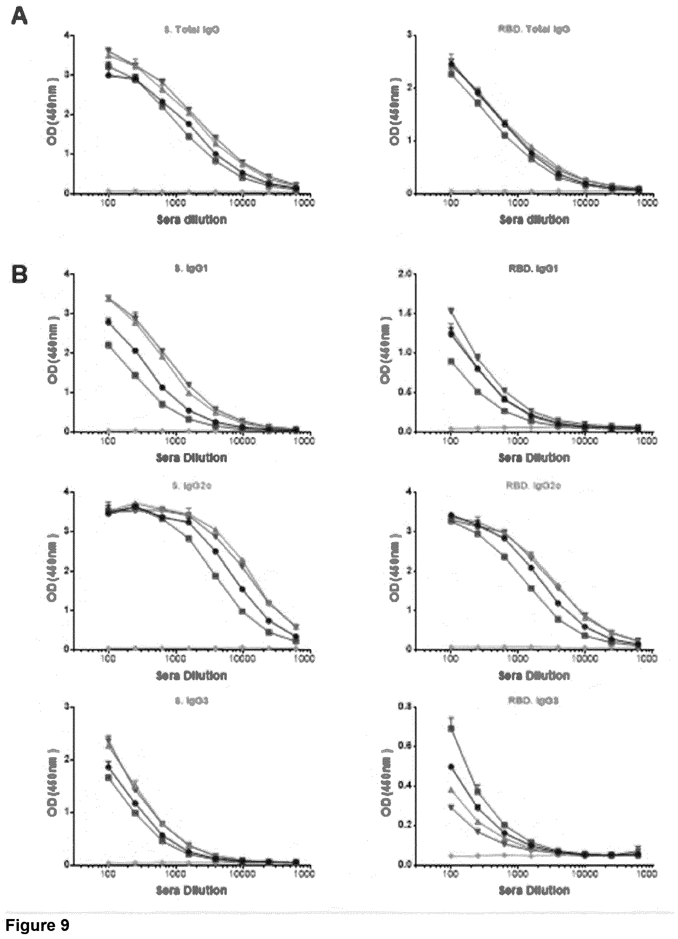

**Figure 9****. SARS-CoV-2 S-specific and RBD-specific humoral immune responses induced by vaccine candidates MVA-COVID19(S) or MVA-Δ-COVID19(S) in C57BL/6 mice immunized with a heterologous (DNA/MVA) or homologous (MVA/MVA) prime-boost immunization protocol.** Groups of C57BL/6 mice (n=6) were immunized with a heterologous (DNA/MVA) or homologous (MVA/MVA) prime-boost protocol. Eleven (11) days after the last immunization, the mice were sacrificed, serum samples were collected, and the levels of total coronavirus SARS-CoV-2 S-specific and RBD-specific IgG antibodies **(A)** or antibodies of subclasses IgG1, IgG2c, and IgG3 **(B)** in pooled sera from mice derived from each immunization group were analyzed by ELISA. Mean absorbance values (measured at 450 nm) and standard deviations of duplicate pooled serum dilutions are represented.

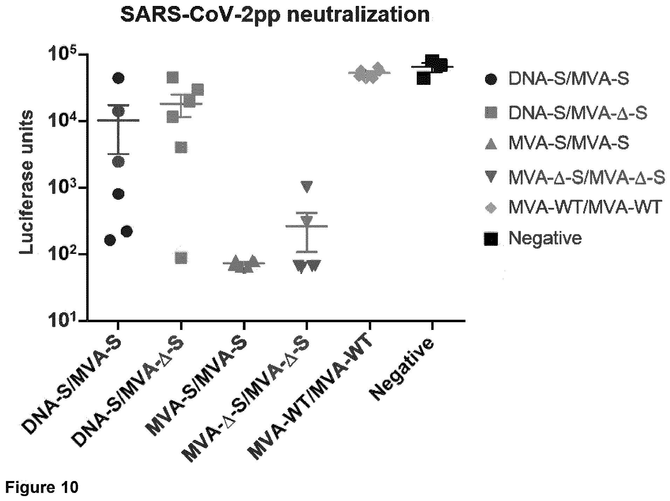

**Figure 10****. Levels of SARS-CoV-2-specific neutralizing antibodies induced by vaccine candidates MVA-COVID19(S) or MVA-Δ-COVID19(S) in C57BL/6 mice immunized with a heterologous (DNA/MVA) or homologous (MVA/MVA) prime-boost immunization protocol.** Groups of C57BL/6 mice (n=6) were immunized with a heterologous (DNA/MVA) or homologous (MVA/MVA) prime-boost protocol. Eleven (11) days after the last immunization, the mice were sacrificed, serum samples were collected, and their capacity to inhibit the entry in VeroE6 cells of lentivirus-based pseudoparticles expressing the coronavirus SARS-CoV-2 S protein was analyzed. RPMI was used as a negative control. Each dot represents the mean luciferase units of triplicates obtained for each individual mouse serum sample from each immunization group, and analyzed at a 1/50 dilution. Mean values and SEM obtained for each immunization group is represented.

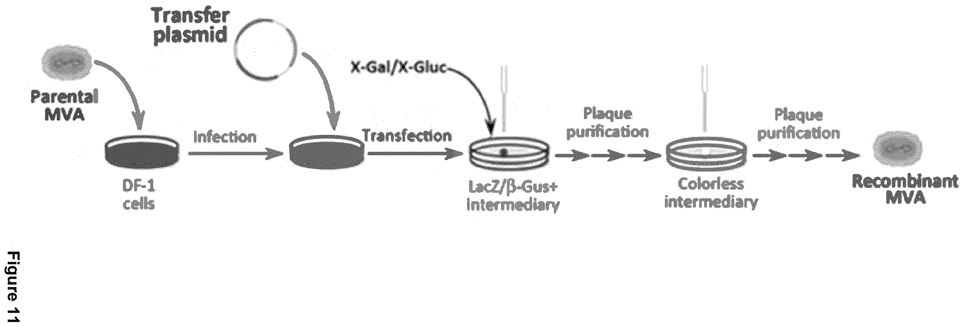

**Figure 11****. Generation of recombinant MVA viruses.** After infection-transfection in DF-1 cells, three rounds of purification were performed selecting blue plaques (LacZ+), followed by another three rounds selecting colorless plaques (without a marker) to finally generate the recombinant MVA virus.

### DESCRIPTION OF THE INVENTION

### General definitions

It must be noted that, as used herein, the singular forms "a", "an", and "the", include plural references unless the context clearly indicates otherwise. Thus, for example, reference to "an antigenic determinant" includes one or more antigenic determinants and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Several documents are cited throughout the text of this specification. Each of the documents cited herein (including all patents, patent applications, scientific publications, manufacturer's specifications, instructions, etc.), whether supra or infra, are hereby incorporated by reference in their entirety. To the extent the material incorporated by reference contradicts or is inconsistent with this specification, the specification will supersede any such material. Nothing herein is to be construed as an admission that the invention is not entitled to antedate such disclosure by virtue of prior invention.

The term "subtype" herein can be replaced with "species". It includes strains, isolates, clades or variants of any SARS-CoV-2. The terms "strain" "clade" or "isolate" are technical terms, well known to the practitioner, referring to the taxonomy of microorganisms. The taxonomic system classifies all so far characterised microorganisms into the hierarchic order of Families, Genera, Species, Strains (Fields Virology, ed. by Fields B. N., Lippincott-Raven Publishers, 4th edition 2001). While the criteria for the members of a Family is their phylogenetic relationship, a Genera comprises all members which share common characteristics, and a Species is defined as a polythetic class that constitutes a replicating lineage and occupies a particular ecological niche. The term "strain" or "clade" describes a microorganism, i.e., virus, which shares common characteristics, like basic morphology or genome structure and organization, but varies in biological properties, like host range, tissue tropism, geographic distribution, attenuation or pathogenicity.

The term "subject" as used herein is a living multi-cellular vertebrate organisms, including, for example, humans, non-human mammals and (non-human) primates. The term "subject" may be used interchangeably with the term "animal" herein.

As used herein, the term "enhanced" when used with respect to an immune response against SARS-CoV-2, such as an antibody response (e.g., neutralizing antigen specific antibody response), a cytokine response or a CD8 T cell response (e.g., immunodominant CD8 T cell response), refers to an increase in the immune response in an animal administered with a homologous prime-boost combination vaccine of recombinant MVA relative to the corresponding immune response observed from the animal administered with a homologous prime-boost combination vaccine of MVA vectors, wherein the MVA vectors do not express any SARS-CoV-2 protein or refers to an increase in the immune response in an animal administered with a heterologous prime-boost combination vaccine of recombinant DNA and MVA vectors according to the invention, relative to the corresponding immune response observed from the animal administered with a heterologous prime-boost combination vaccine of DNA and MVA vectors according to the invention, wherein the MVA vector does not express any SARS-CoV-2 protein.

"Modified vaccinia virus Ankara (MVA) vector" as used herein is an attenuated replication-deficient vaccinia virus that has been genetically modified to include in its genome foreign DNA (that is, DNA that does not naturally belong to poxviruses). In particular, the modified vaccinia virus Ankara vector of the present invention has been genetically modified to express SARS-CoV-2 protein(s). Thus, the modified vaccinia virus Ankara vector of the present invention refers to viral particles of MVA that comprise inside the viral particle the MVA genome, wherein at least one nucleic acid encoding an antigenic protein of at least one SARS-CoV-2 virus subtype has been introduced in the MVA genome.

### Recombinant MVA virus vaccine compositions

Vaccinia virus (VACV) is amongst the most extensively evaluated live vectors and has particular features in support of its use as recombinant vaccine: It is highly stable, cheap to manufacture, easy to administer, and it can accommodate large amounts of foreign DNA. It has the advantage of inducing both antibody and cytotoxic T cell responses and allows presentation of antigens to the immune system in a more natural way, and it was successfully used as vector vaccine protecting against several infectious diseases in a broad variety of animal models. Additionally, vaccinia vectors are extremely valuable research tools to analyse structure-function relationships of recombinant proteins, determine targets of humoral and cell- mediated immune responses, and investigate the type of immune parameters needed to protect against a specific disease.

However, VACV is infectious for humans and its use as expression vector in the laboratory has been affected by safety concerns and regulations. Furthermore, possible future applications of recombinant VACV e.g. to generate recombinant proteins or recombinant viral particles for novel therapeutic or prophylactic approaches in humans, are hindered by the productive replication of the recombinant VACV vector. Most of the recombinant VACVs described in the literature are based on the Western Reserve (WR) strain of VV. On the other hand, it is known that this strain is highly neurovirulent and is thus poorly suited for use in humans and animals (Morita et al., Vaccine 5, 65-70 [1987]).

Concerns with the safety of standard strains of VACV have been addressed by the development of VACV vectors from highly attenuated virus strains which are characterized by their restricted replicative capacity in vitro and their avirulence in vivo. Strains of viruses specially cultured to avoid undesired side effects have been known for a long time. Thus, it has been possible, by long-term serial passages of the chorioallantoic VACV Ankara (CVA) strain on chicken embryo fibroblasts, to culture MVA (for review see Mayr, A., Hochstein-Mintzel, V. and Stickl, H. (1975) Infection 3, 6-14; Swiss Patent No. 568 392). The MVA virus was deposited in compliance with the requirements of the Budapest Treaty at CNCM (Institut Pasteur, Collectione Nationale de Cultures de Microorganisms, 25, rue de Docteur Roux, 75724 Paris Cedex 15) on Dec. 15, 1987 under Depositary No. 1-721.

The MVA virus has been analysed to determine alterations in the genome relative to the wild type CVA strain. Six major deletions (deletion I, II, III, IV, V, and VI) have been identified (Meyer, H., Sutter, G. and Mayr A. (1991) J. Gen. Virol. 72, 1031-1038). This MVA has only low virulence, that is to say it is followed by no side effects when used for vaccination. Hence it is particularly suitable for the initial vaccination of immunocompromised subjects. The excellent properties of the MVA strain have been demonstrated in a number of clinical trials (Mayr et al., Zbl. Bakt. Hyg. I, Abt. Org. B 167, 375-390 [1987], Stickl et al., Dtsch. med. Wschr. 99, 2386-2392 [1974]). Thus, MVA is a valuable tool as safe viral vector for expression of recombinant genes and can be used for such different purposes as the in vitro study of protein functions or the in vivo induction of antigen-specific cellular or humoral immune responses. A major advantage of MVA is to allow for high level gene expression despite being replication defective in human and most mammalian cells. MVA as a vaccine has an excellent safety track-record, can be handled under biosafety level 1 conditions and has proven to be immunogenic and protective when delivering heterologous antigens in animals, and first human candidate vaccines have proceeded into clinical trials.

Although unable to multiply in most mammalian cell lines, MVA retains its genome plasticity that allows the insertion of large amounts of foreign DNA (heterologous genes) (Sutter and Moss, 1992). Absence of pathogenicity for humans, inherent avirulence even in immunocompromised hosts, high-level expression of foreign antigens and adjuvant effect for immune responses make recombinant MVA (rMVA), expressing heterologous genes, an ideal vector for both prophylactic and therapeutic vaccination, as demonstrated by the wide use in prime-boost immunization strategies

Indeed, recombinant MVA (rMVA)-based vaccines comprising heterologous genes and therefore expressing heterologous proteins are able to elicit both humoral and cell-mediated adaptive immune responses (Ramirez et al., 2000) and have been proved to be protective in animal models of several infectious diseases (García-Arriaza et al., 2014, Journal of Virology (Chikungunya) Perez et al., 2018, Scientific Reports (Zika) Lázaro-Frías et al., 2018 (Ebola) Gómez et al., 2013; Marín et al., 2019, Journal of Virology (Hepatitis C)).

Standard procedures to generate recombinant VACV (rVACV) have been described in detail (Earl et al., 1998) and rely on in vivo homologous recombination between acceptor VACV DNA and the transfected transfer plasmid, in which the foreign gene is flanked by VACV sequences. Additional approaches for the generation of recombinant MVA are, for example, disclosed in WO 04074493, WO 03023040 and WO 9702355.

An important question is thus whether rMVA-based vaccines would or not be capable of generating a strong immunogenic and protective immune response against the SARS-CoV-2 virus in a subject in need thereof. In this regard, the authors of the present invention have found and demonstrated that a vaccine comprising:
- an immunologically effective amount of a recombinant modified vaccinia virus Ankara (MVA) vector comprising at least a nucleic acid encoding an antigenic protein, or an antigenic fragment thereof, of at least one SARS-CoV-2 virus subtype, preferably the structural protein S of the SARS-CoV-2 virus, optionally together with a pharmaceutically acceptable carrier, diluent and/or additive (from hereinafter such vaccine candidate shall be referred to as MVA-COVID19); and/or
- an immunologically effective amount of such a modified vaccinia virus Ankara (MVA) vector further modified by deletion or inactivation of the MVA immunomodulatory genes C6L, K7R, and A46R, comprising at least a nucleic acid encoding an antigenic protein, or an antigenic fragment thereof, of at least one SARS-CoV-2 virus subtype, preferably the structural protein S of the SARS-CoV-2 virus, optionally together with a pharmaceutically acceptable carrier, diluent and/or additive (from hereinafter such vaccine candidate shall be referred to as MVA-Δ-COVID19),
provides a SARS-CoV-2 vaccine capable of generating a strong immunogenic and protective immune response against the SARS-CoV-2 virus in a subject in need thereof.

As used herein, term "antigenic protein" or "antigenic determinant" refers to any molecule that stimulates a host's immune system to make an antigen-specific immune response, whether a cellular response or a humoral antibody response. Antigenic determinants may include proteins, polypeptides, antigenic protein fragments, antigens, and epitopes which still elicit an immune response in a host and form part of an antigen, homologues or variants of proteins, polypeptides, and antigenic protein fragments, antigens and epitopes including, for example, glycosylated proteins, polypeptides, antigenic protein fragments, antigens and epitopes, and nucleotide sequences encoding such molecules. Thus, proteins, polypeptides, antigenic protein fragments, antigens and epitopes are not limited to particular native nucleotide or amino acid sequences but encompass sequences identical to the native sequence as well as modifications to the native sequence, such as deletions, additions, insertions and substitutions.

The term "epitope" refers to a site on an antigen to which B- and/or T-cells respond, either alone or in conjunction with another protein such as, for example, a major histocompatibility complex ("MHC") protein or a T-cell receptor. Epitopes can be formed both from contiguous amino acids or noncontiguous amino acids juxtaposed by secondary and/or tertiary folding of a protein. Epitopes formed from contiguous amino acids are typically retained on exposure to denaturing solvents, while epitopes formed by tertiary folding are typically lost on treatment with denaturing solvents. An epitope typically includes at least 5, 6, 7, 8, 9, 10 or more amino acids - but generally less than 20 amino acids - in a unique spatial conformation. Methods of determining spatial conformation of epitopes include, for example, x-ray crystallography and 2-dimensional nuclear magnetic resonance. See, e.g., "Epitope Mapping Protocols" in Methods in Molecular Biology, Vol. 66, Glenn E. Morris, Ed (1996).

Preferably, a homologue or variant has at least about 50%, at least about 60% or 65%, at least about 70% or 75%, at least about 80%, 81 %, 82%, 83%, 84%, 85%, 86%, 87%, 88%, or 89%, more typically, at least about 90%, 91 %, 92%, 93%, or 94% and even more typically at least about 95%, 96%, 97%, 98% or 99%, most typically, at least about 99% identity with the referenced protein, polypeptide, antigenic protein fragment, antigen and epitope at the level of nucleotide or amino acid sequence.

Techniques for determining sequence identity between nucleic acids and amino acids are known in the art. Two or more sequences can be compared by determining their "percent identity." The percent identity of two sequences, whether nucleic acid or amino acid sequences, is the number of exact matches between two aligned sequences divided by the length of the shorter sequences and multiplied by 100.

"Percent (%) amino acid sequence identity" with respect to proteins, polypeptides, antigenic protein fragments, antigens and epitopes described herein is defined as the percentage of amino acid residues in a candidate sequence that are identical with the amino acid residues in the reference sequence (i.e., the protein, polypeptide, antigenic protein fragment, antigen or epitope from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publically available computer software such as BLAST, ALIGN, or Megalign (DNASTAR) software. Those skilled in the art can determine appropriate parameters for measuring alignment, including any algorithms needed to achieve maximum alignment over the full-length of the sequences being compared.

The same applies to "percent (%) nucleotide sequence identity", mutatis mutandis.

For example, an appropriate alignment for nucleic acid sequences is provided by the local homology algorithm of Smith and Waterman, (1981), Advances in Applied Mathematics 2:482-489. This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov (1986), Nucl. Acids Res. 14(6):6745-6763. An exemplary implementation of this algorithm to determine percent identity of a sequence is provided by the Genetics Computer Group (Madison, Wis.) in the "BestFit" utility application. The default parameters for this method are described in the Wisconsin Sequence Analysis Package Program Manual, Version 8 (1995) (available from Genetics Computer Group, Madison, Wis.). A preferred method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, California). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+ GenBank CDS translations+Swiss protein+Spupdate+PIR. Details of these programs can be found at the following internet address: http://wvw.ncbi.nlm.gov/cqi-bin/BLAST.

In the context of the present invention, the heterologous nucleic acid of any of the vaccine compositions described herein may also optionally encode antigenic domains or antigenic protein fragments rather than the entire antigenic protein. These fragments can be of any length sufficient to be antigenic or immunogenic. Fragments can be at least 8 amino acids long, preferably 10-20 amino acids, but can be longer, such as, e.g., at least 50, 100, 200, 500, 600, 800, 1000, 1200, 1600, 2000 amino acids long, or any length in between. In some embodiments, at least one nucleic acid fragment encoding an antigenic protein fragment or immunogenic polypeptide thereof is inserted into the genome of the recombinant viral vector of the invention. In another embodiment, about 2-6 different nucleic acids encoding different antigenic proteins are inserted into the genome of one or more of the recombinant viral vectors. In some embodiments, multiple immunogenic fragments or subunits of various proteins can be used. For example, several different epitopes from different sites of a single protein or from different proteins of the same SARS-CoV-2 strain, or from a protein orthologue from different strains can be expressed from the vectors.

It is herein thus noted that, as described above, vaccine candidates MVA-COVID19 and MVA-Δ-COVID19, may comprise any nucleic acid encoding an antigenic protein, or an antigenic fragment thereof, of at least one SARS-CoV-2 virus subtype; wherein such antigenic proteins are preferably selected from the list consisting of structural proteins S (spike), E (envelope), M (membrane), RBD (receptor binding domain) and/or N (nucleocapsid) of SARS-CoV-2, preferably such antigenic proteins are those selected from any of SEQ ID NO 2, SEQ ID NO 4, SEQ ID NO 6, SEQ ID NO 8 and SEQ ID NO 10, preferably, respectively, encoded by SEQ ID No 1, SEQ ID NO 3, SEQ ID NO 5, SEQ ID NO 7 and SEQ ID NO 9.

More particularly, the present inventors have found that a vaccine comprising:
- an immunologically effective amount of a recombinant modified vaccinia virus Ankara (MVA) vector comprising at least a nucleic acid encoding the structural protein S of the SARS-CoV-2 virus, or an antigenic fragment thereof, preferably, optionally together with a pharmaceutically acceptable carrier, diluent and/or additive (from hereinafter such vaccine candidate shall be referred to as MVA-COVID19(S)); and/or
- an immunologically effective amount of such a modified vaccinia virus Ankara (MVA) vector further modified by deletion or inactivation of the MVA immunomodulatory genes C6L, K7R, and A46R, comprising at least a nucleic acid encoding the structural protein S of the SARS-CoV-2 virus, or an antigenic fragment thereof, preferably, optionally together with a pharmaceutically acceptable carrier, diluent and/or additive (from hereinafter such vaccine candidate shall be referred to as MVA-Δ-COVID19(S)),
provides a SARS-CoV-2 vaccine capable of generating a remarkably strong immunogenic immune response against the SARS-CoV-2 virus in a subject in need thereof. In particular, and as illustrated in the figures of the present invention (i.e. figures 5, 6, 7, 8, 9 and 10), vaccine candidates comprising priming and/or boosting compositions which in turn comprise MVA-COVID19(S) or MVA-Δ-COVID19(S), do not only induced high levels of total IgG, IgG1, IgG2c, and IgG3 antibodies against SARS-CoV-2 S- and RBD proteins, with a Th1 response (Figure 9), but the results showed that individual serum samples obtained from all mice vaccinated with vaccine compositions MVA-COVID19(S) or MVA-Δ-COVID19(S) neutralized SARS-CoV-2 pseudoparticles, significantly reducing the levels of luciferase units, compared to serum from the control group MVA-WT/MVA-WT, where no neutralization was observed (Figure 10). Moreover, vaccine candidates comprising priming and/or boosting vaccine compositions MVA-COVID19(S) or MVA-Δ-COVID19(S), are capable of inducing broad and polyfunctional coronavirus SARS-CoV-2 S-protein-specific adaptive CD4+ and CD8+ T cell-mediated immune responses of great magnitude (Figures 5, 6, 7 and 8).

Thus, a recombinant MVA vector comprising in its genome a nucleotide sequence encoding an antigenic determinant of SARS-CoV-2, preferably encoding the structural protein S of the SARS-CoV-2 virus, reveals very good and specific immune responses against SARS-CoV-2 strains. Of course, from the data generated by the present inventors and their observations, it is more than reasonable and plausible to conclude that the MVA-based vaccine would also induce a protective efficacy in animal models and will be able to generate immunogenic and effective immune responses in humans.

In addition, in the study underlying the present invention it has also been found that the use of vaccine combination comprising:
a. a first composition comprising an immunologically effective amount of a MVA-COVID19 or MVA-Δ-COVID19 vaccine composition, preferably MVA-COVID19(S) or MVA-Δ-COVID19(S); and
b. a second composition comprising an immunologically effective amount of a MVA-COVID19 or MVA-Δ-COVID19 vaccine composition, preferably MVA-COVID19(S) or MVA-Δ-COVID19(S);
as a homologous prime and boost generates a strong protective immune response against a SARS-CoV-2 immunogen. Such response is particularly strong when both compositions comprises an immunologically effective amount of a MVA-COVID19 or MVA-Δ-COVID19 composition comprising at least a nucleic acid encoding the S antigenic protein of at least one SARS-CoV-2 virus subtype, preferably encoding for the S antigenic protein of SEQ ID NO 2 (please refer to the examples and figures).

Furthermore, in the study underlying the present invention it has been further found that the use of vaccine combination comprising:
a. a first composition comprising an immunologically effective amount of a polynucleotide plasmid that comprises at least one nucleic acid encoding an antigenic protein of at least one SARS-CoV-2 virus subtype (from hereinafter DNA vaccine); and
b. a second composition comprising an immunologically effective amount of a MVA-COVID19 or MVA-Δ-COVID19, preferably MVA-COVID19(S) or MVA-Δ-COVID19(S);
as a heterologous prime and boost generates a protective immune response against a SARS-CoV-2 immunogen. Such response is particularly strong when both compositions comprise at least a nucleic acid encoding the S antigenic protein of at least one SARS-CoV-2 virus subtype, preferably encoding for the S antigenic protein of SEQ ID NO 2.

It is noted that the recombinant MVA and/or DNA heterologous or homologous vaccines may be either monovalent, i.e., the nucleic acid comprised in the recombinant MVA and/or DNA vectors may comprise only one heterologous sequence encoding an antigenic determinant of SARS-CoV-2, or multivalent, i.e., the nucleic acid comprised in the recombinant MVA and/or DNA vectors may comprise at least two heterologous sequences encoding antigenic determinants of SARS-CoV-2, such as those selected from the list consisting of structural proteins S, E or M of SARS-CoV-2, preferably such antigenic proteins are those selected from any of SEQ ID NO 2, SEQ ID NO 4 and SEQ ID NO 6, respectively encoded by nucleic acid sequences SEQ ID NO 1, SEQ ID NO 3 and SEQ ID NO 5. Further, the nucleic acid comprised in the recombinant MVA/or DNA vectors may encode for structural proteins such as RBD (receptor binding domain) and/or N (nucleocapsid) of SARS-CoV-2 that can also be useful as heterologous sequences to work the present invention, preferably such antigenic proteins are those selected from any of SEQ ID NO 8 and SEQ ID NO 10, respectively encoded by nucleic acid sequences SEQ ID NO 7 and SEQ ID NO 9.

The invention thus provides, for the first time, effective vaccines or vaccine combinations for use in generating a protective immune response against infections by at least SARS-CoV-2 and vaccines or vaccine combinations which can be used for manufacturing of a vaccine against SARS-CoV-2.

Thus, a first aspect of the present invention provides a vaccine composition comprising
a. an immunologically effective amount of a modified vaccinia virus Ankara (MVA) vector comprising at least one nucleic acid encoding an antigenic protein, or an antigenic fragment thereof, of at least one SARS-CoV-2 virus subtype, optionally together with a pharmaceutically acceptable carrier, diluent and/or additive (from hereinafter such vaccine candidate shall be referred to as MVA-COVID19); and/or
b. an immunologically effective amount of the modified vaccinia virus Ankara (MVA) vector of a), wherein said MVA vector is further modified by deletion of the MVA immunomodulatory genes *C6L, K7R,* and *A46R,* optionally together with a pharmaceutically acceptable carrier, diluent and/or additive (from hereinafter such vaccine candidate shall be referred to as MVA-Δ-COVID19).

In a preferred embodiment, the nucleic acid encoding the antigenic protein is incorporated into a non-essential region of the genome of the MVA. In an embodiment of the present invention, the nucleic acid encoding the antigenic protein is incorporated into the MVA by substituting a non-essential region of the genome of the MVA. In a preferred embodiment of the present invention, the nucleic acid encoding the antigenic protein is incorporated into the MVA thymidine kinase (TK) locus or hemagglutinin (HA) locus. Integration of the nucleic acid encoding the antigenic protein is performed preferentially by homologous recombination of the flanking regions of a so-called transfer vector, which initially comprises the nucleic acid encoding the antigenic protein according to the invention prior to its integration into the parental MVA genome. In a preferred embodiment, the transfer vector is the pCyA vector. The pCyA vector has been previously described in WO 2014/162031 A1.

Upon homologous recombination by means of an infection with parental virus followed by a transfection with the plasmid transfer vector, selection and purification of recombinant viruses encoding the antigenic protein is performed. In an embodiment, the intermediate MVA recombinant viruses also expresses marker genes that facilitate the tracking and selection of the desired final recombinant viruses. For instance, the intermediate recombinant MVA viruses might express β- galactosidase gene, a fluorescent protein such as the green fluorescent protein (GFP), or an antibiotic resistance gene. Methods for generating recombinant MVAs by homologous recombination by means of intermediate MVAs encoding for selectable genes are described in WO 2014/162031 A1 and WO 2016/086980 A1.

On the other hand, early expression of antigens by VACV vectors is crucial for efficient antigen-specific CD8 T cell responses. The specific properties of the early elements poxviral promoters might thus be crucial for induction of an antigen-specific T cell response. Moreover, it is commonly known that higher amounts of antigen are beneficial for induction of stronger antigen-specific immune responses.

Therefore, in a further embodiment, the recombinant MVA vector comprises at least one nucleic acid encoding an antigenic protein, or an antigenic fragment thereof, of at least one SARS-CoV-2 virus subtype that is operably linked and under the control of a VACV-specific promoter, an orthopox virus-specific promoter, a poxvirus-specific promoter or a synthetic promoter. A number of promoters may be used for the present invention. For the expression of the nucleic acid encoding an antigenic protein according to the invention several promoters, such the 30K and 40K promoters (US 5,747,324, A Strong Synthetic Early-Late Promoter (Sutter, et al., Vaccine (1994), 12, 1032-1040)), the P7.5 promoter (Endo et al., J. Gen. Virol. (1991) 72, 699-703) and a promoter derived from the cow pox virus type A (inclusion ATI gene) (Lee et al., J. Gen. Virol. 1998), 79, 613) can be used.

In another preferred embodiment, the recombinant MVA vector comprises at least one nucleic acid encoding an antigenic protein, or an antigenic fragment thereof, of at least one SARS-CoV-2 virus subtype that is operably linked and under the control of a synthetic promoter. Synthetic promoters are DNA sequences that do not exist in nature and which are designed to regulate the activity of genes to which they are operably linked, controlling a gene's ability to produce its encoded protein. In a preferred embodiment, the synthetic promoter drives the expression of the antigenic protein early during infection. For instance, in an embodiment, the expression of the antigenic protein can be detected as early as 3-hour post-infection. In another embodiment, the synthetic promoter may also drive the expression of the antigenic protein late during infection. In a preferred embodiment, the expression of the antigenic protein is operably linked and under the control of a synthetic promoter that drives the expression of the antigenic protein both, early and late, during viral infection.

The synthetic promoter may be comprised by one or more elements driving early expression of said antigen. The synthetic promoter may be comprised by one or more elements driving late expression of said antigen. Different types of synthetic early/late promoters are further described in WO 2010/102822 A1. In another embodiment, the synthetic promoter is formed by both early and late elements, referred as synthetic early/late (sE/L) promoter (Chakrabarti, S.; Sisler, J.R.; Moss, B. Compact, synthetic, vaccinia virus early/late promoter for protein expression. Biotechniques 1997, 23, 1094-1097) or the LEO160 promoter (Di Pilato, M.; Sanchez-Sampedro, L.; Mejías-Pérez, E.; Sorzano, C.O.S.; Esteban, M. Modification of promoter spacer length in vaccinia virus as a strategy to control the antigen expression. J. Gen. Virol. 2015, 96, 2360-2371. Perez et al., 2019, Vaccines 2019, 7, 208). In another embodiment, the early and late elements are overlapping.

On the other hand, the nucleic acid encoding the antigenic protein inserted in MVA genome according to the invention can be selected from any group of nucleic acids encoding proteins from SARS-CoV-2 virus subtype. The nucleic acid encoding the antigenic protein according to the present invention may preferably be the protein S, M, E. In a preferred embodiment, the nucleic acid encoding an antigenic protein of at least one SARS-CoV-2 virus subtype encodes for the protein S.

In another preferred embodiment, the at least one nucleotide sequence encoding an antigenic protein of at least one SARS-CoV-2 virus subtype is preferably the structural protein S of SARS-CoV-2, and said nucleic acid encoding the structural protein S of SARS-CoV-2, if present, is inserted into the thymidine kinase (TK) locus of the MVA genome; and wherein the MVA vector regulates the expression of the nucleic acid encoding the antigenic protein and is operably linked to said nucleotide sequence.

In another preferred embodiment of the first aspect of the invention, the antigenic protein is selected from the any of the group of structural proteins S, E or M of SARS-CoV-2, or any combination thereof. It is noted that if at least one nucleotide sequence encodes for any of antigenic proteins E or M of SARS-CoV-2, such nucleic acids, if present, should be preferably inserted into the HA locus of the MVA genome; wherein the MVA vector regulates the expression of the nucleic acids encoding the antigenic proteins and should be operably linked to said nucleotide sequences.

In another preferred embodiment of the first aspect of the invention, the antigenic protein is the structural protein S of SARS-CoV-2 resulting in vaccine compositions MVA-COVID19(S) and/or MVA-Δ-COVID19(S), preferably the antigenic protein is the protein of SEQ ID NO 2, more preferably the antigenic protein of is the protein of SEQ ID NO 2 encoded by the nucleic acid consisting of SEQ ID NO 1. Still, more preferably, any of vaccine compositions MVA-COVID19(S) and/or MVA-Δ-COVID19(S) are further characterized by comprising at least one nucleotide sequence encoding for any of antigenic proteins E or M of SARS-CoV-2; wherein such nucleic acids, if present, should be preferably inserted into the HA locus of the MVA genome.

It is noted that the recombinant MVA viruses described herein are highly replication restricted and, thus, highly attenuated, and thus they are ideal candidates for the treatment of a wide range of mammals including humans and even immune-compromised humans. Hence, provided herein are pharmaceutical compositions and vaccines, as described above in the first aspect of the invention, suitable for inducing an immune response in a living animal body, including a human against the SARS-CoV-2 virus.

The vaccines of the first aspect preferably comprise any of the recombinant MVA viruses described herein formulated in solution in a concentration range of from 1 x 10e7 to 1 x 10e8 PFUs per dosis.

The vaccine compositions provided herein in the first aspect may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

For the preparation of vaccines, the recombinant MVA viruses provided herein can be converted into a physiologically acceptable form. This can be done based on experience in the preparation of poxvirus vaccines used for vaccination against smallpox as described by H. Stickl et al., Dtsch. med. Wschr. 99:2386-2392 (1974).

For example, purified viruses can be stored at -80°C with a titer of 5x10⁸ a PFU/ml formulated in about 10 mM Tris, 140 mM NaCl pH 7.4. For the preparation of vaccine shots, e.g., 10²-10⁸ or 10²-10⁹ particles of the virus can be lyophilized in 100 ml of phosphate-buffered saline (PBS) in the presence of 2% peptone and 1% human albumin in an ampoule, preferably a glass ampoule. Alternatively, the vaccine shots can be produced by stepwise freeze-drying of the virus in a formulation. This formulation can contain additional additives such as mannitol, dextran, sugar, glycine, lactose or polyvinylpyrrolidone or other aids such as antioxidants or inert gas, stabilizers or recombinant proteins (e.g., human serum albumin) suitable for in vivo administration. The glass ampoule is then sealed and can be stored between 45°C and room temperature for several months. However, as long as no need exists, the ampoule is stored preferably at temperatures below -20°C.

For vaccination or therapy, the lyophilisate can be dissolved in an aqueous solution, preferably physiological saline or Tris buffer, and administered either systemically or locally, i.e., parenteral, subcutaneous, intravenous, intramuscular, intranasal, or any other path of administration known to the skilled practitioner. The mode of administration, the dose and the number of administrations can be optimized by those skilled in the art in a known manner. However, most commonly a patient is vaccinated with a second shot about one month to six weeks after the first vaccination shot, although booster doses can also be administered years after the first dose.

In addition, for working the present invention any avirulent MVA vector suitable for human vaccination could be used. "Derivatives" or "variants" of MVA are also suitable for working the present invention. In preferred embodiments, the MVA vector is further modified by deletion of one or more vaccinia immunomodulatory genes; preferably one, two, or three of the genes C6L, K7R, and A46R. Most preferably, all three genes are deleted. Deletion of these genes has been shown to enhance the innate immune response triggered by the viral vector, and also adaptive antigen-specific immune responses.

In the context of the present invention, "Derivatives" or "variants" of MVA refer to viruses exhibiting essentially the same replication characteristics as MVA but exhibiting differences in one or more parts of their genomes. These "Derivatives" or "variants" of MVA must fail to reproductively replicate in vivo in humans and mice, even in severely immune suppressed mice. Tests and assays for the properties of MVA variants are described in WO 02/42480 (US 2003/0206926) and WO 03/048184 (US 2006/0159699).

The term "fails to reproductively replicate" refers to a virus that has a virus amplification ratio at 4 days after infection of less than 1 . Assays described in WO 02/42480 or in U.S. Patent No. 6,761,893 are applicable for the determination of the virus amplification ratio.

The amplification or replication of a virus is normally expressed as the ratio of virus produced from an infected cell (output) to the amount originally used to infect the cell in the first place (input) referred to as the "amplification ratio". An amplification ratio of "1" defines an amplification status where the amount of virus produced from the infected cells is the same as the amount initially used to infect the cells, meaning that the infected cells are permissive for virus infection and reproduction. In contrast, an amplification ratio of less than 1, i.e., a decrease in output compared to the input level, indicates a lack of reproductive replication and therefore attenuation of the virus.

The advantages of MVA-based vaccine include their safety profile as well as availability for large scale vaccine production. In a preferred embodiment, the recombinant MVA vector of any of the embodiments used for generating the recombinant virus is a MVA virus or a derivative having the capability of reproductive replication in vitro in chicken embryo fibroblasts (CEF) cells and in established chick cells DF-1, but no capability of reproductive replication in human cervix adenocarcinoma cell line HeLa. Also preferably, the recombinant MVA vector of any of the embodiments used for generating the recombinant virus is a MVA vector further modified by deletion of one or more vaccinia immunomodulatory genes; preferably one, two, or three of the genes C6L, K7R, and A46R. Most preferably, all three genes are deleted.

MVA vectors useful for the present invention can be prepared using methods known in the art, such as those described in WO 02/042480 and WO 02/24224, both of which are incorporated by reference herein.

In another aspect, a MVA viral strain suitable for generating the recombinant viruses of the present invention may be strain MVA-572, MVA-575 or any similarly attenuated MVA strain. Also suitable may be a mutant MVA, such as the deleted chorioallantoic vaccinia virus Ankara (dCVA). A dCVA comprises del I, del II, del III, del IV, del V, and del VI deletion sites of the MVA genome. The sites are particularly useful for the insertion of multiple heterologous sequences. The dCVA can reproductively replicate (with an amplification ratio of greater than 10) in a human cell line (such as human 293, 143B, and MRC-5 cell lines), which then enable the optimization by further mutation useful for a virus-based vaccination strategy (see WO 201 1/092029).

### Combination Vaccines Using Homologous/Heterologous Prime-Boost Regimens

The Combination Vaccines and methods described herein may be used as part of a homologous prime-boost regimen. In the homologous prime-boost, a first priming vaccination is given followed by one or more subsequent boosting vaccinations. The boosting vaccinations are configured to boost the immune response generated in the first vaccination by administration of the same recombinant MVA that was used in the first vaccination.

In one exemplary embodiment a homologous prime-boost regimen may be employed wherein a MVA viral vector as defined herein in the first aspect of the invention is administered in a first dosage. One or more subsequent administrations of a MVA viral vector as defined herein in the first aspect of the invention can be given to boost the immune response provided in the first administration. Preferably, the one or more antigenic determinants are the same or similar to those of the first administration.

The MVA recombinant viral vectors according to the present invention may also be used in prime-boost regimens in which one or more of the initial prime vaccinations are done with either the MVA or the DNA vector (see definition of DNA vaccine below) as defined herein and one or more subsequent boosting vaccinations are done with the MVA vector not used in the prime vaccination, e.g., if a MVA vector defined herein is given in a prime boost, then subsequent boosting vaccinations would be DNA vectors and vice versa.

Thus, a second aspect of the invention refers to a vaccine combination (from hereinafter referred to as homologous vaccine combination) comprising:
a. a first composition comprising an immunologically effective amount of a MVA-COVID19 or MVA-Δ-COVID19 vaccine composition, preferably a MVA-COVID19(S) or MVA-Δ-COVID19(S) vaccine composition; and
b. a second composition comprising an immunologically effective amount of a MVA-COVID19 or MVA-Δ-COVID19 vaccine composition, preferably a MVA-COVID19(S) or MVA-Δ-COVID19(S) composition;
wherein the first compositions is a priming composition and the second composition is a boosting composition, preferably wherein the boosting composition comprises two or more doses of the vector of the boosting composition.

Furthermore, an alternative second aspect of the invention refers to a vaccine combination (from hereinafter referred to as heterologous vaccine combination) comprising:
a. a first composition comprising an immunologically effective amount of a polynucleotide plasmid that comprises at least one nucleic acid encoding an antigenic protein of at least one SARS-CoV-2 virus subtype (from hereinafter DNA vaccine); preferably DNA-S and
b. a second composition comprising an immunologically effective amount of a MVA-COVID19 or MVA-Δ-COVID19, preferably MVA-COVID19(S) or MVA-Δ-COVID19(S);
wherein the first compositions is a priming composition and the second composition is a boosting composition or vice versa, and preferably wherein the boosting composition comprises two or more doses of the vector of the boosting composition.

It is noted that the recombinant heterologous or homologous vaccine combinations may be either monovalent, i.e., comprising only one heterologous sequence encoding an antigenic determinant of SARS-CoV-2, or multivalent, i.e., comprising at least two heterologous sequences encoding antigenic determinants of SARS-CoV-2, such as those selected from the list consisting of structural proteins S, E or M of SARS-CoV-2, preferably such antigenic proteins are those selected from any of SEQ ID NO 2, SEQ ID NO 4 and SEQ ID NO 6, respectively encoded by nucleic acid sequences SEQ ID NO 1, SEQ ID NO 3 and SEQ ID NO 5.

In one preferred embodiment of the second aspect of the invention, the antigenic protein of any of the homologous or heterologous vaccine combinations, is selected from any of the group of structural proteins S, E or M of SARS-CoV-2, or any combination thereof. It is noted that the at least one nucleotide sequence encoding an antigenic protein of at least one SARS-CoV-2 virus subtype is preferably the structural protein S of SARS-CoV-2, and said nucleic acid encoding the structural protein S of SARS-CoV-2, if present, should be preferably inserted into the thymidine kinase (TK) locus of the MVA genome. It is further noted that if at least one nucleotide sequence encodes for any of antigenic proteins E or M of SARS-CoV-2, such nucleic acids, if present, should be preferably inserted into the HA locus of the MVA genome.

As regards the heterologous vaccine compositions it is important to note that upon injection, preferably intramuscularly, of the DNA vaccine, cells, preferably muscle cells, may be efficiently transfected and produce a relatively large amount of antigen that may be secreted or otherwise released. Once the plasmid inserts itself into the transfected cell nucleus, it codes for the antigen of interest. Next, the transfected cell displays on its surface the foreign antigen in both histocompatibility complex (MHC) classes I and class II molecules. The antigen-presenting cell primed with the antigen travels to the lymph nodes and presents the antigen peptide and costimulatory molecules, initiating the immune response.

DNA-based vectors suitable for used as DNA vaccines, as disclosed herein for the heterologous regimes or heterologous vaccine combinations, are known in the art. For instance, Hobernik and Bros Int J Mol Sci. 2018 Nov 15;19(11):3605 summarize the knowledge on the course of action of DNA vaccines and the methods for DNA vaccine immunogenicity optimization. Lee et al. Acta Biomater. 2018 Oct 15;80:31-47 relates to engineering DNA vaccines against infectious diseases.

In one aspect of the present invention, a DNA-based vaccine, which comprises antigen-expressing plasmid DNA vectors, is provided. DNA vaccines may be introduced into animal tissues by multiple methods. For instance, naked DNA can be administrated intramuscularly. Other examples are DNA delivered by electroporation, by gene gun or using a liposoluble carrier. Methods of DNA-based vaccines administration are not limited and are known in the art.

The DNA-based vaccine vector encodes for one or more antigens against which the immune response is to be elicited. Further, the DNA vector can also encode other costimulatory molecules, such as interleukins, cytokines, etc.

If the DNA-based vaccine is to be administrated to a mammal, the promoter operably linked to the antigen of interest would need to be recognised by mammal cellular transcription machinery. Promoters for expressing proteins in mammalian cells are also known in the art. For instance, some viral promoters might be used, such as cytomegalovirus promoter or T7 phage promoter.

The DNA backbone to construct the DNA-based vaccine of the present invention is not limited. Any DNA, linear or plasmid, able to express the antigen in a mammalian cell may be used. Mammalian expression DNA vectors are widely known in the art.

In a preferred embodiment, the mammalian expression vector is a plasmid vector and produces high levels of the antigen. For instance, suitable mammalian expression plasmid vectors that can be used in the present inventions are, but not limited to, pCDNA, pCAGGS, etc. The mammalian expression vector can be grown in bacterial cells for high-yield production.

Methods for generating, producing and isolating plasmid DNA vectors are known in the art. In a preferred embodiment of the present invention, the DNA-based plasmid vaccine has been produced by endo-free means, for instance, by using EndoFree Plasmid Kits by Qiagen.

In an embodiment of the present invention, the mammalian expression plasmid vector is a pCDNA vector encoding an antigenic protein of at least one SARS-CoV-2 virus subtype. In another embodiment of the present invention, the mammalian expression plasmid vector is a pCDNA vector encoding at least one or a combination of the proteins S, M, E of SARS-CoV-2 virus subtype. In a more preferred embodiment, the plasmid vector encodes for the protein S of SARS-CoV-2 virus subtype. In a more preferred embodiment, the plasmid vector encoding an antigenic protein of at least one SARS-CoV-2 virus subtype has a SEQ ID 1 encoding for SEQ ID NO 2.

### Vaccines and Kits Comprising Recombinant MVA Viruses

Also provided herein are vaccines and kits comprising any one or more of the recombinant MVAs described herein. The kit can comprise one or multiple containers or vials of the recombinant MVA, together with instructions for the administration of the recombinant MVA to a subject at risk of SARS-CoV-2 infection. In certain embodiments, the subject is a human. In certain embodiments, the instructions indicate that the recombinant MVA is administered to the subject in a single dose, or in multiple (i.e., 2, 3, 4, etc.) doses. In certain embodiments, the instructions indicate that the recombinant MVA virus is administered in a first (priming) and second (boosting) administration to naive or non-naive subjects. Preferably, a kit comprises at least two vials for prime/boost immunization comprising the recombinant MVAs as described herein for a first inoculation ("priming inoculation") in a first vial/container and for an at least second and/or third and/or further inoculation ("boosting inoculation") in a second and/or further vial/container.

Thus, a third aspect of the invention refers to kits comprising one, preferably two, or more doses of any of the vaccine compositions as defined in the first aspect of the invention, or of any of the homologous or heterologous vaccine combinations of the second aspect of the invention. Preferably, the kit comprises an immunologically effective amount of a MVA-COVID19 or MVA-Δ-COVID19 vaccine composition, preferably MVA-COVID19(S) or MVA-Δ-COVID19(S), according to the first aspect of the invention in a first vial or container for a first administration (priming) and in a second vial or container for a second administration (boosting).

A preferred embodiment of the third aspect of the invention refers to kits comprising an immunologically effective amount of a polynucleotide plasmid that comprises at least one nucleic acid encoding an antigenic protein of at least one SARS-CoV-2 virus subtype, preferably the structural protein S of SARS-CoV-2, in a first vial or container for a first administration (priming), and an immunologically effective amount of a MVA-COVID19 or MVA-Δ-COVID19 vaccine composition, preferably MVA-COVID19(S) or MVA-Δ-COVID19(S), according to the first aspect of the invention in a second vial or container for a second administration (boosting).

Another preferred embodiment of the third aspect of the invention refers to kits comprising an immunologically effective amount of a a MVA-COVID19 or MVA-Δ-COVID19 vaccine composition, preferably MVA-COVID19(S) or MVA-Δ-COVID19(S), according to the first aspect of the invention, in a first vial or container for a first administration (priming), and an immunologically effective amount of a polynucleotide plasmid that comprises at least one nucleic acid encoding an antigenic protein of at least one SARS-CoV-2 virus subtype, preferably the structural protein S of SARS-CoV-2, in a second vial or container for a second administration (boosting).

In another preferred embodiment of the third aspect of the invention, any of the kits referred to herein, comprise a third, fourth or further vial or container compring any of the vaccine compositions indicated throughout the present invention for a third, fourth or further administration.

In a further preferred embodiment the vaccines, vaccine combinations and kits provided in any of the previous aspects are for use in generating a protective immune response against at least one SARS-CoV-2 subtype, wherein if the kits or vaccine combinations are used, the first composition is used for priming said immune response and the second composition is used for boosting said immune response or for use in generating a protective immune response against at least one SARS-CoV-2 subtype, wherein the second composition is used for priming said immune response and the first composition is used for boosting said immune response. In any of the vaccines, vaccine combinations and kits provided herein the boosting composition can comprise two or more doses of the vector of the boosting composition.

As discussed previously above, the present invention also relates to heterologous vaccination regimes using two different non-replicating viral vectors.

For heterologous vaccine programs, the present invention provides a combination vaccine and/or vaccination kit which comprises:
a. a first composition comprising an immunologically effective amount of a polynucleotide plasmid that comprises at least one nucleic acid encoding an antigenic protein of at least one SARS-CoV-2 virus subtype (from hereinafter DNA vaccine); preferably DNA-S and
b. a second composition comprising an immunologically effective amount of a MVA-COVID19 or MVA-Δ-COVID19, preferably MVA-COVID19(S) or MVA-Δ-COVID19(S);
wherein the first compositions is a priming composition and the second composition is a boosting composition or vice versa, and preferably wherein the boosting composition comprises two or more doses of the vector of the boosting composition.

The present invention also provides homologous vaccination regimes using two equal non-replicating viral vectors. For homologous vaccine programs, the present invention thus provides a combination vaccine and/or vaccination kit which comprises:
a. a first composition comprising an immunologically effective amount of a MVA-COVID19 or MVA-Δ-COVID19 vaccine composition, preferably a MVA-COVID19(S) or MVA-Δ-COVID19(S) vaccine composition; and
b. a second composition comprising an immunologically effective amount of a MVA-COVID19 or MVA-Δ-COVID19 vaccine composition, preferably a MVA-COVID19(S) or MVA-Δ-COVID19(S) composition;
wherein the first compositions is a priming composition and the second composition is a boosting composition, preferably wherein the boosting composition comprises two or more doses of the vector of the boosting composition.

In this embodiment, the combination vaccines and/or kit comprises at least two vials for prime/boost immunization comprising the recombinant MVAs/DNAs as described herein for a first inoculation ("priming inoculation") in a first vial/container and for an at least second and/or third and/or further inoculation ("boosting inoculation") in a second and/or further vial/container.

The combination vaccine and/or kit can comprise multiple containers or vials of the recombinant MVA/DNA, together with instructions for the administration of the recombinant MVA/DNA to a subject at risk of SARS-CoV-2 infection. In certain embodiments, the subject is a human. In certain embodiments, the instructions indicate that the recombinant MVA/DNA is administered to the subject in a single dose, or in multiple (i.e., 2, 3, 4, etc.) doses. In certain embodiments, the instructions indicate that the recombinant MVA/DNA virus is administered in a first (priming) and second (boosting) administration to naive or non-naive subjects.

The first and/or second composition or MVA and/or DNA of any combination vaccine, vaccination kit and/or any homologous or heterologous vaccine program of the invention can comprise any of the MVA and/or DNA vector described herein and any combination thereof.

### Methods and Uses of Recombinant MVA Viruses

Also provided herein are methods of use of any of the recombinant MVA-COVID19 or MVA-Δ-COVID19 vaccine compositions, preferably the MVA-COVID19(S) or MVA-Δ-COVID19(S) vaccine compositions, as described in the first aspect of the invention, for immunizing a subject animal or for affecting an immune response in a subject against SARS-CoV-2 infection. Also covered are uses of the said recombinant vaccine compositions described herein for the preparation of a medicament or pharmaceutical for the immunization of a subject animal, in particular for the preparation of a medicament or vaccine for treating and/or preventing a SARS-CoV-2-caused disease in a subject. Provided are also said recombinant MVAs according to any embodiment herein for use in priming or boosting an immune response against a SARS-CoV-2 infection, preferably wherein the recombinant MVA is administered once, twice, three times or four times.

Further covered herein are vaccine combinations of any of the embodiments for use as a medicament or vaccine for inducing an enhanced immune response against a SARS-CoV-2 infection wherein the combination is capable of producing the antigenic determinant, and/or SARS-CoV-2 -like particles in the subject to be treated, preferably, wherein the combination vaccine is producing the encoded antigenic determinant in the subject to be treated.

Accordingly, a fourth aspect of the present invention provides a method of generating an immunogenic and protective immune response against the SARS-CoV-2 virus in a subject in need thereof, preferably in a human subject, the method comprising administering to the subject the recombinant MVA-COVID19 or MVA-Δ-COVID19 vaccine composition, preferably the MVA-COVID19(S) or MVA-Δ-COVID19(S) vaccine composition, as described in the first aspect of the invention; or a kit or any of the vaccine combinations, including the homologous or heterologous vaccination regimes, as described in any of the second or third aspects of the invention.

As used herein, the term "immunogenic and protective immune response", "protective immunity" or "protective immune response" means that the vaccinated subject is able to control an infection with the pathogenic agent against which the vaccination was done. Usually, the subject having developed a "protective immune response" develops only mild to moderate clinical symptoms or no symptoms at all. Usually, a subject having a "protective immune response" or "protective immunity" against a certain agent will not die as a result of the infection with said agent. In certain embodiments, the subject animal is a mammal. The mammal may be an adult cow, a calf, in particular a juvenile calf, a rat, rabbit, pig, mouse, but preferably a human, and the method comprises administering a dose of any one or more of the recombinant MVAs or combinations thereof as described in the fourth aspect of the invention.

In certain embodiments of the fourth aspect of the invention, the subject is a human. In certain embodiments, the subject is an adult. In certain embodiments, the adult is immune-compromised. In certain embodiments, the adult is over the age of 10, 15, 20, 25, 30, 25, 40, 45, 50, 55, 60, 65, 70, 75, 80, or 85 years.

Any of the recombinant MVAs provided in the first aspect of the invention, or any of the kits or vaccine combinations, including the homologous or heterologous vaccination regimes, as described in any of the second or third aspects of the invention, may be preferably administered to the subject at a dose of 1x10^7 or 1x10^8 PFU/dose.

In certain embodiments, any of the recombinant MVAs provided in the first aspect of the invention, or any of the kits or vaccine combinations, including the homologous or heterologous vaccination regimes, as described in any of the second or third aspects of the invention, are administered to the subject at any of the doses provided herein prior to SARS-CoV-2 virus exposure as, e.g., 1 , 2, 3, or 4 weeks or 1, 2, 3, 4, 5, 6, 7, 8, 9, 10, 11, or 12 months before SARS-CoV-2 virus exposure. In certain embodiments, any of the said vaccine compositions or kits provided herein is administered to the subject at any of the doses provided herein after SARS-CoV-2 virus exposure as, e.g., 1, 2, 3, 4, 5, 6, 7, 8, 9. 10, 11, 12, 13, 14, 15, 16, 17, 18, 19, 20, 21, 22, 23, or 24 hours or 1, 2, 3, 4, 5, 6, or 7 days after SARS-CoV-2 exposure.

In certain embodiments, the recombinant MVAs provided in the first aspect of the invention, or any of the kits or vaccine combinations, including the homologous or heterologous vaccination regimes, as described in any of the second or third aspects of the invention, are administered to the subject in a single dose, or in multiple (i.e., 2, 3, 4, etc.) doses. In certain embodiments, the said kits or vaccine compositions provided herein are administered in a first (priming) and second (boosting) administration.

Boosting compositions are generally administered once or multiple times weeks or months after administration of the priming composition, for example, about 1 or 2 weeks or 3 weeks, or 4 weeks, or 6 weeks, or 8 weeks, or 16 weeks, or 20 weeks, or 24 weeks, or 28 weeks, or 32 weeks or one to two years. Preferably, the initial boosting inoculation is administered 1-12 weeks or 2-12 weeks after priming, more preferably 1, 2, 4 or 8 weeks after priming. In a preferred embodiment, the initial boosting inoculation is administered 4 or 8 weeks after priming. In additional preferred embodiments, the initial boosting is conducted at least 2 weeks or at least 4 weeks after priming. In still another preferred embodiment, the initial boosting is conducted 4-12 weeks or 4-8 weeks after priming.

The recombinant MVAs provided in the first aspect of the invention, or any of the kits or vaccine combinations, including the homologous or heterologous vaccination regimes, as described in any of the second or third aspects of the invention, can be administered systemically or locally. In certain embodiments, the said recombinant vaccine compositions are administered parenterally, subcutaneously, intravenously, intramuscularly, or intranasally, in particular subcutaneously or intramuscularly. Preferably, the recombinant MVAs are administered intranasally. In other embodiments, the said recombinant vaccine compositions are administered by any other path of administration known to the skilled practitioner. In a further preferred embodiment, the said recombinant vaccine composition is administered intramuscularly, preferably the recombinant vaccine composition is administered intramuscularly in a volume ranging between about 0.10 and 1.0 ml, preferably containing concentrations of e.g., about 10⁷ to 10^9 virus particles/ml. Preferably, the said recombinant vaccine composition is administered in a volume ranging between 0.25 and 1.0 ml. More preferably, the said recombinant vaccine composition is administered in a volume of about 0.5 ml.

Other embodiments of the invention will be apparent to those skilled in the art from consideration of the specification and practice of the invention disclosed herein. It is intended that the specification and examples be considered as exemplary only, with a true scope and spirit of the invention being indicated by the appended claims.

### Examples

### MATERIALS AND METHODS

### 1. Materials

### 1.1. Culture media

DF-1 and HeLa cells were grown in Dulbecco's modified Eagle's medium (DMEM) (Gibco) supplemented with non-essential amino acids (1X, Sigma-Aldrich) and L-glutamine (2 mM, Merck) and with 10 % fetal calf serum (FCS) (Gibco). Opti-MEM medium (Gibco) was used as a transient culture medium during plasmid transfection. DMEM was used as a medium for absorbing the virus and was removed after 1 hour, and DMEM-2 % FCS was used as a medium for incubating infections. The solid infection medium was used to select the recombinant virus plaques and consisted of a 1:1 ratio of DMEM 2X-4 % FCS and 1.9 % previously melted agarose (Conda). All the cells were kept in a humidified incubator at 37°C and 5 % CO₂.

### 1.2. Bacteria

The bacterial strains used for plasmid growth and transformations during cloning were chemically competent *Escherichia coli* DH5α cells (CNB) and electrocompetent *Escherichia coli* DH10B cells (New England Biolabs). The bacteria were cultured in Luria-Bertani (LB) medium supplemented with 1 % bacto-tryptone (BD Biosciences), 1 % NaCl (Sigma-Aldrich), and 0.5 % yeast extract (BD Biosciences) at pH 7 in the presence of ampicillin (100 µg/ml, Merck) or kanamycin (100 µg/ml, Merck) (Green MR, Sambrook J. 2012. Molecular Cloning: a laboratory manual, 4 ed. Cold Spring Harbor Laboratory Press, New York, USA)

### 1.3. Coronavirus SARS-CoV-2 S antigen (SEQ ID NO 2)

The antigen used to generate recombinant MVA viruses (and the mammalian expression vector DNA-S) is the coronavirus SARS-CoV-2 S gene (Wuhan seafood market pneumonia virus isolate Wuhan-Hu-1; the most contemporary isolate available at the time we initiated this work. GenBank accession number: MN908947.3). The nucleotide sequence of this protein was edited with codons optimized for humans by GeneArt (Thermo Fisher Scientific).

### 1.4. Virus

### 1.4.1. MVA-based viruses

The parental modified vaccinia virus Ankara (MVA) used for generating MVA-COVID-19(S) vaccine candidates was a wild-type MVA (MVA-WT) donated by Dr. G. Sutter of the Institute of Molecular Virology of Munich (Germany), or an MVA-GFP previously generated from MVA-WT, by inserting the gene encoding green fluorescent protein (GFP) in the thymidine kinase (TK) locus and by eliminating immunomodulatory genes *C6L*, *K7R*, and *A46R* of MVA. The GFP present in the MVA-GFP TK locus was then replaced by the coronavirus SARS-CoV-2 S gene to generate the different vaccine candidates MVA-COVID19(S) or MVA-Δ-COVlD19(S).

The S gene was inserted in the viral TK locus (gene *J2R*), and they are under the control of the synthetic early/late viral promoter (pE/L).

### 1.5. Plasmids

### 1.5.1. Plasmid transfer vector used to generate recombinant MVAs

A plasmid transfer vector pCyA was used for inserting the coronavirus SARS-CoV-2 S gene into the MVA genome. The cells were first infected with the parental MVA virus (MVA-WT or MVA-GFP) and then transfected with these plasmids. The recombinant MVA viruses were subsequently selected after several consecutive plaque purification steps. The process whereby a heterologous gene is inserted into the MVA genome is called homologous recombination, a type of genetic recombination in which nucleotides sequences are exchanged between two similar or identical DNA molecules. In this case, recombination occurs between the genetic sequences of the transfer plasmid and the viral genome. In order to insert the heterologous genes into the MVA genome, the transfer plasmids must contain the following elements:
- Flanking regions of the desired insertion area: These are sequences including the left (L) and right (R) regions of the MVA TK gene such that they allow recombination processes whereby the antigens of interest are inserted in the TK locus.
- Antigens of interest: These must be located between the L and R flanking regions of the TK gene, such that as a consequence of recombination, the antigens of interest are inserted in the viral genome. These antigens were inserted into the plasmid multiple cloning site (MCS) under the control of the VACV synthetic early/late (sE/L) promoter cloned into that same site.
- Selectable marker gene in cell culture: This is located in the area adjacent to the genes of the antigens of interest and within the flanking regions, such that it is inserted into the viral genome next to the genes of interest. Another area of recombination (L flanking region) necessary for the later removal of the marker gene by additional recombination processes is found to be repeated between the antigens of interest and the marker. Recombinant MVAs containing only the antigen of interest are therefore generated, which is necessary for use as vaccine candidates in humans. In this invention, the LacZ (β-galactosidase) marker gene was used to select in cell culture the intermediary recombinant viruses forming blue lysis plaques, after insertion in the TK locus. Furthermore, in the case of insertion of the antigens of interest in the TK locus, the genetic sequence of GFP will be removed, and loss of green fluorescence in recombinant virus plaques may therefore be used as an additional selection criterion.
- Selectable gene in bacterial culture: To clone and grow plasmids, the ampicillin resistance gene (β-Lactamase) is included in such plasmids in order to select the bacteria transformed with that gene.

The plasmid transfer vector used to introduce the coronavirus SARS-CoV-2 S protein gene into the MVA TK locus is briefly described below:
▪ **pCyA-S:** Plasmid transfer vector pCyA-S was used to generate MVA-COVID19(S) and MVA-Δ-COVID19(S) recombinant viruses and was developed by the CNB. It directs the insertion of the coronavirus SARS-CoV-2 S gene sequence, under the control of the VACV sE/L promoter, in the TK locus of the parental MVA-WT or MVA-Δ-GFP virus. We provided the pCyA vector to GeneArt (Thermo Fisher Scientific) for optimization of codons, synthesis of the S gene of SARS-CoV-2 and insertion into the plasmid vector. More specifically, the S gene was introduced between the VACV TK-L and TK-R flanking regions, under the control of the VACV sE/L promoter, in plasmid pCyA-20 MCS (Gómez et al., 2013, J Virol 87:7282-7300). Furthermore, this plasmid also contains the selectable marker genes for ampicillin and β-galactosidase (LacZ gene). Polymerase chain reaction (PCR) and sequencing techniques confirmed that plasmid pCyA-S (11,322 bp) had been generated correctly.

### 1.5.2. DNA plasmid expressing SARS-CoV-2 S protein

A DNA plasmid expressing SARS-CoV-2 S protein was generated by inserting human codon optimized SARS-CoV-2 S gene (see section 1.3) into mammalian expression vector pcDNA 3.1. The plasmid generated was termed DNA-S, and was used in in vivo animal studies. Plasmid DNA-S was generated by Dr. Jose Maria Casasnovas (CNB, Madrid).

### 1.6. Mice

Female mice of strain C57BL/6JOlaHsd (Envigo Laboratories) 6-8 weeks of age at the start of the experiment were used for the immunogenicity studies. These tests were approved by the Ethics Committee for Animal Testing (CEEA, *Comité Ético de Experimentación Animal*) of the CNB (Spanish National Center for Biotechnology), and by the Division of Animal Protection of the Comunidad de Madrid (PROEX 49/20), in accordance with national and international guidelines and Spanish Royal Decree (RD 53/2013), and were carried out in the animal facility of the National Center for Biotechnology (CNB; Madrid, Spain) in a pathogen-free area.

### 2. Reagents

### 2.1. Oligonucleotides

The following table (**Table 1**) shows the different oligonucleotides used for recombinant virus cloning, sequencing, and generation processes and those used in the PCR for detecting mycoplasma contamination.

**Table 1. Oligonucleotides. Targets of the corresponding restriction enzymes are shown in bold. F = Forward (sense), R = Reverse (antisense).**

| **Oligonucleotide** | **Sequence (5' → 3')** | **Template** |
|---|---|---|
| **Oligonucleotides used to check for mycoplasma contamination** | | |
| Myc A (F) | GGCGAATGGGTGAGTAACACG SEQ ID NO: 11 | Mycoplasma genome |
| Myc B (R) | CGGATAACGCTGCGACCTATG SEQ ID NO: 12 | Mycoplasma genome |

| **Oligonucleotides used to sequence the MVA genome and to characterize recombinant MVA viruses** | | |
|---|---|---|
| TK-L (F) | TGATTAGTTTGATGCGATTC SEQ ID NO: 13 | Left flank of TK |
| TK-R (R) | TGTCCTTGATACGGCAG SEQ ID NO: 14 | Right flank of TK |

| **Oligonucleotides used to sequence plasmid pCyA-S and to characterize recombinant MVA-COVID19(S) and MVA-Δ-COVID19(S) viruses** | | |
|---|---|---|
| SARS-CoV-2-S-F | AGCTCGAGGCCACCATGTTCGTGTTT CTGGTGCTGC SEQ ID NO: 15 | SARS-CoV-2 S gene |
| SARS-CoV-2-S-F1 | GGTTTCAGACACTGCTGGCCCTGCAC AGAAGC SEQ ID NO: 16 | SARS-CoV-2 S gene |
| SARS-CoV-2-S-F2 | GCAGTCCTACGGCTTTCAGCCCACAA ATGGCG SEQ ID NO: 17 | SARS-CoV-2 S gene |
| SARS-CoV-2-S-R2 | ATCCCTGTCAGGGCTCTATTCAGCTG GGTGCAG SEQ ID NO: 18 | SARS-CoV-2 S gene |
| SARS-CoV-2-S-R1 | TCTGGCCCAGCACACACTCAGACATC TTGGTGGCGG SEQ ID NO: 19 | SARS-CoV-2 S gene |
| SARS-CoV-2-S-R | AGGCGGCCGCTCAGGTGTAGTGCAG TTTCACGCCC SEQ ID NO: 20 | SARS-CoV-2 S gene |

### 2.2. Peptides and proteins

### 2.2.1. Peptides

Various coronavirus SARS-CoV-2 S peptides were used for the stimulation of splenocytes isolated from mice immunized with vaccine candidates:
▪ **SARS-CoV-2 S peptide pools:** These peptides are grouped into two mixtures, S1 (158 peptides) and S2 (157 peptides) (JPT). They are overlapping peptides of the coronavirus SARS-CoV-2 S-protein formed by 15 amino acids (15-mers), 11 of which are overlapping. Each peptide pool (S1 or S2) is at a concentration of 500 µg/ml.

### 2.2.2. Proteins

The coronavirus SARS-CoV-2 S and RBD proteins were used in enzyme-linked immunosorbent assays (ELISA) at a concentration of 2 µg/ml. Those proteins were provided by Dr. José María Casasnovas (CNB, Madrid)

### 2.3. Antibodies

The antibodies used in this invention are summarized in the following table (**Table 2**):

**Table 2. Primary and secondary antibodies. α = anti.**

| **Antibody** | **Characteristics** | **Origin** |
|---|---|---|
| **Primary antibodies** | | |
| **Rabbit α WR** | Anti-VACV Western Reserve (WR) strain protein polyclonal antibody | National Center for Biotechnology |
| **Rabbit α E3** | Anti-VACV 28-kDa protein (E3) polyclonal antibody | Donated by Dr. B. Jacobs and Dr. B. Moss |
| **Mouse α S** | SARS-CoV / SARS-CoV-2 (COVID-19) mouse spike monoclonal antibody [1A9] | GeneTex |
| **Rabbit α S** | SARS-CoV-2 (COVID-19) spike rabbit polyclonal antibody | GeneTex |

| **Secondary antibodies** | | |
|---|---|---|
| **Goat α mouse IgG-HRP** | Horseradish peroxidase (HRP)-conjugated anti-mouse IgG polyclonal antibody | Merck |
| **Goat α rabbit IgG-HRP** | HRP-conjugated anti-rabbit IgG polyclonal antibody | Merck |
| **Goat α mouse IgG-HRP** | HRP-conjugated anti-mouse total IgG polyclonal antibody | Southern Biotechnology |
| **Goat α mouse IgG1-HRP** | HRP-conjugated anti-mouse IgG1 subtype polyclonal antibody | Southern Biotechnology |
| **Goat α mouse IgG2c-HRP** | HRP-conjugated anti-mouse IgG2c subtype polyclonal antibody | Invitrogen |
| Goat **α** mouse **IgG3-HRP** | HRP-conjugated anti-mouse IgG3 subtype polyclonal antibody | Southern Biotechnology |

| **Conjugated antibodies for flow cytometry - CD4⁺ and CD4⁺ T cells** | | |
|---|---|---|
| **Hamster α mouse CD3e-PE-CF594** | Phycoerythrin-CF594-conjugated anti-CD3 monoclonal antibody, clone 145-2C11 | BD Biosciences |
| **Rat α mouse CD4-APC-Cy7** | Allophycocyanin-cyanin 7-conjugated anti-CD4 monoclonal antibody, clone GK1.5 | BD Biosciences |
| **Rat α mouse CD8a-V500** | V500-conjugated anti-CD8 monoclonal antibody, clone 53-6.7 | BD Biosciences |
| **Rat α mouse CD62L-Alexa700** | Alexa Fluor 700-conjugated anti-CD62L monoclonal antibody, clone MEL-14 | BD Biosciences |
| **Rat α mouse CD127-PerCP-Cy5.5** | PerCP-cyanin 5.5-conjugated anti-CD127 monoclonal antibody, clone A7R34 | BD Biosciences |
| **Rat α mouse IL-2-APC** | Allophycocyanin-conjugated anti-IL-2 monoclonal antibody, clone JES6-5H4 | BD Biosciences |
| **Rat α mouse IFN-γ-PECy7** | Phycoerythrin-cyanin 7-conjugated anti-IFN-γ monoclonal antibody, clone XMG1.2 | BD Biosciences |
| **Rat α mouse TNF-α-PE** | Phycoerythrin-conjugated anti-TNF-α monoclonal antibody, clone MP6-XT22 | eBioscience |
| **Rat α mouse CD107a-FITC** | Fluorescein isothiocyanate-conjugated anti-CD107 monoclonal antibody, clone 1D4B | BD Biosciences |
| **Rat α mouse CD16/CD32 (Fc block)** | Anti-CD16 and CD32 monoclonal antibody, clone 2.46 | Biosciences |

| **Conjugated antibodies for flow cytometry - Follicular helper T cells** | | |
|---|---|---|
| **Rat α mouse CD3-FITC** | Fluorescein isothiocyanate-conjugated anti-CD3 monoclonal antibody | BD Biosciences |
| **Rat α mouse CD4-Alexa700** | Alexa Fluor 700-conjugated anti-CD4 monoclonal antibody | BD Biosciences |
| **Rat α mouse CD44-PE-Cy5** | anti-CD44 monoclonal antibody phycoerythrin-cyanin 5-conjugated | BD Biosciences |
| **Rat α mouse CXCR5-PECF594** | Phycoerythrin-CF594-conjugated anti-CXCR5 monoclonal antibody | BD Biosciences |
| **Rat α mouse PD1(CD279)-APC-Efluor780** | Allophycocyanin-efluor780-conjugated anti-PD1 monoclonal antibody | BD Biosciences |
| **Rat α mouse IL-4-FITC** | Fluorescein isothiocyanate-conjugated anti-IL-4 monoclonal antibody | BD Biosciences |
| **Rat α mouse IL-21-APC** | Allophycocyanin-conjugated anti-IL-21 monoclonal antibody | BD Biosciences |
| **Rat α mouse CD154-PE** | Phycoerythrin-conjugated anti-CD154 monoclonal antibody | BD Biosciences |
| **Rat α mouse NKP46-APC** | Allophycocyanin-conjugated anti-NKP46 monoclonal antibody, clone 29A1.4 | BioLegend |
| **Rat α mouse CD3-APC-Cy7** | Allophycocyanin-cyanin 7-conjugated anti-CD3 monoclonal antibody, clone 17A2 | BioLegend |

### 3. Methodology

### 3.1. DNA manipulation techniques

The DNA manipulation techniques used were carried out according to the methodology described in the book *Molecular cloning: a laboratory manual* (Green MR, Sambrook J. 2012. Molecular Cloning: a laboratory manual, 4 ed. Cold Spring Harbor Laboratory Press, New York, USA).

### 3.1.1. DNA purification

The methodology used for DNA purification differed according to the source of the genetic material:
- Purification of plasmid DNA from bacterial cultures: To extract plasmid DNA from a bacterial culture, the bacteria were previously grown in LB and then DNA was then extracted by means of different commercial kits. The purification of plasmid DNA during the molecular cloning process was performed using the *Qiagen Plasmid Mini kit* (Qiagen), from 2 ml of positive bacterial clone cultures, following the manufacturer's instructions. Larger amount of purified plasmid DNA were obtained (from 200-ml bacterial cultures) for use in transfections and infections-transfections in cell cultures using the *Qiagen Plasmid Maxi kit* (Qiagen), following the manufacturer's protocol. The plasmid DNAs used for *in vivo* experiments were purified by means of the *Qiagen Plasmid endo-free Mega kit* (Qiagen) from 500-ml bacterial cultures, following the manufacturer's recommendations.
- Purification of DNA fragments: The purification of DNA from agarose gels or PCR reactions was carried out with the *Wizard^{®} Genomic DNA purification Kit* (Promega), following the manufacturer's recommendations. The purification of these DNA fragments was performed during the plasmid cloning processes and for sequencing regions of interest in plasmids and recombinant viruses to verify that they are generated correctly.
- Purification of genomic DNA of cells infected in culture: This technique was used to obtain the total DNA of cells infected with the recombinant viruses and to check for purity and that such viruses were generated correctly. For that purpose, the infected cells were collected when a cytopathic effect was observed and centrifuged at 3000 revolutions per minute (rpm) for 5 min at room temperature. The supernatant was removed, the precipitate was resuspended in 50 mM Tris-HCI pH 8, and proteinase K (200 µg/ml, Roche) in its corresponding buffer was added. After 1 hour of incubation at 55°C, RNAse A (40 µg/ml, PanReac AppliChem) was added, and it was incubated for 30 min at 37°C. Then saturated NaCl was added. It was mixed by inverting the tube and centrifuged at 13000 rpm for 10 min at room temperature. The supernatant was collected, mixed with isopropanol at a ratio of 1:0.7 to precipitate the DNA, and centrifuged at 13,000 rpm for 10 min at room temperature. The precipitate was washed with 75 % ethanol and centrifuged again at 13,000 rpm for 10 min at room temperature. Lastly the supernatant was carefully removed and the precipitated DNA was left to dry at room temperature, after which it was resuspended in sterile distilled water.

In all cases, DNA concentrations were measured in a NanoDrop^{®} ND-1000 full-spectrum spectrophotometer (Thermo Fisher Scientific). Isolated DNA was later used for different experiments, including PCR, sequencing, transfections, infections-transfections, and *in vivo* assays in mice.

### 3.1.2. Polymerase chain reaction (PCR)

The PCR technique was used to amplify DNA fragments for later cloning or to check for the gene insertion in transfer plasmids or in the recombinant MVA genome. The polymerase used was *Phusion*^{®} *High Fidelity* (New England Biolabs) with its corresponding buffers and reagents and following the manufacturer's recommendations. About 10-100 ng of template DNA together with 0.4 mM of the corresponding oligonucleotides, 1-2.5 units of *Phusion*^{®} *High Fidelity* polymerase with the corresponding buffer, and 0.2 mM of each of the four deoxynucleotide triphosphates (dNTPs) (Roche Diagnostics GmbH) were used for each reaction. The temperature used for annealing was selected according to the oligonucleotides used, and the extension time used depended on the length of the fragment to be amplified. The reactions were carried out in a 96-well VeritiTM thermocycler (Applied Biosystems).

### 3.1.3. DNA detection

To separate the different DNA fragments, the electrophoresis technique in 1 % agarose gel (Conda), with 1 % *SYBR* Safe (Invitrogen) as an intercalating agent was used. The DNA fragments were then viewed by means of the Gel Doc 2000 UV light transilluminator system (Bio-Rad) and QuantityOne software (Bio-Rad).

### 3.1.4. Gene cloning into plasmids transfer vectors

Coronavirus SARS-CoV-2 S gene was cloned into transfer plasmid pCyA to generate plasmid transfer vector pCyA-S by GeneArt (Thermo Fisher Scientific).

### 3.2. Protein manipulation techniques

### 3.2.1. Protein analysis by means of sodium dodecyl sulfate-polyacrylamide gel (SDS-PAGE) electrophoresis and membrane immunodetection (Western blot)

The protein samples of the cell extracts and supernatants were analyzed by means of one-dimensional sodium dodecyl sulfate-polyacrylamide gel (SDS-PAGE) electrophoresis according to standard protocols previously described (Green MR, Sambrook J. 2012. Molecular Cloning: a laboratory manual, 4 ed. Cold Spring Harbor Laboratory Press, New York, USA). In detail, the infected or transfected cell samples were collected, centrifuged at 2,000 rpm for 5 min at room temperature, and the precipitate was resuspended in Laemmli 1X-β-mercaptoethanol loading buffer. The cell supernatants were also centrifuged at 2,000 rpm for 5 min at 4°C to separate them from T cells that might be present. Then the proteins were precipitated from the supernatant with 10 % trichloroacetic acid (TCA) (Sigma-Aldrich), centrifuged at 13,000 rpm for 15 min at room temperature, and the precipitate was resuspended in Laemmli 1X-β-mercaptoethanol. After that, both the cell extract and supernatant samples were denatured at 95°C for 10 min and were loaded in 7 % polyacrylamide gels in the presence of SDS. The proteins were separated by means of one-dimensional electrophoresis at 100 V and at room temperature for about 90 min.

Once electrophoresis ended, the SDS-PAGE gels were transferred to nitrocellulose membranes (GE Healthcare), following the wet system protocol recommended by the manufacturer (Mini Trans-Blot^{®} Cell, Bio-Rad). The gel and previously hydrated nitrocellulose membrane were arranged between two Whattman-3MM^{®} filter papers (GE Healthcare), which were also hydrated. They were then mounted in the transfer system in the presence of the transfer buffer, and the transfer was performed at a current intensity of 100 V for 50 min. Once this ended, checking for the presence of proteins was performed by staining the membrane with the reversible Ponceau S stain (0.2 % Ponceau in 3 % TCA; Sigma-Aldrich).

The nitrocellulose membrane was then blocked with a 5 % skim milk powder solution prepared in PBS 1X-0.05 % Tween20 (Sigma-Aldrich) (PBS-T) at room temperature for 1 hour with gentle stirring. Next, the corresponding primary antibodies, prepared at the desired dilution in the blocking buffer, were added and incubated under stirring at 4°C overnight. After four washes with PBS-T, the membrane was incubated with the appropriate secondary antibody (suitably diluted in the same blocking buffer) for 1 hour at room temperature and was then washed again four times with PBS-T. The membranes were developed with the ECL^{®} luminol system (Amersham), exposing *Carestream Kodak BioMax XAR* autoradiography film (Kodak) or directly in the *ChemiDoc*^{™} *Imaging System* (Bio-Rad). The proteins bands were analyzed and quantified by means of *Image Lab software* (Bio-Rad).

### 3.3. Virus manipulation techniques

### 3.3.1. Viral infections

All the viruses were kept at -70°C and thawed at 37°C in a bath prior to use. Once thawed, they were stirred with a vortex and sonicated with a 3-cycle 10-second sonication and 10-second pause program (Misonix Incorporated S-3000 Sonicator, Cole-Parmer). The viruses were then added to the cell monolayer with the minimum volume of (serum-free) DMEM needed to cover the cell monolayer at the desired MOI. After 1 hour of adsorption at 37°C, the inoculum was removed and fresh DMEM-2 % FCS medium was added. The time the different infections were maintained varied with the objective of the infection, and the manner in which the cells were collected also varied.

### 3.3.2. Generation of recombinant MVA viruses

In this invention, 2 recombinant MVA viruses expressing coronavirus SARS-CoV-2 S-protein were generated in DF-1 cells according to the previously described standard infection/transfection protocol (Earl et al., 2001, Curr Protoc Mol Biol, 16:16 17), with certain modifications. To generate vaccine candidate MVA-COVID19(S), MVA-WT was used as the parental virus, and to generate vaccine candidate MVA-Δ-COVID19(S), MVA-Δ-GFP was used as a parental virus, as previously described (Garcia-Arriaza et al., 2014, J Virol 88:3527-3547).

Figure 11 shows a diagram summarizing the process followed for generating the recombinant MVA viruses. In detail, the DF-1 cell monolayer cultured in p60 dishes (Nunc) at 80-90 % confluence was infected at a MOI of 0.05 PFU/cell with the corresponding parental virus. In the meantime, a mixture of DNA-lipofectamine 2000 was prepared by mixing 10 µg of the corresponding transfer plasmid with the suitable volume of lipofectamine 2000 (Invitrogen) in Opti-MEM (Gibco) (1.5 µl lipofectamine 2000/µg of DNA) for 20 min at room temperature to form DNA-liposome complexes. After 1 hour of adsorption of the virus, the inoculum was removed, the cells were washed two times with Opti-MEM and incubated with 1 ml of the DNA-lipofectamine mixture for 4-6 hours at 37°C and 5 % CO₂. Next the transfection medium was removed, the cells were washed two times with Opti-MEM and incubated with DMEM-2 % FCS medium at 37°C and 5 % CO₂.

When a cytopathic effect was observed (usually after 3 days), the cells were collected, centrifuged at 1,500 rpm for 5 min at room temperature, and resuspended in 500 µl of serum-free DMEM. The cells were then frozen (at -70°C) and thawed (at 37°C) three times, stirring with a vortex between each freeze-thaw cycle to lyse them and release the virus into the supernatant, which was obtained after centrifuging at 3,000 rpm for 5 min at room temperature. The virus was then used to infect confluent monolayers of DF-1 cells grown in 6-well plates (M6) (Nunc) at different serial dilutions (from 10⁻¹ to 10⁻⁶) in serum-free DMEM. After 1 hour of infection, the inoculum was removed and replaced with 3 ml of solid infection medium consisting of a mixture of 1.9 % (previously melted) agarose (Conda) with DMEM 2X-4 % FCS medium at a 1:1 ratio. After 48 hours of infection, 1 ml of the 1.9 % agarose-DMEM 2X-4 % FCS 1:1 mixture and 1.2 mg/ml of X-Gal substrate (Sigma-Aldrich) or 0.8 mg/ml of the substrate X-Gluc (Sigma-Aldrich), whichever is appropriate, was added to view the lysis plaques produced by the virus incorporated in the insert of interest along with the LacZ gene or β-Gus, respectively.

After 24 hours, the blue plaques that were more isolated from other lysis plaques expressing GFP (green fluorescence), originating from the parental virus, were selected.

The selected lysis plaques were obtained with a 150-mm glass Pasteur pipette (Deltalab) and added to 500 µl of serum-free DMEM. After three freeze-thaw cycles and subsequent stirring with a vortex and sonication, these plaques were used as an inoculum to infect monolayers of confluent cells in 24-well plates (M24) (Nunc) as explained in section 3.3.1.

These infections were performed to re-grow the virus, and they were collected when a cytopathic effect was observed. These regrown plaques were next used to re-infect monolayers of confluent cells in M24 plates, and once a cytopathic effect was observed, these cells were collected and lysed for PCR and Western Blot analysis to check that the recombinant MVA virus was generated correctly. Oligonucleotides TK-L and TK-R were used in the PCR to amplify the antigens inserted in the TK locus (oligonucleotides described in **Table 1**). Anti-S-protein rabbit polyclonal or mouse monoclonal antibodies (described in **Table 2**) were used to analyze expression of the antigens of interest by Western blot.

Once the presence of the antigen and its correct expression by the different plaques were confirmed, one of them was selected and used as an inoculum to infect monolayers of confluent cells in M6 plates at dilutions from 10⁻¹ to 10⁻⁶. This purification process was carried out three times selecting blue plaques, and three more times selecting colorless plaques (neither blue nor GFP) (Figure 11). A purified recombinant virus containing the antigen of interest in the TK locus and without the LacZ marker, which was lost through homologous recombination, is therefore ultimately obtained.

Three types of viral stocks are distinguished in this invention. The initial viral stock (stock P1) consists of the generated recombinant virus originating from cell monolayers infected with the final plaque and collected after observing a cytopathic effect. Once having verified the expression of the antigens of interest by means of Western Blot and the purity of the recombinant virus by means of PCR and sequencing, it was used to generate the working viral stock (stock P2). For that purpose, 5 p150 plates (Nunc) containing confluent DF-1 cells were infected at an MOI of 0.05 PFU/cell and cells were collected when a cytopathic effect was observed. Stock P2 was used in all the experiments for characterizing the virus *in vitro.* Lastly, for the *in vivo* assays, the purified viral stock (stock P3) was generated from stock P2 (described in section 3.3.4.).

### 3.3.3. Characterization of the recombinant MVA viruses

The experiments performed to characterize the different recombinant MVA viruses include titration of the viruses, viral growth kinetics, stability analysis of the inserted antigens, and analysis of their apoptotic capacity. As mentioned above, stock P2 was used to perform these characterization experiments.

### • Viral titration

Titration of the MVA viruses was carried out according to the previously described standard protocols (Ramírez et al., 2000, J. Virol, 74:923-933). DF-1 cells seeded in M6 plates were infected in duplicate with serial dilutions of the virus in serum-free DMEM, as described in section 3.3.1. The medium was removed 30-40 hours post-infection and the cells were fixed for 3 min using a 1:1 mixture of methanol and acetone and the titer was determined by means of an immunostaining assay which allows detecting and counting lysis plaques formed by the virus. The monolayers were thereby incubated with a rabbit anti-VACV WR polyclonal primary antibody (diluted 1:1.000 in PBS-3 % FCS; CNB) for 1 hour at room temperature; it was then washed three times with PBS 1X and incubated for 1 hour at room temperature with the HRP-conjugated goat anti-rabbit secondary antibody (diluted 1:1.000 in PBS-3 % FCS; Sigma-Aldrich). After three additional washes with PBS 1X, the plates were developed using 1 mg/ml of 3,3'-diaminobenzidine tetrahydrochloride (DAB; Sigma-Aldrich), as the HRP substrate, with 30 % hydrogen peroxide (Sigma-Aldrich) and 3 % nickel sulfate (NiSO₄) (Sigma-Aldrich). After counting the lysis plaques, the titer was referenced as PFU/ml.

### • Replication kinetics

To evaluate the replication capacity of the different recombinant MVA viruses and analyze if the insertion of the S gene affects their replication capacity, monolayers of DF-1 cells were infected with each virus at an MOI of 0.01 PFU/cell, in duplicate. After 1 hour of adsorption, the inoculum was removed and was added to the DMEM-2 % FCS infection medium monolayer. The cells were collected at different times post-infection (0, 24, 48, and 72 hours), subjected to three freeze-thaw cycles (stirred with a vortex between each cycle), and kept at -70°C until titration. Lastly, the viruses were sonicated and titrated using the method described in the preceding section.

### • Genetic stability analysis

The genetic stability of vaccine candidates MVA-COVID19(S) and MVA-Δ-COVID19(S) was analyzed. Monolayers of DF-1 cells were infected with the different recombinant viruses at an MOI of 0.05 PFU/cell. The cells were collected 72 hours post-infection, frozen-thawed three times, and briefly sonicated. The cell extracts were then centrifuged at 1,500 rpm for 5 minutes, and the supernatant was used for a new round of infection at a low MOI. The same method was repeated nine times. Expression of the coronavirus SARS-CoV-2 S-protein was analyzed by means of Western blot (see section 3.2.3.) after infecting DF-1 cells with the viral stocks from each passage.

### 3.3.4. Virus purification

Purified virus stocks (stock P3) were used for the *in vivo* assays according to the method first described by Joklik (Joklik, 1962, Virology, 18:9-18) and modified by Esteban (Esteban, 1984, Virology, 133:220-227). In summary, 30 p150 plates containing confluent CEF cells were infected with stock P2 at an MOI of 0.01 PFU/cell. The cells were collected when a cytopathic effect was observed (usually after 3 days), centrifuged at 1,500 rpm for 5 min at 4°C, and the precipitate was washed with PBS and resuspended in 10 mM Tris-HCI buffer pH 9. The infected cells were then lysed by means of three sonication cycles, stirring with a vortex, and centrifugation to release the viral particles into the supernatant. Next, this supernatant was centrifuged for 1 hour at 20,000 rpm at 4°C in the SW28 rotor (Beckman) on a 36 % sucrose pad in 10 mM Tris-HCI pH 9. The obtained precipitate was resuspended in 10 mM Tris-HCI pH 9 and centrifuged again on another 36 % sucrose pad under the same conditions. Lastly, the precipitate obtained in this second centrifugation was resuspended in 10 mM Tris-HCI pH 9, aliquoted, and frozen at -70°C until use.

The purified viruses were tittered in duplicate and checked for the absence of mycoplasma contamination (through PCR with mycoplasma-specific oligonucleotides; see **Table 1**), bacteria (by means of growth in LB agar plates), and fungi (by means of growth in blood agar plates, Oxoid). Furthermore, checking for the correct expression of the antigens of stocks P3 was performed by means of PCR and Western blot before use in the *in vivo* assays.

### 3.4. In vivo immunological techniques

### 3.4.1. Immunization protocols

In assays for analyzing adaptive immune responses, two different immunization protocols were carried out. In all the assays, 6 female C57BL/6 mice per group were inoculated by intramuscular route. On one hand, a homologous prime-boost immunization was used, which consisted of a first inoculation with 1 x 10⁷ PFU of vaccine candidates MVA-COVID19(S) and MVA-Δ-COVID19(S), followed by a 1 x 10⁷ PFU booster dose of the same vaccine candidates MVA-COVID19(S) and MVA-Δ-COVID19(S) 2 weeks later. The mice were sacrificed 11 days after the booster dose. On the other hand, a heterologous prime-boost immunization regimen was used, which consisted of a first inoculation with 100 µg (50 µg/leg) of a plasmid vector expressing the coronavirus SARS-CoV-2 S-protein (DNA-S), followed by a 1 x 10⁷ PFU booster dose of vaccine candidates MVA-COVID19(S) and MVA-Δ-COVID19(S) 2 weeks later. The mice were sacrificed 11 days after the booster dose as well.

All the preparations of the inocula were performed in endotoxin-free PBS (Gibco). Intramuscular immunization was performed in the triceps of both hind legs in a total volume of 100 µl of PBS (50 µl/leg), using a BD Micro-Fine (30 G) x 8 mm insulin syringe (BD Biosciences). At the end of each immunization study the corresponding animals were sacrificed using CO₂, and blood samples were drawn and spleens removed, as appropriate in each experiment.

### 3.4.2. Analysis of the adaptive T cell-mediated immune response by means of ELISpot

The ELISpot assay was used to detect S antigen-specific IFNγ-secreting T cells (300). First, 96-well nitrocellulose-bottom plates (Millipore) were covered with 75 µl/well of a solution of the rat anti-mouse IFNγ monoclonal antibody (Pharmingen, San Diego, California) at a concentration of 6 µg/ml in PBS. After incubating overnight at room temperature, the wells were washed three times with RPMI medium and blocked with RPMI-10 % FCS for at least one hour at 37°C in 5 %CO₂ atmosphere. Moreover, the spleens of each group of immunized animals were collected in RPMI-10 % FCS medium and processed together to obtain the splenocytes as previously described (García-Arriaza et al., 2013, PLoS One, 8:e66894; Nájera et al., 2010, PLoS One, 5:e11406). In summary, the spleens were homogenized by means of mechanical disintegration through a 40-µm cell strainer (Falcon). The disintegrated cells were centrifuged for 5 min at 1,500 rpm at 4°C and washed with RPMI-10 % FCS. Next, the erythrocytes were lysed by adding 0.1 M NH₄Cl (2 ml/spleen) for 5 min in ice. After this time, the cells were washed three times with RPMI-10 % FCS to eliminate fat and resuspended in 12 ml of RPMI-10 % FCS, and the number of living splenocytes was counted by means of staining with trypan blue (4 % in water; Sigma-Aldrich).

To evaluate the specific response to the different antigens, 10⁶ splenocytes per condition were re-stimulated with 1 µg/ml of the S1 or S2 peptide pool. As a control of the response to the MVA vector, 10⁶ splenocytes were stimulated with 2.5 µg/ml of VACV E3 peptide. The plates were incubated with the peptides for 48 hours at 37°C in 5 % CO₂ atmosphere, washed five times with PBS-T, and incubated with 2 µg/ml of biotinylated rat anti-mouse IFNγ monoclonal antibody XMG1.2 (Pharmingen) diluted in PBS-T, for 2 hours at room temperature. The plates were then washed five times with PBS-T and a 1:800 dilution of HRP-conjugated streptavidin (0.5 mg/ml; Sigma-Aldrich) was added. After 1 hour at room temperature, it was washed three times with PBS-T and two times with PBS, and finally 1 µg/ml of the DAB substrate (Sigma-Aldrich), resuspended in 50 mM Tris-Cl pH 7.5 and 0.015 % H₂O₂, was added to develop the plates. The reaction was stopped by washing the plate with abundant water and once it was dry, the spots were counted using the *ELISpot Reader System -ELR02-* plate reader (AID Autoimmun Diagnostika GmbH) with the aid of *AID ELISpot reader system software* (Vitro).

### 3.4.3. Analysis of the adaptive cell-mediated immune response by means of flow cytometry

### 3.4.3.1. Analysis of the specific responses of CD4⁺ and CD8⁺ T cells

To study the response of SARS-CoV-2-specific T cells generated by the different immunization protocols (see section 3.4.1.), 6 mice per group were immunized and sacrificed 11 days after the last immunization. After sacrificing the animals, the spleens were removed from the immunized animals in RPMI-10 % FCS medium and processed as described in the preceding section. The splenocytes were then resuspended in RPMI-10 % FCS medium with 2 µl/ml of Monensin 1X (Thermo Fisher Scientific), 2 µl/ml of brefeldin A (BFA) (*Protein Transport Inhibitor BD GolgiPlug,* BD Biosciences), and a 1:300 dilution of anti-CD107a-FITC antibody. 4 x 10⁶ cells were added in M96 conical bottom wells and were incubated for 6 hours in the presence of 1 µg/ml of the S1 or S2 peptide pool, or with 5 µg/ml of VACV E3 peptide to determine the response to the MVA vector. As controls, the splenocytes were stimulated with 2 µl/ml of LAC (Leukocyte Activation Cocktail, BD Biosciences) and Monensin 1X. After 6 hours of stimulation, the splenocytes were washed with IB buffer and incubated with *Violet Dye* (0.5 µl/ml, Invitrogen) for 20 min in the dark at 4°C to evaluate cell viability. Next, two washes were performed with IB buffer and the splenocytes were incubated for 20 min in the dark at 4°C with 50 µl of the primary surface antibodies (described in the **Table 2**) diluted as indicated for each batch. The splenocytes were then washed two times and 100 µl of *Cytofix*/*Cytoperm* (BD Biosciences) was added per well for 20 min in the dark to 4°C for fixing and permeabilizing the cells, and they were then left in IB buffer overnight at 4°C in the dark. A wash was performed the next day with *Permwash* 1X (BD Biosciences) and 25 µl of *Fc Block* diluted 1:100 in *Permwash* 1X were added for 5 min at 4°C in the dark. Next, 25 µl of the antibodies needed for intracellular cytokine staining (ICS) were added to the 25 µl of *Fc Block,* at different dilutions in *Permwash* 1X according to the batch of each antibody (see **Table 2**), with incubation being performed for 20 min at 4°C in the dark. Lastly, two washes were performed with *Permwash* 1X and the cells resuspended in 200 µl of IB buffer to later be analyzed by means of flow cytometry in a Gallios cytometer (Beckman Coulter) and by means of the *FlowJo* program (*Tree Star,* Ashland, Oregon). The analysis strategy was performed by selecting the living cells expressing CD3 on their surface; then those expressing CD4, CD8, CD127, or CD62L; and finally those secreting the different cytokines (IFN-γ, TNF-α, and IL-2) or expressing degranulation marker CD107a.

### 3.4.3.2. Analysis of the specific responses of follicular helper T cells

To analyze the response of follicular helper T cells (Tfh) against SARS-CoV-2, spleens were extracted from sacrificed animals and processed as explained above. The splenocytes were then resuspended in RPMI-10% FCS medium with 1 µl/ml of BFA (BD Biosciences), 1 µl/ml of Monensin 1X (eBioscience), and a 1:100 dilution of the anti-CD154 (CD40L)-PE antibody. Next, 4 x 10⁶ splenocytes were stimulated with 5 µg/ml of S-protein and 1 µg/ml of S1 and S2 peptide pools for 6 hours at 37°C in a conical-bottom 96-well plate. The cells were then stained with Fixable Viability Stain (FVS) 520 (BD Biosciences) for 20 min at 4°C in the dark to analyze cell viability. The splenocytes were then washed two times with IB buffer and stained for 20 min at 4°C in the dark with 50 µl of the antibodies specific against the surface markers (described in **Table 2**), using the dilutions recommended by the manufacturer. Next, the splenocytes were again washed two times with IB buffer, fixed, and permeabilized with the *BD Cytofix*/*Cytoperm*^{™} *kit* (BD Biosciences) for 20 min at 4°C in the dark, and they were left in IB buffer overnight at 4°C in the dark. The cells were washed the next day with *Permwash* 1X (BD Biosciences) and the Fc receptors were blocked with 25 µl of *Fc Block* (diluted to 1:100 in *Permwash* 1X) for 5 min at 4°C in the dark. Next, 25 µl of the antibodies (diluted in *Permwash* 1X at the dilution indicated by the manufacturer) required for ICS (see **Table 2**) were added on 25 µl of *Fc Block,* incubating for 20 min at 4°C in the dark. Finally, the splenocytes were washed two times in *Permwash* 1X, resuspended in 200 µl of IB buffer, and passed through a Gallios flow cytometer (Beckman Coulter). The analysis was performed with the *FlowJo* software (*Tree Star,* Ashland, Oregon). The analysis strategy was performed by selecting living cells expressing CD3, CD4, and CD44 on their surface, followed by cells expressing CXCR5, PD1, and CD154 (CD40L), and finally cells secreting the different cytokines (IFN-γ, IL-4, and IL-21).

### 3.4.4. Analysis of the humoral immune response

The humoral immune response was determined by analyzing the levels of IgG antibodies in the sera of immunized mice and the capacity of these antibodies to neutralize the SARS-CoV-2 virus. To obtain the sera, blood was collected from the animals after sacrificing them by means of intracardiac puncture. Next, the blood samples were maintained at 37°C for 1 hour and kept at 4°C overnight. The tubes of blood were centrifuged the next day at 3600 rpm for 20 min at 4°C and the serum which was kept at -20°C until use was obtained. Serial dilutions of each serum sample (or group of sera) were then performed to measure the levels of coronavirus SARS-CoV-2 S-protein-specific and RBD-specific antibodies by means of ELISA, or to measure their SARS-CoV-2 neutralizing capacity by means of a luciferase assay. These techniques are explained in the following sections.

### 3.4.4.1. Enzyme-linked immunosorbent assay (ELISA)

ELISA assays were performed to analyze the levels of antibodies in the serum of immunized animals. NUNC MaxiSorp^{™} 96-well plates (Thermo Fisher Scientific) were coated with 2 µg/ml of the coronavirus SARS-CoV-2 S-protein and RBD in PBS and incubated at 4°C overnight. The plates were washed three times the next day with PBS-T and blocked for 2 hours at room temperature with 5% skim milk prepared in PBS-T. After this time elapsed, the plates were washed three times with PBS-T, 50 µl of the serial dilutions of the serum samples diluted in PBS with 1% skim milk and 0.01% Tween20 were added, and the plates were incubated for 1.5 hours at room temperature. Next, the plates were again washed three times with PBS-T and incubated with 50 µl of HRP-conjugated mouse anti-IgG, -IgG1, -IgG2c, or -IgG3 antibodies (diluted to 1:1000 in PBS with 1% skim milk and 0.01% Tween20) for 1 hour at room temperature. Finally, after another washing step, the plates were developed by adding 100 µl of TMB substrate (Sigma-Aldrich) and the reaction was stopped by adding 50 µl of 1 M H₂SO₄. Absorbance was read at 450 nm by means of an EZ Read 400 microplate reader (Biochrom).

### 3.4.4.2. Neutralization assay

The capacity of the sera obtained from mice immunized with vaccine candidates MVA-COVID19(S) and MVA-Δ-COVID19(S), to neutralize the SARS-CoV-2 S virus was determined using lentivirus-based pseudoparticles expressing the SARS-CoV-2 S-protein (generated by Dr. Pablo Gastaminza and Dr. Urtzi Garaigorta; CNB, Madrid). Vero E6 cells were seeded in 96-well plates at 15,000 cells/well. Twenty-four hours post-seeding, a mix of SARS-CoV-2 pseudoparticles and mice serum samples (at a final dilution 1:50) were preincubated for 1 hour at 37°C and then added to the cells in triplicates. The DMEM-2%FCS medium was replaced 24 h after infection. Cells were lysed 48 h later and luciferase assays were performed as described: cells were lysed with reporter lysis buffer (Promega, Madison, WI) and luciferase activity measured for 2.5 seconds in a luminometer (Orion II Microplate Luminometer, Berthold Detection Systems, Pforzheim, Germany).

### RESULTS

In this invention, new MVA viral vector-based vaccines have been designed and developed against the coronavirus SARS-CoV-2 causing the COVID-19 pandemic. Once generated, these recombinant MVA viruses have been characterized *in vitro* and their capacity to stimulate adaptive SARS-CoV-2-specific T cell-mediated immune responses and humoral immune responses *in vivo* in immunized mice has been analysed.

### 1. Design and generation and in vitro Characterization of vaccine candidates MVA-COVID19(S) and MVA-Δ-COVID19(S) expressing the coronavirus SARS-CoV-2 S-protein

To generate novel vaccines against SARS-CoV-2, the causative agent of COVID-19 pandemic, that could stimulate SARS-CoV-2-specifc T- and B-cell immune responses, we have developed two MVA-based vaccine candidates expressing the SARS-CoV-2 full-length S structural gene (Wuhan seafood market pneumonia virus isolate Wuhan-Hu-1, Genbank number: MN908947.3; the most contemporary isolate available at the time we initiated this work) [termed MVA-COVID19(S) and MVA-Δ-COVID19(S)] (see Materials and Methods). Vaccine candidate MVA-COVID19(S) was generated following the insertion of the complete sequence of the coronavirus SARS-CoV-2 S gene in the genome of the parental virus MVA-WT, whereas the other vaccine candidate, MVA-Δ-COVID19(S), was generated following the insertion of the complete sequence of the coronavirus S gene in the genome of an MVA, referred to as MVA-GFP, expressing the GFP protein and containing complete deletions in the vaccinia virus immunomodulatory genes *C6L*, *K7R,* and *A46R.* The expression of the SARS-CoV-2 gene is under the transcriptional control of a synthetic early/late (sE/L) promoter.

The authors of the present invention have previously described that an MVA viral vector lacking said immunomodulatory genes *C6L, K7R,* and *A46R* and expressing the structural genes of the Chikungunya virus is capable of providing full protection to mice and non-human primates after a Chikungunya virus challenge (García-Arriaza et al., 2014, J. Virol, 88:3527-3547; Roques et al., 2017, JCI Insight, 2(6):e83527). Additionally, Zika and Ebola vaccine candidates based on this MVA viral vector lacking said immunomodulatory genes are capable of providing protection to mice after a Zika virus challenge (Pérez et al., 2018, Scientific Reports 8:17385) or an Ebola virus challenge (Lázaro-Frías et al., 2018, J. Virol, 92:e00363-18). Furthermore, the elimination of genes *C6L* and *K7R* in the HIV/AIDS vaccine candidate, MVA-B, expressing Env, Gag, Pol, and Nef of HIV-1 subtype B, induced an increased in the expression of IFN-β and IFN type 1-inducible genes in cultured immune cells and promoted an increase in the HIV-1 specific immune response in immunized mice (García-Arriaza et al., 2013, PLoS One, 8(6):e66894).

Figure 1A shows a diagram of the generated vaccine candidates MVA-COVID19(S) and MVA-Δ-COVlD19(S). For the sake of simplicity, MVA-COVID19(S) will be referred to as MVA-S and MVA-Δ-COVID19(S) as MVA-Δ-S throughout the text and figures. The methodology used for generating these vaccine candidates has been described above in Materials and Methods. The correct insertion of the coronavirus SARS-CoV-2 S gene and the purity of the generated vaccine candidates MVA-COVID19(S) and MVA-Δ-COVID19(S) were confirmed by means of PCR and sequencing, after amplifying the DNA obtained from DF-1 cells that were not infected (mock) or infected with 5 PFU/cell of MVA-WT, MVA-GFP, MVA-COVID19(S), and MVA-Δ-COVlD19(S). Oligonucleotides (described in **Table 3** of Materials and Methods) which hybridize in the flanking regions of the MVA TK gene (*J2R* gene) were used in the PCR, and the obtained results confirmed the presence of the S gene in MVA-COVID19(S) and MVA-Δ-COVID19(S), without contamination with the parental MVA-WT or MVA-GFP, respectively (Figure 1B). The TK locus of parental viruses MVA-WT or MVA-GFP, was amplified as PCR control, with the TK gene and the GFP gene being amplified, respectively. The correct presence of the S gene inserted in the TK locus was also confirmed by means of DNA sequencing. To demonstrate that MVA-S and MVA-Δ-S constitutively express the SARS-CoV-2 S protein, we performed a Western blot analysis of cell extracts from MVA-infective permissive chicken DF-1 cells infected at a MOI of 5 PFU/cell for 4 and 24 h with MVA-S, MVA-Δ-S, or MVA-WT using specific antibodies that recognize the SARS-CoV-2 S protein. Furthermore, DF-1 infected cells were leave in the presence or absence of tunicamycin, an inhibitor of protein N-glycosylation, to analyze the glycosylation pattern of SARS-CoV-2 S protein expressed from MVA. The results proved that MVA-S and MVA-Δ-S vaccine candidates correctly expressed the SARS-CoV-2 S protein (of an expected molecular weight of about 180 kDa, corresponding to the full-length glycosylated S protein) at 4, and 24 h postinfection (hpi), being detected either using a mouse monoclonal antibody against SARS-CoV (against SARS-CoV S2 region) or a rabbit polyclonal antibody against SARS-CoV-2 (against SARS-CoV-2 S1 region) (Figure 1C). Furthermore, tunicamycin treatment inhibited SARS-CoV-2 S glycosylation and a single band of about 130 kDa, corresponding to the full-length unglycosylated S protein was detected (Figure 1C).

The secretion of the S-protein in the supernatant was then analysed at different times post-infection. Cell extracts, or supernatants were collected from DF-1 cells that were not infected or infected with vaccine candidates MVA-COVID19(S) and MVA-Δ-COVID19(S), and with parental viruses MVA-GFP and MVA-WT, at an MOI of 5 PFU/cell 4 and 24 hours post-infection. Next, the expression of the S-protein in cell extracts and the secretion of said protein in the extracellular medium was analysed by means of Western blot (Figure 1D). The results showed that the expression of the S-protein was detected 24 hours post-infection (Figure 1D) in the cell extracts originating from vaccine candidates MVA-COVID19(S) and MVA-Δ-COVlD19(S). However, the expression of the S-protein in the supernatants (Figure 1D) was not detected because the S-protein inserted in the MVA genome is a transmembrane protein.

In conclusion, vaccine candidates MVA-COVID19(S) and MVA-Δ-COVID19(S), efficiently express the coronavirus SARS-CoV-2 S-protein.

### 2. In vitro characterization of vaccine candidates MVA-COVID19(S) and MVA-Δ-COVID19(S)

### 2.1. Vaccine candidates MVA-COVID19(S) and MVA-Δ-COVID19(S) form oligomers

Next, we analyzed the ability of the SARS-CoV-2 S protein expressed from MVA-S and MVA-Δ-S recombinants to form oligomers. Thus, at 24 hpi, we collected cell extracts under reducing or nonreducing conditions (with or without β-mercaptoethanol, respectively) from DF-1 cells infected at a MOI of 5 PFU/cell with MVA-S, and MVA-Δ-S, and then we analyzed by Western blotting the expression of SARS-CoV-2 S protein, using either a rabbit monoclonal antibody against SARS-CoV-2 or a mouse monoclonal antibody against SARS-CoV (Figure 2A). Immunoblots showed that under nonreducing conditions, the SARS-CoV-2 S protein expressed from MVA-S and MVA-Δ-S recombinants existed mainly as high-molecular-mass oligomers; while under reducing conditions, the protein was present as ∼180-kDa monomers (Figure 2A).

### 2.2. Vaccine candidates MVA-COVID19(S) and MVA-Δ-COVID19(S) replicate in permissive cells

To determine if the expression of the coronavirus SARS-CoV-2 S-protein affects MVA replication in cell culture, DF-1 cells were infected with vaccine candidates MVA-COVID19(S) and MVA-Δ-COVID19(S), and parental viruses MVA-WT and MVA-GFP, and cell extracts were collected at different times post-infection (0, 24, 48, and 72 hours), and the amount of virus present was determined. The results showed that the viral growth kinetics of vaccine candidates MVA-COVID19(S) and MVA-Δ-COVID19(S), was similar (Figure 2B). Furthermore, it was also similar and even greater than the growth kinetics of parental viruses MVA-GFP and MVA-WT, which demonstrates that the expression of the S-protein does not negatively affect MVA vector replication in permissive conditions. Moreover, similarly to parental MVA-WT and MVA-GFP viruses and as expected, MVA-S and MVA-Δ-S does not replicate in human HeLa cells (data not shown).

### 2.3. Vaccine candidates MVA-COVID19(S) and MVA-Δ-COVID19(S) are stable in cell culture

To confirm that vaccine candidates MVA-COVID19(S) and MVA-Δ-COVID19(S) are stable and can be maintained in cultured cells without the loss of the S gene, DF-1 cells were infected with MVA-COVID19(S) and MVA-Δ-COVID19(S) with a low MOI for 9 consecutive serial passages, and the expression of the S-protein was determined by means of Western Blot. The results showed that MVA-COVID19(S) and MVA-Δ-COVID19(S) efficiently expressed the S-protein for all the passages, which demonstrates that vaccine candidates MVA-COVID19(S) and MVA-Δ-COVID19(S), are genetically very stable (Figure 2C).

### 3. Analysis of the SARS-CoV-2-specific adaptive immune response of vaccine candidates MVA-COVID19(S) and MVA-Δ-COVID19(S) in C57BL/6 mice after heterologous (DNA/MVA) and homologous (MVA/MVA) immunization protocols.

The immunogenicity of vaccine candidates MVA-COVID19(S) and MVA-Δ-COVID19(S) in C57BL/6 mice was then evaluated using heterologous (DNA/MVA) or homologous (MVA/MVA) prime-boost immunization regimens, and the coronavirus SARS-CoV-2 S-protein-specific adaptive cell-mediated immune responses and humoral immune responses induced by those vaccine candidates were analysed in detail.

Therefore, in the heterologous (DNA/MVA) prime-boost protocols 6 C57BL/6 mice per group were immunized with the plasmid pcDNA-S (DNA-S) followed by vaccine candidates MVA-COVID19(S) or MVA-Δ-COVID19(S), whereas in the homologous (MVA/MVA) prime-boost protocols 6 C57BL/6 mice per group were immunized with two doses of each vaccine candidate MVA-COVID19(S) or MVA-Δ-COVID19(S). The mice were sacrificed on day 11 after the boost for measuring adaptive SARS-CoV-2-specific cell-mediated immune responses and humoral immune responses (Figure 3) (see Materials and Methods).

### 3.1. Immunization of C57BL/6 mice with vaccine candidates MVA-COVID19(S) and MVA-Δ-COVID19(S) induces high levels of SARS-CoV-2 S-specific cells secreting IFN-γ

The coronavirus SARS-CoV-2 S-protein-specific adaptive cell-mediated immune response was first analysed by means of ELISpot (see Materials and Methods) 11 days after the boost in C57BL/6 mice (n = 6/group) immunized with the different immunization protocols. As a control group, the mice were immunized with two doses of MVA-WT Splenocytes originating from the spleens of each group were stimulated *ex vivo* with a panel of peptide pools covering the complete sequence of the coronavirus SARS-CoV-2 S-protein (S1 and S2 peptide pools). The samples were stimulated with RPMI as a negative control, and with a VACV E3 protein peptide as a positive control. After 48 hours of stimulation, the cells were labeled with antibodies to identify IFN-γ-secreting cells specific for these peptides (Figure 4).

The results showed that all the immunization groups including vaccine candidates MVA-COVID19(S) and MVA-Δ-COVID19(S), induced high levels of S1- and S2-specific IFN-γ-secreting cells, with the response being directed to a greater extent against S1 peptide pools and to a lesser extent against S2 peptide pools (Figure 4A). In particular, the DNA-S/MVA-Δ-S group induced the highest levels of S1- and S2-specific IFN-γ-secreting cells. Moreover, heterologous (DNA/MVA) immunizations induced better response than homologous (MVA/MVA) immunizations, and vaccine candidate MVA-Δ-S, increased the responses in comparison with MVA-S, in both heterologous immunizations and homologous immunizations (Figure 4A). The response against MVA vector (E3 peptide) showed that homologous (MVA/MVA) immunizations induced a better response than heterologous (DNA/MVA) immunizations, with the MVA-Δ-S/MVA-Δ-S group inducing the highest levels of IFN-γ-secreting cells (Figure 4B).

### 3.2. Immunization of C57BL/6 mice with vaccine candidates MVA-COVID19(S) and MVA-Δ-COVID19(S) induces a strong, broad, and polyfunctional SARS-CoV-2 S-specific adaptive CD4⁺ and CD8⁺ T cell-mediated immune response

Next, splenocytes originating from the spleens of each immunization group were stimulated *ex vivo* with a panel of peptide pools covering the complete sequence of the coronavirus SARS-CoV-2 S-protein (S1 and S2 peptide pools). The samples were stimulated with RPMI as a negative control. After 6 hours of stimulation, the cells were labelled with specific antibodies to identify (CD4⁺ and CD8⁺) T cell populations and respondent cells (expressing CD107a on the surface of the T cells as an indirect cytotoxicity marker and/or producing cytokines IFN-γ, TNF-α, and IL-2) by ICS.

The results showed that all the immunization regimens (DNA/MVA or MVA/MVA) are capable of inducing coronavirus SARS-CoV-2 S-protein (S1 + S2)-specific adaptive CD4⁺ T cell-mediated (Figure 5A, left) and CD8⁺ T cell-mediated (Figure 5A, right) immune responses, with the levels of specific CD8⁺ T cells being greater than the levels of CD4⁺ T cells in all the groups. Moreover, heterologous (DNA/MVA) immunizations induced better CD4⁺ T cell-mediated response and CD8⁺ T cell-mediated response than homologous (MVA/MVA) immunizations (Figure 5A).

Like between groups MVA-S/MVA-S and MVA-Δ-S/MVA-Δ-S, the levels of S-protein-specific CD4⁺ T cells induced by immunization groups DNA-S/MVA-S and DNA-S/MVA-Δ-S are similar (Figure 5A). However, the levels of S-protein-specific CD8⁺ T cells induced by immunization groups including MVA-Δ-S in the boost are greater than groups including MVA-S in the boost (Figure 5A).

The detailed analysis of the response showed that, in all the immunization groups, the coronavirus SARS-CoV-2 S-protein-specific CD4⁺ T cells and CD8⁺ T cells are directed to a greater extent against S1 peptide pools and to a lesser extent against S2 peptide pools (Figure 5B), maintaining the aforementioned differences between groups.

These aforementioned differences can also be observed when analyzing the percentages of S-protein (S1+S2)-specific CD4⁺ T cells (Figure 5C, left) or CD8⁺ T cells (Figure 5C, right) secreting each of the analyzed cytokines (IFN-y, TNF-α, or IL-2) or expressing the cytotoxicity marker, CD107a. It must be pointed out that specific CD8⁺ T cell responses are characterized by a low IL-2 secretion.

Finally, the polyfunctionality of the CD4⁺ and CD8⁺ T cell-mediated immune response generated by the different immunization groups was analyzed by measuring the simultaneous production pattern of cytokines (IFN-y, TNF-α, and/or IL-2) plus the cytotoxic potential thereof (CD107a as a degranulation marker). The coronavirus SARS-CoV-2 S-protein (S1+S2)-specific cell-mediated responses generated by the different immunization groups were characterized by a high polyfunctionality (Figure 6). The CD4⁺ T cell-mediated response was mainly characterized by cells which are capable of carrying out all 4 functions (secreting all 3 cytokines and expressing the marker, CD107a), followed by cells capable of carrying out 3 functions (IFN-y, IL-2, and TNF-a), and cells capable of carrying out 2 functions (IFN-γ and TNF-α) (Figure 6A). On the other hand, the CD8⁺ T cell-mediated response was mainly characterized by cells which are capable of carrying out 3 functions (CD107a, IFN-γ and TNF-a), followed by cells capable of carrying out 4 functions (CD107a, IFN-γ, IL-2, and TNF-α) (Figure 6B).

In conclusion, vaccine candidates MVA-COVID19(S) or MVA-Δ-COVID19(S), are capable of inducing broad and polyfunctional coronavirus SARS-CoV-2 S-protein-specific adaptive CD4⁺ and CD8⁺ T cell-mediated immune responses of great magnitude.

### 3.3. Immunization of C57BL/6 mice with vaccine candidates MVA-COVID19(S) and MVA-Δ-COVID19(S) induces adaptive SARS-CoV-2 S-specific CD4⁺ and CD8⁺ T cell-mediated immune response with effector memory phenotype

Next, we determined the phenotype of the adaptive SARS-CoV-2-specific CD4+ and CD8+ T cells by measuring the expression of CD127 and CD62L surface markers, which defined different memory subpopulations: T central memory (TCM, CD127+/CD62L+), T effector memory (TEM, CD127+/CD62L⁻), and T effector (TE, CD127⁻/CD62L⁻) cells, and determined the sums of the individual responses expressing CD107a, IFN-γ, TNF-α, and/or IL-2 obtained for the S1, and S2 peptide pools (Figure 7). The results showed that in all vaccinated groups, adaptive SARS-CoV-2-specific CD4+ and CD8+ T cells were mainly of the TEM phenotype (Figure 7), maintaining the magnitude differences between groups previously described (see Figures 5 and 6).

### 3.4. Immunization of C57BL/6 mice with vaccine candidates MVA-COVID19(S) and MVA-Δ-COVID19(S) induces adaptive SARS-CoV-2 S-specific CD4⁺ T follicular helper (Tfh) cell-mediated immune response

Next, the proportion of coronavirus SARS-CoV-2 S-specific CD4⁺ T follicular helper (Tfh) cells was analyzed by means of ICS 11 days after the boost in C57BL/6 mice immunized with a heterologous (DNA/MVA) or homologous (MVA/MVA) prime-boost protocol, as described above.

Splenocytes isolated from the spleen were stimulated *ex vivo* with a combination of the coronavirus SARS-CoV-2 S-protein, together with the S1 and S2 peptide pools covering the complete sequence of said protein. The samples were stimulated with RPMI as a negative control. After 6 hours of stimulation, the cells were labelled with specific antibodies to identify the population of S-specific Tfh cells (CD4⁺, CXCR5⁺, and PD1⁺) expressing the marker CD40L (marker related to T cell effector functions) and/or producing the cytokines IFN-γ and/or IL-21.

The results showed that all vaccinated groups induced SARS-CoV-2-specific CD4⁺ Tfh cells expressing CD40L and/or secreting IFN-γ and/or IL-21 (Figure 8A). Heterologous DNA/MVA immunizations induced higher magnitude of SARS-CoV-2-specific CD4⁺ Tfh cells than homologous MVA/MVA immunizations (Figure 8A), with DNA-S/MVA-S triggering higher responses than DNA-S/ MVA-Δ-S and MVA-Δ-S/MVA-Δ-S higher responses than MVA-S/MVA-S (Figure 8A).

The pattern of SARS-CoV-2-specific CD4⁺ Tfh cells showed that in all vaccinated groups most of the CD4⁺ Tfh secreted IFN-γ and IL-21, followed by the presence of CD40L (Figure 8B).

Finally, the polyfunctionality of the Tfh cell-mediated immune response generated by the different immunization groups was analyzed by measuring the simultaneous production pattern of cytokines (IFN-γ and/or IL-21) plus the marker, CD40L. The coronavirus SARS-CoV-2 S-protein-specific Tfh cell-mediated responses generated by the different immunization groups were characterized by a high polyfunctionality (Figure 9B). The Tfh cell-mediated response was mainly characterized by cells which are capable of carrying out all 3 functions (secreting the 2 cytokines and expressing the marker, CD40L), followed by cells capable of carrying out 2 functions (IFN-y, IL-21) (Figure 9B). In conclusion, vaccine candidates MVA-COVID19(S) and MVA-Δ-COVID19(S) induce Tfh cell-mediated responses.

### 3.5. Immunization of C57BL/6 mice with vaccine candidates MVA-COVID19(S) and MVA-Δ-COVID19(S) induces high levels of binding IgG antibodies against SARS-CoV-2 S and RBD proteins

Next, the total levels of coronavirus SARS-CoV-2 S-protein-specific and S-protein receptor binding domain (RBD)-specific IgG antibodies and the levels of subclasses thereof, i.e., IgG1, IgG2c, and IgG3, present in the sera of C57BL/6 mice immunized with the different immunization protocols, were analyzed by means of ELISA 11 days after the second immunization (boost). The animals immunized with two doses of MVA-WT were used as a control group.

The results showed that all the immunization groups induced high levels of coronavirus SARS-CoV-2 S-protein-specific IgG antibodies (Figure 9A, left), as well as high levels of RBD-specific IgG antibodies (Figure 9A, right). In general, homologous (MVA/MVA) immunizations induced slightly higher levels of S-protein-specific and RBD-specific IgG antibodies than heterologous (DNA/MVA) immunizations (Figure 9A). The presence of antibodies against the RBD region suggest that those antibodies could be neutralizing antibodies, as this domain is the main region recognized by neutralizing antibodies.

Regarding the IgG subclasses of antibodies, all vaccinated groups elicited higher levels of IgG2c antibodies than IgG1 and IgG3 antibodies against SARS-CoV-2 S protein (Figure 9B, left), and RBD protein (Figure 9B, right); again with homologous MVA/MVA prime/boost immunizations triggering slightly higher levels than heterologous DNA/MVA immunizations regimens, except in the analysis of IgG3 antibodies against SARS-CoV-2 RBD protein where heterologous DNA/MVA immunizations regimens elicited slightly higher levels than homologous MVA/MVA prime/boost immunizations. Moreover, the determination of the IgG2c/IgG1 ratio showed that in all vaccinated groups it was higher than 1, indicating a Th1 response.

In conclusion, vaccine candidates MVA-COVID19(S) and MVA-Δ-COVID19(S) induced high levels of total IgG, IgG1, IgG2c, and IgG3 antibodies against SARS-CoV-2 S- and RBD proteins, with a Th1 response.

### 3.6. Immunization of C57BL/6 mice with vaccine candidates MVA-COVID19(S) and MVA-Δ-COVID19(S) induces high levels of SARS-CoV-2-specific neutralizing antibodies

Next, the capacity of the different immunization groups with vaccine candidates MVA-COVID19(S) and MVA-Δ-COVID19(S), to induce coronavirus SARS-CoV-2-specific neutralizing antibodies in individual sera originating from C57BL/6 mice immunized with the different immunization groups was analyzed as described previously. Sera originating from mice immunized with MVA-WT/MVA-WT were used as a negative control of the experiment.

The results showed that individual serum samples obtained from all mice vaccinated with MVA-S and MVA-Δ-S vaccine candidates neutralized SARS-CoV-2 pseudoparticles, significantly reducing the levels of luciferase units, compared to serum from the control group MVA-WT/MVA-WT, where no neutralization was observed (Figure 10). In detail, heterologous DNA/MVA prime/boost immunization protocols DNA-S/MVA-S and DNA-S/MVA-Δ-S triggered a positive neutralization, although being variable and of low levels. On the other hand, homologous MVA/MVA prime/boost immunization protocols MVA-S/MVA-S and MVA-Δ-S/MVA-Δ-S elicited a high neutralization profile with a reduction in the luciferase units of at least 3 logs, compared to the control group MVA-WT/MVA-WT (Figure 10).

In summary, vaccine candidates MVA-COVID19(S) and MVA-Δ-COVID19(S) induce coronavirus SARS-CoV-2-specific neutralizing antibodies.

### SEQUENCE LISTING

### SARS-CoV-2 S nucleotide sequence inserted in MVA-COVID19(S) and MVA-Δ-COVID19(S)

The SARS-CoV-2 S sequence inserted in vaccine candidates MVA-COVID19(S) and MVA-Δ-COVID19(S) described in the present examples was synthetized by GeneArt and was human codon optimized. The sequence derived from Wuhan seafood market pneumonia virus isolate Wuhan-Hu-1, GenBank: MN908947.3, https://www.ncbi.nlm.nih.gov/nuccore/MN908947
- The S nucleotide sequence reads as follows (SEQ ID NO 1): 
ATGTTCGTGTTTCTGGTGCTGCTGCCTCTGGTGTCCAGCCAGTGTGTGAACCTGAC
CACCAGAACACAGCTGCCTCCAGCCTACACCAACAGCTTTACCAGAGGCGTGTAC
TACCCCGACAAGGTGTTCAGATCCAGCGTGCTGCACTCTACCCAGGACCTGTTCC
TGCCTTTCTTCAGCAACGTGACCTGGTTCCACGCCATCCACGTGTCCGGCACCAAT
GGCACCAAGAGATTCGACAACCCCGTGCTGCCCTTCAACGACGGGGTGTACTTTG
CCAGCACCGAGAAGTCCAACATCATCAGAGGCTGGATCTTCGGCACCACACTGGA
CAGCAAGACCCAGAGCCTGCTGATCGTGAACAACGCCACCAACGTGGTCATCAAA
GTGTGCGAGTTCCAGTTCTGCAACGACCCCTTCCTGGGCGTCTACTACCACAAGA
ACAACAAGAGCTGGATGGAAAGCGAGTTCCGGGTGTACAGCAGCGCCAACAACTG
CACCTTCGAGTACGTGTCCCAGCCTTTCCTGATGGACCTGGAAGGCAAGCAGGGC
AACTTCAAGAACCTGCGCGAGTTCGTGTTCAAGAACATCGACGGCTACTTCAAGAT
CTACAGCAAGCACACCCCTATCAACCTCGTGCGGGATCTGCCTCAGGGCTTCTCT
GCTCTGGAACCCCTGGTGGATCTGCCCATCGGCATCAACATCACCCGGTTTCAGA
CACTGCTGGCCCTGCACAGAAGCTACCTGACACCTGGCGATAGCAGCAGCGGAT
GGACAGCTGGTGCCGCCGCTTACTATGTGGGCTACCTGCAGCCTAGAACCTTCCT
GCTGAAGTACAACGAGAACGGCACCATCACCGACGCCGTGGATTGTGCTCTGGAT
CCTCTGAGCGAGACAAAGTGCACCCTGAAGTCCTTCACCGTGGAAAAGGGCATCT
ACCAGACCAGCAACTTCCGGGTGCAGCCCACCGAATCCATCGTGCGGTTCCCCAA
TATCACCAATCTGTGCCCCTTCGGCGAGGTGTTCAATGCCACCAGATTCGCCTCTG
TGTACGCCTGGAACCGGAAGCGGATCAGCAATTGCGTGGCCGACTACTCCGTGCT
GTACAACTCCGCCAGCTTCAGCACCTTCAAGTGCTACGGCGTGTCCCCTACCAAG
CTGAACGACCTGTGCTTCACAAACGTGTACGCCGACAGCTTCGTGATCCGGGGAG
ATGAAGTGCGGCAGATTGCCCCTGGACAGACAGGCAAGATCGCCGACTACAACTA
 
CAAGCTGCCCGACGACTTCACCGGCTGTGTGATTGCCTGGAACAGCAACAACCTG
GACTCCAAAGTCGGCGGCAACTACAATTACCTGTACCGGCTGTTCCGGAAGTCCA
ATCTGAAGCCCTTCGAGCGGGACATCTCCACCGAGATCTATCAGGCCGGCAGCAC
CCCTTGTAACGGCGTGGAAGGCTTCAACTGCTACTTCCCACTGCAGTCCTACGGC
TTTCAGCCCACAAATGGCGTGGGCTATCAGCCCTACAGAGTGGTGGTGCTGAGCT
TCGAACTGCTGCATGCCCCTGCCACAGTGTGCGGCCCTAAGAAAGCACCAATCT
CGTGAAGAACAAATGCGTGAACTTCAACTTCAACGGCCTGACCGGCACCGGCGTG
CTGACAGAGAGCAACAAGAAGTTCCTGCCATTCCAGCAGTTTGGCCGGGATATCG
CCGATACCACAGACGCCGTTAGAGATCCCCAGACACTGGAAATCCTGGACATCAC
CCCTTGCAGCTTCGGCGGAGTGTCTGTGATCACCCCTGGCACCAACACCAGCAAT
CAGGTGGCAGTGCTGTACCAGGACGTGAACTGTACCGAAGTGCCCGTGGCCATTC
ACGCCGATCAGCTGACACCTACATGGCGGGTGTACTCCACCGGCAGCAATGTGTT
TCAGACCAGAGCCGGCTGTCTGATCGGAGCCGAGCACGTGAACAATAGCTACGAG
TGCGACATCCCCATCGGCGCTGGCATCTGTGCCAGCTACCAGACACAGACAAACA
GCCCCAGACGGGCCAGATCTGTGGCCAGCCAGAGCATCATTGCCTACACAATGTC
TCTGGGCGCCGAGAACAGCGTGGCCTACTCCAACAACTCTATCGCTATCCCCACC
AACTTCACCATCAGCGTGACCACAGAGATCCTGCCTGTGTCCATGACCAAGACCA
GCGTGGACTGCACCATGTACATCTGCGGCGATTCCACCGAGTGCTCCAACCTGCT
GCTGCAGTACGGCAGCTTCTGCACCCAGCTGAATAGAGCCCTGACAGGGATCGCC
GTGGAACAGGACAAGAACACCCAAGAGGTGTTCGCCCAAGTGAAGCAGATCTACA
AGACCCCTCCTATCAAGGACTTCGGCGGCTTCAATTTCAGCCAGATTCTGCCCGAT
CCTAGCAAGCCCAGCAAGCGGAGCTTCATCGAGGACCTGCTGTTCAACAAAGTGA
CACTGGCCGACGCCGGCTTCATCAAGCAGTATGGCGATTGTCTGGGCGACATTGC
CGCCAGGGATCTGATTTGCGCCCAGAAGTTTAACGGACTGACAGTGCTGCCTCCT
CTGCTGACCGATGAGATGATCGCCCAGTACACATCTGCCCTGCTGGCCGGCACAA
TCACAAGCGGCTGGACATTTGGAGCTGGCGCCGCTCTGCAGATCCCCTTTGCTAT
GCAGATGGCCTACCGGTTCAACGGCATCGGAGTGACCCAGAATGTGCTGTACGAG
AACCAGAAGCTGATCGCCAACCAGTTCAACAGCGCCATCGGCAAGATCCAGGACA
GCCTGAGCAGCACAGCAAGCGCCCTGGGAAAGCTGCAGGACGTGGTCAACCAGA
ATGCCCAGGCACTGAACACCCTGGTCAAGCAGCTGTCCTCCAACTTCGGCGCCAT
CAGCTCTGTGCTGAACGATATCCTGAGCAGACTGGACAAGGTGGAAGCCGAGGTG
CAGATCGACAGACTGATCACCGGAAGGCTGCAGTCCCTGCAGACCTACGTTACCC
AGCAGCTGATCAGAGCCGCCGAGATTAGAGCCTCTGCCAATCTGGCCGCCACCAA
GATGTCTGAGTGTGTGCTGGGCCAGAGCAAGAGAGTGGACTTTTGCGGCAAGGG
CTACCACCTGATGAGCTTCCCTCAGTCTGCCCCTCACGGCGTGGTGTTTCTGCAC
 
GTGACATACGTGCCCGCTCAAGAGAAGAATTTCACCACCGCTCCAGCCATCTGCC
ACGACGGCAAAGCCCACTTTCCTAGAGAAGGCGTGTTCGTGTCCAACGGCACCCA
TTGGTTCGTGACCCAGCGGAACTTCTACGAGCCCCAGATCATCACCACCGACAAC
ACCTTCGTGTCTGGCAACTGCGACGTCGTGATCGGCATTGTGAACAATACCGTGTA
CGACCCTCTGCAGCCCGAGCTGGACAGCTTCAAAGAGGAACTGGATAAGTACTTT
AAGAACCACACAAGCCCCGACGTGGACCTGGGCGATATCAGCGGAATCAATGCCA
GCGTCGTGAACATCCAGAAAGAGATCGACCGGCTGAACGAGGTGGCCAAGAATCT
GAACGAGAGCCTGATCGACCTGCAAGAACTGGGGAAGTACGAGCAGTACATCAAG
TGGCCCTGGTACATCTGGCTGGGCTTTATCGCCGGACTGATTGCCATCGTGATGG
TCACAATCATGCTGTGTTGCATGACCAGCTGCTGTAGCTGCCTGAAGGGCTGTTGT
AGCTGTGGCAGCTGCTGCAAGTTCGACGAGGACGATTCTGAGCCCGTGCTGAAG
GGCGTGAAACTGCACTACACCTGA
- The S protein sequence reads as follows (SEQ ID NO 2):

### SARS-CoV-2 E nucleotide sequence inserted in MVA-COVID19(S)

SARS-CoV-2 E sequence inserted in MVAs was synthetized by GeneArt and was human codon optimized. The sequence derived from Wuhan seafood market pneumonia virus isolate Wuhan-Hu-1, GenBank: MN908947.3, https://www.ncbi.nlm.nih.gov/nuccore/MN908947
- The E nucleotide sequence reads as follows (SEQ ID NO 3): 
ATGTACAGCTTCGTGTCCGAGGAAACCGGCACACTGATCGTGAACAGCGTGCTGC
TGTTCCTGGCCTTCGTGGTGTTCCTGCTGGTTACCCTGGCTATCCTGACAGCCCTG
AGACTGTGCGCCTACTGCTGCAACATCGTGAACGTGTCCCTCGTGAAGCCCAGCT
TCTACGTGTACAGCAGAGTGAAGAACCTGAACTCCAGCAGGGTGCCCGACCTGCT
GGTCTAA
- The E protein sequence reads as follows (SEQ ID NO 4):

MYSFVSEETGTLIVNSVLLFLAFVVFLLVTLAILTALRLCAYCCNIVNVSLVKPSFYVYSR
VKNLNSSRVPDLLV

### SARS-CoV-2 M nucleotide sequence inserted in MVA-COVID19(S)

SARS-CoV-2 M sequence inserted in MVAs was synthetized by GeneArt and was human codon optimized. The sequence derived from Wuhan seafood market pneumonia virus isolate Wuhan-Hu-1, GenBank: MN908947.3, https://www.ncbi.nlm.nih.gov/nuccore/MN908947
- The M nucleotide sequence reads as follows (SEQ ID NO 5): 
ATGGCCGATAGCAATGGCACCATCACCGTGGAAGAACTGAAGAAACTGCTGGAAC
AGTGGAACCTCGTGATCGGCTTCCTGTTCCTGACCTGGATCTGCCTGCTGCAGTT
CGCCTACGCCAACCGGAACAGATTCCTGTATATCATCAAGCTGATCTTTCTGTGGC
TGCTGTGGCCCGTGACACTGGCCTGTTTTGTGCTGGCCGCCGTGTACAGAATCAA
 
CTGGATCACCGGCGGAATCGCCATTGCCATGGCTTGTCTTGTGGGCCTGATGTGG
CTGAGCTACTTTATCGCCAGCTTCCGGCTGTTCGCCCGGACCAGATCCATGTGGT
CCTTCAATCCCGAGACAAACATCCTGCTGAACGTGCCCCTGCACGGCACCATTCT
GACAAGACCTCTGCTGGAAAGCGAGCTGGTTATCGGCGCCGTGATCCTGAGAGGA
CACCTGAGAATTGCCGGCCACCACCTGGGCAGATGCGATATCAAGGACCTGCCTA
AAGAAATCACAGTGGCCACCAGCAGAACCCTGTCCTACTATAAGCTGGGCGCCTC
TCAGAGAGTGGCCGGCGATAGCGGATTTGCCGCCTACAGCAGATACCGGATCGG
CAACTACAAGCTGAACACCGACCACAGCTCCAGCAGCGACAATATCGCACTGCTG
GTGCAGTGA
- The M protein sequence reads as follows (SEQ ID NO 6):

MADSNGTITVEELKKLLEQWNLVIGFLFLTWICLLQFAYANRNRFLYIIKLIFLWLLWPVT
LACFVLAAVYRINWITGGIAIAMACLVGLMWLSYFIASFRLFARTRSMWSFNPETNILLN
VPLHGTILTRPLLESELVIGAVILRGHLRIAGHHLGRCDIKDLPKEITVATSRTLSYYKLGA
SQRVAGDSGFAAYSRYRIGNYKLNTDHSSSSDNIALLVQ

### SARS-CoV-2 RBD nucleotide sequence inserted in MVA

SARS-CoV-2 RBD sequence inserted in MVAs was synthetized by GeneArt and was human codon optimized. The sequence derived from Wuhan seafood market pneumonia virus isolate Wuhan-Hu-1, GenBank: MN908947.3, https://www.ncbi.nlm.nih.gov/nuccore/MN908947
- The RBD nucleotide sequence reads as follows (SEQ ID NO 7): 
ATGGAAACCGACACACTGCTGCTGTGGGTGCTGCTTTTGTGGGTGCCAGGCAGCA
CAGGCGATTACCCCGGGTCTAGATTCCCCAACATCACCAATCTGTGCCCCTTCGG
CGAGGTGTTCAATGCCACAAGATTTGCCAGCGTGTACGCCTGGAACCGGAAGAGA
ATCAGCAACTGCGTGGCCGACTACAGCGTGCTGTACAATAGCGCCAGCTTCAGCA
CCTTCAAGTGCTACGGCGTGTCCCCTACCAAGCTGAACGACCTGTGCTTCACCAAT
GTGTACGCCGACAGCTTCGTGATCAGAGGCGACGAAGTTCGGCAGATCGCTCCTG
GACAGACAGGCAAGATCGCCGATTACAACTACAAGCTGCCCGACGACTTCACCGG
CTGCGTGATCGCCTGGAATAGCAACAACCTGGACAGCAAAGTCGGCGGCAACTAC
 
AACTACCTGTACCGGCTGTTCCGGAAGTCCAACCTGAAGCCTTTCGAGCGGGACA
TCAGCACCGAGATCTATCAGGCCGGCAGCACCCCTTGTAATGGCGTGGAAGGCTT
CAACTGCTACTTCCCACTGCAGTCCTACGGCTTCCAGCCTACAAACGGCGTGGGC
TACCAGCCTTATAGAGTGGTGGTGCTGAGCTTCGAACTGCTGCATGCCCCTGCTA
CCGTGTGTGGCCCTAAGAAGTCCACCAACTGA
- The RBD protein sequence reads as follows (SEQ ID NO 8):

METDTLLLWVLLLWVPGSTGDYPGSRFPNITNLCPFGEVFNATRFASVYAWNRKRISN
CVADYSVLYNSASFSTFKCYGVSPTKLNDLCFTNVYADSFVIRGDEVRQIAPGQTGKIA
DYNYKLPDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKKSTN

### SARS-CoV-2 N nucleotide sequence inserted in MVA

SARS-CoV-2 N sequence inserted in MVAs was synthetized by GeneArt and was human codon optimized. The sequence derived from Wuhan seafood market pneumonia virus isolate Wuhan-Hu-1, GenBank: MN908947.3, https://www.ncbi.nlm.nih.gov/nuccore/MN908947
- The N nucleotide sequence reads as follows (SEQ ID NO 9): 
ATGAGCGACAACGGCCCTCAGAATCAGCGGAACGCCCCTAGAATCACATTTGGCG
GCCCTAGCGATAGCACCGGCAGCAATCAGAATGGCGAGAGAAGCGGCGCCAGAA
GCAAACAGAGAAGGCCTCAGGGCCTGCCTAACAACACCGCCTCTTGGTTTACAGC
CCTGACACAGCACGGCAAAGAGGACCTGAAGTTTCCTAGAGGCCAGGGCGTGCC
CATCAACACCAATAGCAGCCCTGATGACCAGATCGGCTACTACAGACGGGCCACC
AGAAGAATTAGAGGCGGCGACGGCAAGATGAAGGATCTGAGCCCCAGATGGTACT
TCTACTACCTCGGAACAGGACCCGAGGCCGGACTTCCTTATGGCGCCAACAAGGA
TGGCATCATCTGGGTTGCAACAGAAGGCGCCCTGAACACCCCTAAGGACCACATC
GGAACCAGAAATCCCGCCAACAATGCCGCCATTGTGCTGCAGTTGCCACAGGGCA
CAACCCTGCCTAAGGGCTTTTATGCCGAGGGCTCTAGAGGCGGAAGCCAGGCCTC
TAGCAGAAGCAGCAGCAGAAGCCGGAACTCCAGCAGAAATAGCACCCCTGGAAG
CTCCAGAGGCACATCTCCTGCCAGAATGGCCGGAAATGGCGGAGATGCTGCTCTT
GCTCTGCTGCTCCTGGATCGGCTGAACCAGCTGGAAAGCAAGATGTCTGGAAAGG
GCCAGCAGCAACAGGGCCAGACCGTGACAAAAAAATCTGCCGCCGAGGCCAGCA
 
AGAAGCCCAGACAGAAGAGAACCGCCACCAAGGCCTACAACGTGACCCAGGCCTT
TGGAAGAAGAGGCCCAGAGCAGACCCAGGGCAATTTCGGCGATCAAGAGCTGAT
CAGACAGGGCACCGACTACAAGCACTGGCCTCAGATTGCCCAGTTTGCCCCTAGC
GCCTCCGCCTTCTTTGGCATGTCTAGAATCGGCATGGAAGTGACCCCTAGCGGCA
CCTGGTTGACATACACAGGCGCCATCAAGCTGGACGACAAGGACCCCAACTTCAA
GGACCAAGTGATCCTGCTGAACAAGCACATCGACGCCTACAAGACATTCCCTCCA
ACCGAGCCTAAGAAGGACAAGAAGAAGGCCGACGAGACACAGGCCCTGCCA
CAGAGACAGAAAAACAGCAGACAGTGACCCTGCTGCCTGCCGCTGATCTGGACG
ATTTTTCCAAGCAGCTGCAGCAGTCCATGAGCAGCGCAGATAGCACACAGGCCTG
A
- The N protein sequence reads as follows (SEQ ID NO 10):

MSDNGPQNQRNAPRITFGGPSDSTGSNQNGERSGARSKQRRPQGLPNNTASWFTA
LTQHGKEDLKFPRGQGVPINTNSSPDDQIGYYRRATRRIRGGDGKMKDLSPRWYFYY
LGTGPEAGLPYGANKDGIIWVATEGALNTPKDHIGTRNPANNAAIVLQLPQGTTLPKGF
YAEGSRGGSQASSRSSSRSRNSSRNSTPGSSRGTSPARMAGNGGDAALALLLLDRL
NQLESKMSGKGQQQQGQTVTKKSAAEASKKPRQKRTATKAYNVTQAFGRRGPEQT
QGNFGDQELIRQGTDYKHWPQIAQFAPSASAFFGMSRIGMEVTPSGTWLTYTGAIKLD
DKDPNFKDQVILLNKHIDAYKTFPPTEPKKDKKKKADETQALPQRQKKQQTVTLLPAAD
LDDFSKQLQQSMSSADSTQA

## Claims

1. A vaccine composition comprising
a. an immunologically effective amount of a modified vaccinia virus Ankara (MVA) vector comprising at least one nucleic acid encoding an antigenic protein of at least one SARS-CoV-2 virus subtype, optionally together with a pharmaceutically acceptable carrier, diluent and/or additive; and/or
b. an immunologically effective amount of the modified vaccinia virus Ankara (MVA) vector of a), wherein said MVA vector is further modified by deletion of the MVA immunomodulatory genes *C6L, K7R,* and *A46R,* optionally together with a pharmaceutically acceptable carrier, diluent and/or additive;
wherein the nucleotide sequence encoding an antigenic protein of at least one SARS-CoV-2 virus subtype is inserted into the thymidine kinase (TK) locus of the MVA genome; and wherein the MVA vector regulates the expression of the nucleic acid encoding the antigenic protein and is operably linked to said nucleotide sequence.

2. The vaccine composition according to claim 1, wherein the antigenic protein is the structural protein S of SARS-CoV-2.

3. The vaccine composition according to claim 2, wherein the antigenic protein is the S protein of SARS-CoV-2 encoded by the nucleic acid consisting of SEQ ID NO 1.

4. The vaccine composition according to any of the precedent claims, wherein the MVA vector further comprises an antigenic protein selected from the any of the group of structural proteins E (envelope), receptor-binding domain (RBD), N (nucleocapsid) or M (membrane) of SARS-CoV-2.

5. The vaccine composition according to any of claims 1-4, wherein the MVA used for generating the recombinant virus is a MVA virus or a derivative having the capability of reproductive replication in vitro in chicken embryo fibroblasts (CEF) cells and in established chick cells DF-1, but no capability of reproductive replication in human cervix adenocarcinoma cell line HeLa.

6. The vaccine composition according to any of the precedent claims, wherein the modified (MVA) vector is the MVA as defined in claim 1a).

7. The vaccine composition according to any of claims 1 to 5, wherein the modified (MVA) vector is the MVA as defined in claim 1b)

8. The vaccine composition according to any of claims 1 to 7, wherein the vaccine further comprises one or more pharmaceutically acceptable carriers, additives, antibiotics, preservatives, adjuvants, diluents and/or stabilizers.

9. A vaccine combination comprising:
c. a first composition comprising an immunologically effective amount of a modified vaccinia virus Ankara (MVA) vector as defined in any of the precedent claims; and
d. a second composition comprising an immunologically effective amount of a modified vaccinia virus Ankara (MVA) vector as defined in any of the precedent claims;
wherein one of the compositions is a priming composition and the other composition is a boosting composition.

10. A vaccine combination comprising:
a. a first composition comprising an immunologically effective amount of a modified vaccinia virus Ankara (MVA) vector as defined in any of claims 1 to 8; and
b. a second composition comprising an immunologically effective amount of a polynucleotide plasmid that comprises at least one nucleic acid encoding an antigenic protein of at least one SARS-CoV-2 virus subtype;
wherein one of the compositions is a priming composition and the other composition is a boosting composition.

11. The vaccine combination comprising:
a. a first composition comprising an immunologically effective amount of a polynucleotide plasmid that comprises at least one nucleic acid encoding an antigenic protein of at least one SARS-CoV-2 virus subtype; and
b. a second composition comprising an immunologically effective amount of a modified vaccinia virus Ankara (MVA) vector as defined in any of claims 1 to 8;
wherein the first compositions is a priming composition and the second composition is a boosting composition.

12. The vaccine combination of any of claims 9 to 11, wherein the antigenic protein for both compositions is the structural protein S of SARS-CoV-2.

13. The vaccine combination of any of claims 9 to 11, wherein the at least one nucleic acid of both compositions consists of SEQ ID NO 1.

14. The vaccine composition according to claims 1-8, for use in generating an immunogenic and protective immune response against the SARS-CoV-2 virus in a subject in need thereof, preferably in a human subject, wherein said composition is used for priming said immune response and/or boosting said immune response and wherein the MVA is capable of producing at least the structural protein S of SARS-CoV-2, preferably of SEQ ID NO 2, in the subject to be treated.

15. The vaccine combination according to claims 9 to 11, for use in generating an immunogenic and protective immune response against the SARS-CoV-2 virus in a subject in need thereof, preferably in a human subject, wherein said combination is capable of producing at least the structural protein S of SARS-CoV-2, preferably of SEQ ID NO 2, in the subject to be treated.

16. The vaccine combination according to claim 9, for use in generating an immunogenic and protective immune response against the SARS-CoV-2 virus in a subject in need thereof, preferably in a human subject, wherein said combination is capable of producing at least the structural protein S of SARS-CoV-2, preferably of SEQ ID NO 2, in the subject to be treated.

17. The vaccine combination according to claim 11, for use in generating an immunogenic and protective immune response against the SARS-CoV-2 virus in a subject in need thereof, preferably in a human subject, wherein said combination is capable of producing at least the structural protein S of SARS-CoV-2, in the subject to be treated.

18. The vaccine composition according to claims 1-8 or the vaccine combination according to claims 9 to 13, for inducing an enhanced immune response against at least one SARS-CoV-2 virus subtype in a subject in need thereof, preferably in a human subject, wherein the MVA is capable of producing any of the group of structural proteins S, E, receptor-binding domain (RBD), N and/or M of SARS-CoV-2, or any combination thereof, in the subject to be treated.

19. The vaccine composition for use according to any of claims 14 or 18, wherein the MVA vector/s of the vaccine composition/s is/are to be administered once, twice, three times or four times.

20. The vaccine composition or combination for use according to any of claims 14 to 19, wherein said composition or combination is administered via the intramuscular route.

21. The vaccine composition or combination for use according to any of claims 14 to 20, wherein there is a boosting composition and said composition is administered at least 2 weeks after administering the priming composition

22. The vaccine composition or combination for use according to any of claims 14 to 20, wherein there is a boosting composition and said composition is administered 2-12 weeks, preferably 2, 3 or 4 weeks, after administering the priming composition

23. A kit comprising one, preferably two, or more doses of any of the vaccine compositions as defined in any of claims 1 to 8, or the vaccine combination of any of claims 9 to 13.

24. A kit comprising an immunologically effective amount of a MVA vector according to any of claims 1 to 8 in a first vial or container for a first administration (priming) and in a second vial or container for a second administration (boosting).

25. A kit comprising an immunologically effective amount of a polynucleotide plasmid that comprises at least one nucleic acid encoding an antigenic protein of at least one SARS-CoV-2 virus subtype, preferably the structural protein S of SARS-CoV-2, in a first vial or container for a first administration (priming), and an immunologically effective amount of a MVA vector according to any of claims 1 to 8 in a second vial or container for a second administration (boosting).

26. A kit comprising an immunologically effective amount of a MVA vector according to any of claims 1 to 8, in a first vial or container for a first administration (priming), and an immunologically effective amount of a polynucleotide plasmid that comprises at least one nucleic acid encoding an antigenic protein of at least one SARS-CoV-2 virus subtype, , preferably the structural protein S of SARS-CoV-2, in a second vial or container for a second administration (boosting).

27. The kit according to any of claims 23 to 26, comprising in a third, fourth or further vial or container a MVA vector according to any of claims 1 to 8 for a third, fourth or further administration.

28. The kit according to claims 23 to 27, for use in generating an immunogenic and protective immune response against the SARS-CoV-2 virus in a subject in need thereof, preferably in a human subject.

29. The kit according to claim 28, wherein the combination of compositions contained in the vials is capable of producing at least the structural protein S of SARS-CoV-2, in the subject to be treated, and generating an immunogenic and protective immune response against the SARS-CoV-2 virus in a subject in need thereof.

30. The kit for use according to any of claims 28 or 29, wherein said compositions contained in the vials are administered via the intramuscular route.

31. The kit for use according to any of claims 28 to 30, wherein the boosting composition is administered at least 2 weeks after administering the priming composition

32. The kit for use according to any of claims 28 to 30, wherein the boosting composition is administered 2-12 weeks, preferably 2, 3 or 4 weeks, after administering the priming composition.

33. A method of generating an immunogenic and protective immune response against the SARS-CoV-2 virus in a subject in need thereof, preferably in a human subject, the method comprising administering to the subject any of the vaccine compositions according to any of claims 1 to 8 or any of the vaccine combinations according to any of claims 9 to 13.

34. The method according to claim 33, wherein said method comprises administering any of the vaccine compositions according to any of claims 1 to 8 for priming said immune response and/or boosting said immune response.

35. The method according to claim 33, wherein said compositions or combinations of compositions contained in the vials are administered via the intramuscular route.

36. The method according to any of claims 33 to 35, wherein the boosting composition is administered at least 2 weeks after administering the priming composition.

37. The method according to any of claims 33 to 35, wherein the boosting composition is administered 2-12 weeks, preferably 2, 3 or 4 weeks, after administering the priming composition.

38. The method according to any of claims 33 to 37, wherein the boosting composition is administered two or more times to the subject.

39. A method for generating an immune response against the SARS-CoV-2 virus in a subject in need thereof, preferably a human subject, comprising administering to the subject any of the vaccine compositions according to any of claims 1 to 8 or any of the vaccine combinations according to any of claims 9 to 13.

40. The method according to claim 39, wherein said method comprises administering any of the vaccine compositions according to any of claims 1 to 8 for priming said immune response and/or boosting said immune response.

41. The method according to claim 39, wherein said compositions or combinations of compositions contained in the vials are administered via the intramuscular route.

42. The method according to any of claims 39 to 41, wherein the boosting composition is administered at least 2 weeks after administering the priming composition.

43. The method according to any of claims 39 to 41, wherein the boosting composition is administered 2-12 weeks, preferably 2, 3 or 4 weeks, after administering the priming composition.

44. The method according to any of claims 39 to 43, wherein the boosting composition is administered two or more times to the subject.

45. A method of inducing an enhanced immune response against the SARS-CoV-2 virus in a subject in need thereof, preferably in a human subject, comprising administering to the subject any of the vaccine compositions according to any of claims 1 to 8 or any of the vaccine combinations according to any of claims 9 to 13.

46. The method according to claim 45, wherein said compositions or combinations of compositions contained in the vials are administered via the intramuscular route.
